(19) Europäisches Patentamt
European Patent Office
Office européen des brevets

(11) **EP 3 693 365 A1**

(12) **EUROPEAN PATENT APPLICATION**
published in accordance with Art. 153(4) EPC

(43) Date of publication:
**12.08.2020 Bulletin 2020/33**

(21) Application number: **18864310.0**

(22) Date of filing: **05.10.2018**

(51) Int Cl.:
*C07D 401/06* (2006.01) *C07D 403/06* (2006.01)
*C07D 239/42* (2006.01) *C07D 213/74* (2006.01)
*C07D 401/10* (2006.01) *C07D 403/10* (2006.01)
*A61K 31/506* (2006.01) *A61K 31/505* (2006.01)
*A61K 31/44* (2006.01) *A61K 31/444* (2006.01)
*A61P 35/00* (2006.01)

(86) International application number:
**PCT/RU2018/050122**

(87) International publication number:
**WO 2019/070167 (11.04.2019 Gazette 2019/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.10.2017 RU 2017135686**
**28.09.2018 RU 2018134159**

(71) Applicant: **Joint Stock Company "Biocad"**
**St.Petersburg 198515 (RU)**

(72) Inventors:
- **ZAVIALOV, Kirill Vadimovich**
  **St.Petersburg 198261 (RU)**
- **GORBUNOVA, Svetlana Leonidovna**
  **St.Petersburg 197198 (RU)**
- **SHEKHAUTSOU, Artsiom Evgenievich**
  **Byhov 213320 (BY)**
- **KASATKINA, Mariia Andreevna**
  **Krasnodar 350040 (RU)**
- **BEKETOVA, Daria Dmitrievna**
  **St.Petersburg 198206 (RU)**
- **KOZHEMYAKINA, Natalia Vladimirovna**
  **St.Petersburg 192238 (RU)**
- **KULISH, Kirill Igorevich**
  **Ozersk**
  **Chelyabinskaya Oblast' 456785 (RU)**
- **MAKSIMENKO, Elena Aleksandrovna**
  **St.Petersburg 192283 (RU)**
- **MELESHINA, Marina Viktorovna**
  **St.Petersburg 198260 (RU)**
- **MELCHAEVA, Olga Anatolevna**
  **141407 (RU)**
- **MINDICH, Aleksei Leonidovich**
  **St.Petersburg 195426 (RU)**
- **MOROZOV, Dmitry Valentinovich**
  **St. Petersburg, 190000 (RU)**
- **POPKOVA, Aleksandra Vladimirovna**
  **Leningradskaya Obl. 188516 (RU)**
- **SMETANIN, Ilia Alexeevich**
  **Arhangelskaya Obl. 164288 (RU)**
- **SILONOV, Sergey Aleksandrovich**
  **Samara 443096 (RU)**
- **SOLDATOVA, Iaroslavna Alexandrovna**
  **St.Petersburg 196066 (RU)**
- **IAKOBSON, Georgii Viktorovich**
  **St.Petersburg 190103 (RU)**

(74) Representative: **Held, Stephan**
**Meissner Bolte Patentanwälte**
**Rechtsanwälte Partnerschaft mbB**
**Widenmayerstraße 47**
**80538 München (DE)**

(54) **EPIDERMAL GROWTH FACTOR RECEPTOR INHIBITORS**

(57) The present invention relates to novel compounds of formula I,

EP 3 693 365 A1

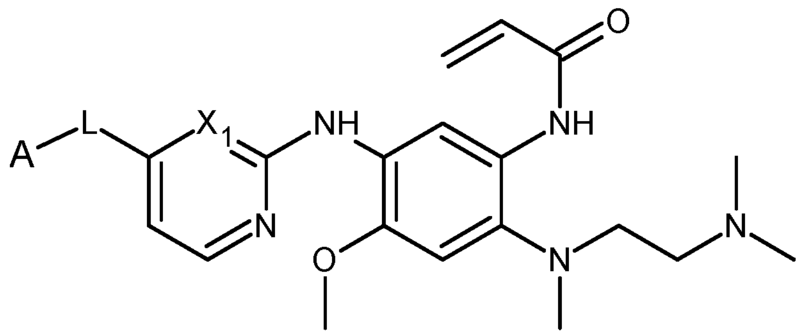

I

or pharmaceutically acceptable salts, solvates or stereoisomers thereof, also to a pharmaceutical composition, a method for inhibiting biological activity of epidermal growth factor receptor (EGFR), a method for treating diseases or disorders mediated by the activation of EGFR and use of the present compounds or the present pharmaceutical composition for the treatment of a disease or disorder mediated by the activation of EGFR.

## Description

### Field of invention

The present invention relates to novel epidermal growth factor receptor (EGFR) inhibitors and to pharmaceutically acceptable salts, solvates or stereoisomers thereof, pharmaceutical compositions comprising the present compounds, to method for treating and use of the present compounds as pharmaceuticals for the treatment of diseases or disorders.

### Background of the invention

Epidermal Growth Factor Receptor (EGFR) is a transmembrane protein, tyrosine kinase member of the erbB receptor family. EGFR consists of a glycosylated external ligand-binding domain (621 residues) and a cytoplasmic domain (542 residues) connected by a short 23 amino acid transmembrane linker. The extracellular part of EGFR contains 25 disulfide bonds and 12 N-linked glycosylation sites, and is generally considered to consist of four subdomains. X-ray crystal structures of the EGFR suggest that the receptor adopts both an autoinhibited tethered-conformation that cannot bind epidermal Growth Factor (EGF) (Ferguson et al., Mol Cell, 2003, vol 11:507-517 and an active conformation that may mediate EGF ligand binding and receptor dimerisation (Garrett et al., Cell 2002, vol 110:763-773; Ogiso et al., Cell, 2002, vol 110:775-787). Upon binding of a growth factor ligand such as epidermal growth factor (EGF), the receptor can homo-dimerise with another EGFR molecule or hetero-dimerise with another family member such as erbB2 (FIER2), erbB3 (HER3), or erbB4 (HER4). Homo- and/or hetero-dimerisation of erbB receptors results in the phosphorylation of key tyrosine residues in the intracellular domain and leads to the stimulation of numerous intracellular signal transduction pathways involved in cell proliferation and survival. Detailed reviews of erbB receptor signalling and its involvement in tumourigenesis are provided in Ciardiello F. N. Engl J Med 2008; 358:1160-1174 and Robert Roskoski Jr., Biochemical and Biophysical Research Communications 319 (2004) 1-11.

The EGFR's link to oncological diseases was first recognized when the transforming v-ErbB oncogene of the avian erythroblatosis virus was found to be a mutant homolog of human EGFR (Downward J. Nature. 1984; 307:521-527). The v-erbB oncogene was found to contain recombinations of the transmembrane and cytoplasmic domains of the EGFR (Olofsson B. Eur. J. Biochem. 1986; 160:261-266), implicating EGFR oncogenic aberrations. In addition to mutations, overexpression of EGFR was then observed to promote the progression of a number of malignant tumours (Gusterson B. Cell Biol. Int. Rep. 1984; 8:649-658), including sarcomas (Gusterson B. Int. J. Cancer. 1985; 36:689-693), non-small cell lung cancer (NSCLC) (Veale D. Br. J. Cancer. 1987; 55:513-516) and malignant gliomas (Wong A.J. Proc. Natl. Acad. Sci. USA. 1987; 84:6899-6903).

It is currently known that EGFR regulates numerous cellular processes via tyrosine-kinase mediated signal transduction pathways, including, but not limited to, activation of signal transduction pathways that control cell proliferation, differentiation, cell survival, apoptosis, angiogenesis, mitogenesis, and metastasis (Atalay et al., Ann. Oncology 14: 1346-1363 [2003]; Herbst R.S. Cancer. 2002; 94: 1593-1611; Modjtahedi et al., Br. J. Cancer. 1996; 73: 228-235). Overexpression of EGFR has been reported in numerous human malignant tumors, including cancers of the bladder, brain, head and neck, pancreas, lung, breast, ovary, colon, prostate, and kidney (Atalay et al., Ann. Oncology 14: 1346-1363 [2003]; Herbst R.S. Cancer. 2002; 94: 1593-1611; Modjtahedi et al., Br. J. Cancer. 1996; 73: 228-235). EGFR is also expressed in the cells of normal tissues, particularly the epithelial tissues of the skin, liver, and gastrointestinal tract, although at generally lower levels than in malignant cells (Herbst R.S. Cancer. 2002; 94: 1593-1611).

Low molecular weight EGFR tyrosine kinase inhibitors are known to be used in the treatment of oncological diseases, for example, in the treatment of non-small cell lung cancer, pancreatic cancer; anti-EGFR antibodies are used in the treatment of colorectal cancer, and head and neck cancer (Ping Wee. Cancers (Basel). 2017 May; 9(5): 52).

Frequent mutations and EGFR hyperexpression are observed in many oncological diseases, therefore, there remains a need for new effective and safe drugs directed to inhibiting EGFR activity.

### Description of the invention

The terms used in the description of this invention appear below.

Optionally substituted in one, two, three, or several positions means the specified group can be substituted by a radical or any combination of radicals in one, two, three, or from one to six positions.

"Alkyl" means an aliphatic straight chain or branched chain hydrocarbon group having from 1 to 12 carbon atoms, more preferably from 1 to 6 carbon atoms. Branched chain means alkyl chain having one or more "lower alkyl" substituents. Examples of alkyl groups include, but are not limited to, methyl, ethyl, *n*-propyl, *iso*-propyl, *n*-butyl, *iso*-butyl, *sec*-butyl, *tert*-butyl, *n*-pentyl, 2-pentyl, 3-pentyl, *neo*pentyl, *n*-hexyl. Alkyl may have substituents which may be same or different structure.

"Cycloalkyl" means a saturated carbocyclic ring that contains from 3 to 10 carbon ring atoms. Examples of cycloalkyl groups include, but are not limited to, monocyclic groups, such as cyclopropyl, cyclobutyl, cyclopentyl, cyclohexyl, cycloheptyl, cyclooctyl, cyclononyl or cyclodecyl, bicyclic groups, such as bicycloheptyl or bicyclooctyl. Cycloalkyl may have substituents which may be same or different structure.

«Alkenyl» means an aliphatic straight chain or branched chain hydrocarbon group having from 1 to 12 carbon atoms, more preferably from 1 to 6 carbon atoms that contains one or more carbon-carbon double bound. Alkenyl may have substituents which may be same or different structure.

«Alkynyl» means an aliphatic straight chain or branched chain hydrocarbon group having from 1 to 12 carbon atoms, more preferably from 1 to 6 carbon atoms that contains one or more carbon-carbon triple bound. Alkynyl may have substituents which may be same or different structure.

"Aryl" means an aromatic monocyclic or polycyclic system having from 6 to 14 carbon atoms, more preferably from 6 to 10 carbon atoms. Examples of aryl groups include, but are not limited to, phenyl, phenylene, benzenetriyl, indanyl, naphthyl, naphthylene, naphthalenetriyl and anthrylene. Aryl may have cyclic system substituents which may be same or different structure. Aryl can be annelated with a nonaromatic cyclic system or heterocycle.

"Alkyloxy", "Alkoxy" or "alkyloxy group" means an alkyl-O- group, wherein alkyl is defined in this section. Examples of alkoxy groups include, but are not limited to, methoxy, ethoxy, *n*-propoxy, *iso*-propoxy, *n*-butoxy, *tert*-butoxy, *iso*-butoxy.

"Aryloxy" or "aryloxy group" means an aryl-O- group, wherein aryl is defined in this section. An example of aryloxy group is, without limitation, phenoxy group.

"Cycloalkyloxy" or "cycloalkyloxy group" means a cycloalkyl-O- group, wherein cycloalkyl is defined in this section. Examples of cycloalkyloxy groups include, but are not limited to, cyclohexyloxy, cyclopentyloxy, cyclobutyloxy or cyclopropyloxy.

"Amino group" means R'R"N-group.

"Aminocarbonyl" means -C(=O)NR'R" group.

Examples of R' and R" include, but are not limited to, substituents selected from the group comprising hydrogen, alkyl, alkenyl, alkynyl, cycloalkyl, aryl, heterocyclyl, heteroaryl, or R' and R" together with the carbon atom they are attached to, can form 4-7-membered heterocyclyl or heteroaryl.

"Lower alkyl" means a straight chain or branched chain alkyl having from 1 to 4 carbon atoms.

"Halo" or "Halogen" (Hal) means fluoro, chloro, bromo and iodo.

"Heterocycle", "heterocyclyl", "heterocyclic ring" means a monocyclic or polycyclic system having from 3 to 11 carbon atoms, of which one or more carbon atoms are substituted by one or more heteroatoms, such as nitrogen, oxygen, sulfur. Heterocycle can be condensed with aryl or heteroaryl. Heterocycle may have one or more substituents which may be same or different structure. Nitrogen and sulfur atoms of heterocycle could be oxidized to N-oxide, S-oxide or S-dioxide. Heterocycle may be fully saturated, partially saturated and unsaturated. Examples of heterocycle include, but are not limited to, azetidine, pyrrolidine, piperidine, 2,8-diazaspiro[4.5]decane, piperazine, morpholine, and others.

"Heteroaryl" means an aromatic monocyclic or polycyclic system having from 5 to 11 carbon atoms, preferably from 5 to 10, of which one or more carbon atoms are substituted by one or more heteroatoms, such as nitrogen, sulfur or oxygen. Nitrogen atom of heteroaryl could be oxidized to N-oxide. Heteroaryl may have one or more substituents which may be same or different structure. Examples of heteroaryl are pyrrolyl, furanyl, thienyl, pyridyl, pyrazinyl, pyrimidinyl, pyridazinyl, isoxazolyl, isothiazolyl, tetrazolyl, oxazolyl, thiazolyl, pyrazolyl, furazanyl, triazolyl, 1,2,4-thiadiazolyl, quinoxalinyl, phthalazinyl, imidazo[1,2-a]pyridinyl, imidazo[2,1-b]thiazolyl, benzofurazanyl, indolyl, azaindolyl, benzimidazolyl, benzothiazenyl, quinolinyl, imidazolyl, pyrazolyl, thienopyridyl, quinazolinyl, naphthyridinyl, thienopyrimidinyl, pyrrolopyridinyl, imidazopyridyl, isoquinolinyl, benzoazaindolyl, 1,2,4-triazinyl, thienopyrrolyl, furopyrrolyl, and the like.

"Partially saturated" means a ring system including at least one double or triple bond. The term "partly saturated" relates to rings having many sites for saturation and does not include aryl and heteroaryl systems as they defined above.

The term "oxo" used in this document relates to the radical =O. "Substituent" means a chemical radical attached to a scaffold (fragment).

"Solvate" is a molecular aggregate that consists of the compound of the present invention, or its pharmaceutically acceptable salt, with one or more solvent molecules. The solvent molecules are molecules of common pharmaceutical solvents, known to be safe for recipients, e.g. water, ethanol, ethylene glycol, etc. Other solvents, such as methanol, methyl-tert-butyl ether, ethyl acetate, methyl acetate, (R)-propylene glycol or (S)-propylene glycol, 1,4-butanediol, and the like, can be used to form intermediate solvates for obtaining preferable solvates.

"Hydrate" means a solvate with water as the solvent.

Solvates and/or hydrates preferably exist in crystalline form.

Terms "bond", "chemical bond", or "single bond" refer to a chemical bonding of two atoms or two moieties (i.e., groups, fragments) when the atoms joined by the bond are considered to be part of larger substructure.

The term "stereoisomers" refers to compounds that have identical chemical composition and the same structure, but differ in the spatial arrangement of atoms or their groups. Stereoisomers may include geometric isomers, enantiomers,

diastereomers.

The term "protecting group" refers to groups that are used to block the reactivity of functional groups, such as an amino group, carboxyl group or hydroxy group. Examples of protecting groups include, but are not limited to, tert-butyloxycarbonyl (Boc), benzyloxycarbonyl (Cbz), 2-(trimethylsilyl) ethoxy)methyl acetal (SEM), trialkylsilyl, alkyl(diaryl)silyl or alkyl.

The term "excipient" is used herein to describe any ingredient other than the compound(s) of the invention.

"Pharmaceutical composition" means a composition, comprising a compound of the invention and one or more pharmaceutically acceptable excipients. Examples of excipients include, but are not limited to, pharmaceutically acceptable and pharmacologically compatible fillers, solvents, diluents, carriers, auxiliary, distributing and sensing agents, delivery agents, such as preservatives, stabilizers, filler, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the type and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugars, sodium chloride, and similar compounds. Prolonged action of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, silicates and the like. Examples of suitable lubricants are magnesium stearate, sodium lauryl sulfate, talc and polyethylene glycol of high molecular weight. Pharmaceutical composition for peroral, sublingual, transdermal, intramuscular, intravenous, subcutaneous, local or rectal administration of active ingredient, alone or in combination with another active compound may be administered to human and animals in a standard administration form, in a mixture with traditional pharmaceutical carriers. Suitable standard administration forms include peroral forms such as tablets, gelatin capsules, pills, powders, granules, chewing-gums and peroral solutions or suspensions; sublingual and transbuccal administration forms; aerosols; implants; local, transdermal, subcutaneous, intramuscular, intravenous, intranasal or intraocular forms and rectal administration forms.

"Pharmaceutically acceptable salt" means relatively nontoxic both organic and inorganic salts of acids and bases disclosed in this invention. Salts could be prepared in situ in processes of synthesis, isolation or purification of compounds or they could be prepared specially. In particular, salts of bases could be prepared specially starting from purified bases disclosed in the invention and suitable organic or inorganic acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of such salts properties is given in: Berge S.M., et al., "Pharmaceutical Salts" J. Pharm. Sci. 1977, 66: 1 - 19). Salts of disclosed acids may be prepared by reaction of purified acids with suitable base; moreover, metal salts and amine salts may be synthesized too. Metal salts are salts of sodium, potassium, calcium, barium, zinc, magnesium, lithium and aluminum; sodium and potassium salts being preferred. Suitable inorganic bases from which metal salts can be prepared are: sodium hydroxide, carbonate, bicarbonate and hydride; potassium hydroxide and bicarbonate, lithium hydroxide, calcium hydroxide, magnesium hydroxide, zinc hydroxide. Organic bases suitable for preparation of salts of disclosed acids are amines and amino acids, the basicity of which is sufficient enough to produce stable salt, and which are suitable for use in medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from aminoacids-lysine, ornithine and arginine.

"Medicament" - is a compound (or a mixture of compounds as a pharmaceutical composition) in the form of tablets, capsules, injections, ointments and other ready forms intended for restoration, improvement or modification of physiological functions in humans and animals, and for treatment and prophylaxis of diseases, for diagnostics, anesthesia, contraception, cosmetology and others.

"Treat", "treating" and "treatment" refer to a method of alleviating or abrogating a biological disorder and/or at least one of its attendant symptoms. As used herein, to "alleviate" a disease, disorder or condition means reducing the severity and/or occurrence frequency of the symptoms of the disease, disorder, or condition. Further, references herein to "treatment" include references to curative, palliative treatment.

"Prophylaxis", "prophylactic therapy" refers to a set of measures aimed at preventing the occurrence, eliminating risk factors or at the early detection of a disease or disorder, their exacerbations, recurrences, complications or other

consequences.

In one aspect, the subject of treatment, or patient, is a mammal, preferably a human subject. Said subject may be either male or female, of any age.

"Disorder" means any condition that would benefit from treatment with the compound of the present invention. This means chronic and acute disorders or diseases including those pathological conditions that predispose the mammal to the disorder in question. Non-limiting examples of disorders to be treated herein include benign and malignant neoplasms, or neoplasms of unspecified nature, including tumors originating from blood cells and lymphoid cells. The examples can be: bladder cancer, ovarian cancer, cervical cancer, colorectal cancer, breast cancer, pancreatic cancer, head and neck cancer, glioma, glioblastoma, melanoma, prostate cancer, leucosis, lymphoma, non-Hodgkin lymphoma, Hodgkin's lymphoma, lung cancer, non-small cell lung cancer, hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

"Therapeutically effective amount" refers to that amount of the therapeutic agent being administered which will relieve to some extent one or more of the symptoms of the disease/ disorder being treated.

As used herein, the words "comprise," "have," "include," or variations such as "comprises," "comprising," "has," "having," "includes" or "including", and all grammatical variations thereof will be understood to imply the inclusion of a stated integer or group of integers but not the exclusion of any other integer or group of integers.

## Detailed description of the invention

In one embodiment, the present invention relates to the compound of formula **I**:

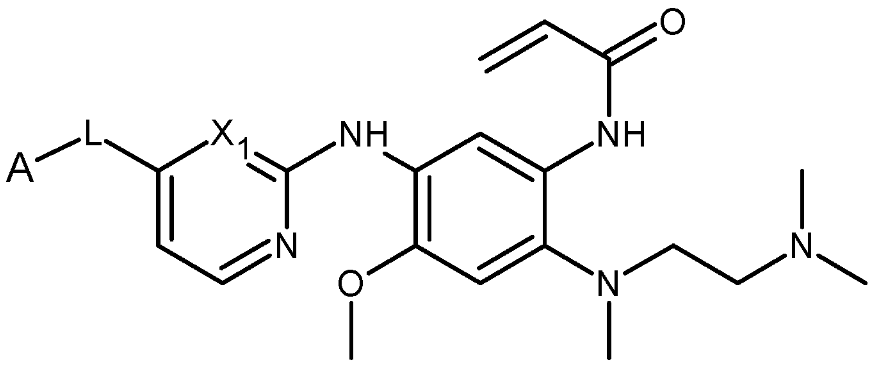

or pharmaceutically acceptable salt, solvate or stereoisomer thereof,

wherein L is -C(O)- or -CHOH-;
$X_1$ is CH or N;
A is

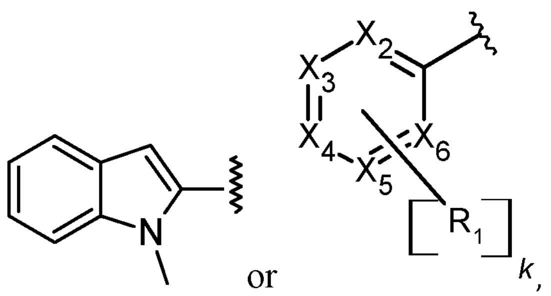

each $X_2$, $X_3$, $X_4$, $X_5$, $X_6$ is independently C, CH or N,
each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkyloxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one

or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N and/or O, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;

each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;

k is 0, 1, 2 or 3;

Hal is fluoro, bromo, chloro or iodo.

In another one embodiment, the present invention relates to the compound of formula I, wherein the fragment

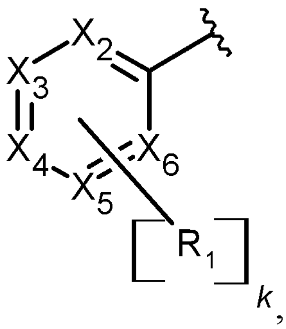

is selected from group, comprising:

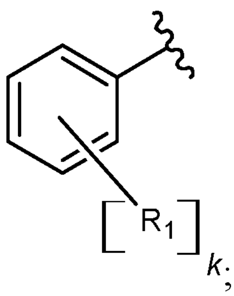
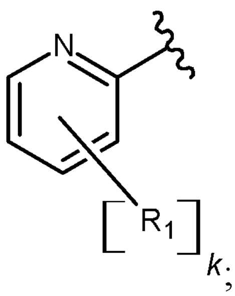
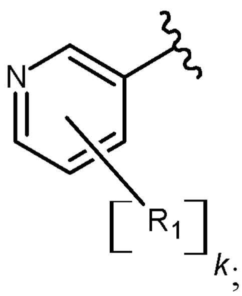
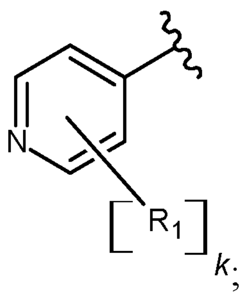
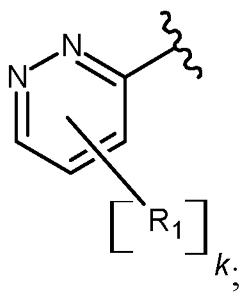

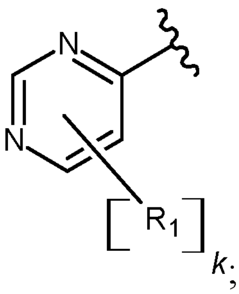
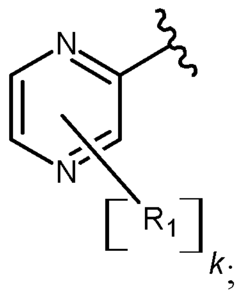
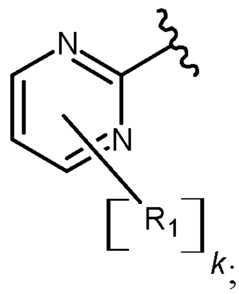
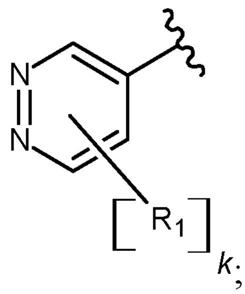
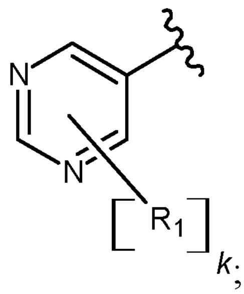

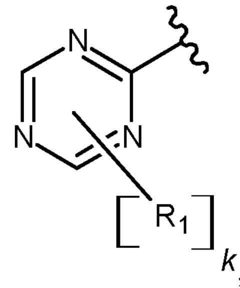,

wherein each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
k is 0, 1, 2 or 3;
Hal is fluoro, bromo, chloro or iodo.

In another one embodiment, the present invention relates to the compound of formula I, wherein the fragment

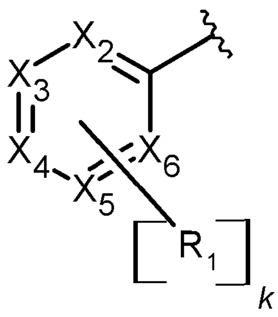,

is selected from group, comprising:

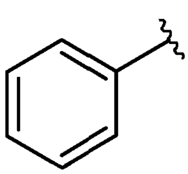 ; 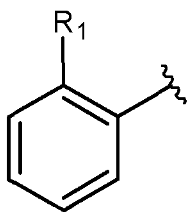 ; 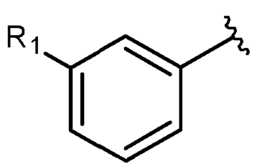 ; 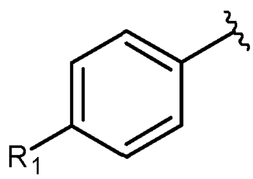 ; 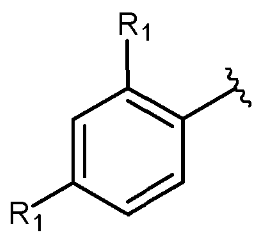 ;

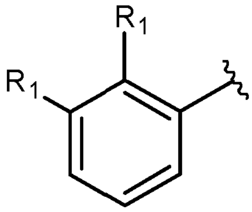 ; 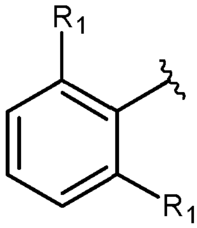 ; 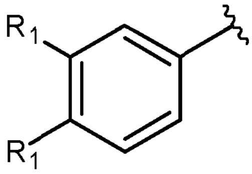 ; 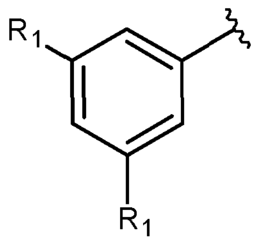 ;

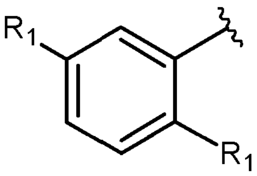 ; 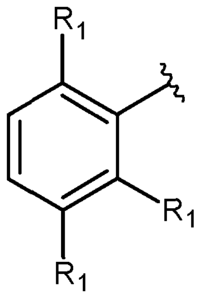 ; 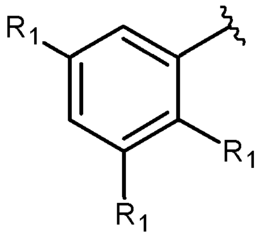 ; 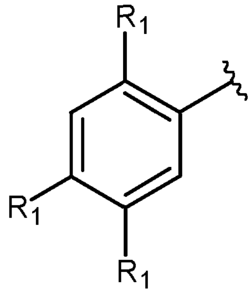 ;

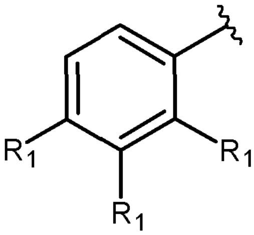 ; 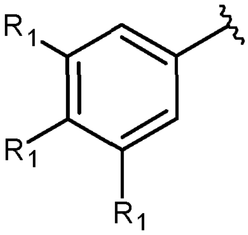 ; 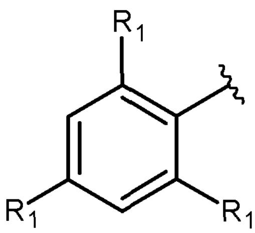 ; 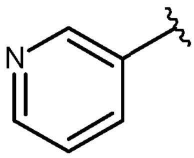 ; 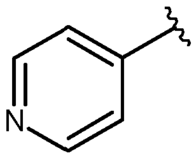 ;

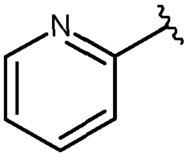 ; 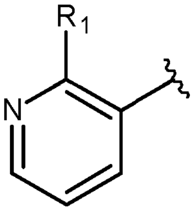 ; 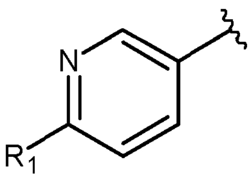 ; 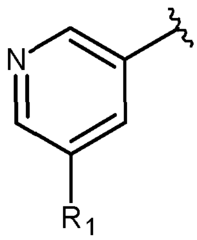 ; 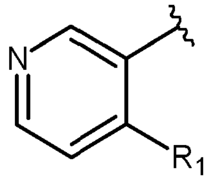 ;

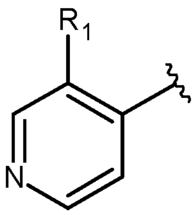 ; 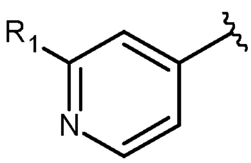 ; 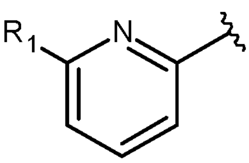 ; 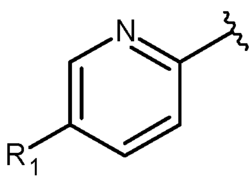 ; 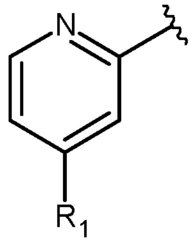 ;

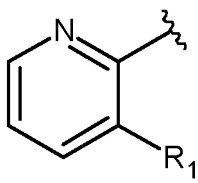 ;

wherein each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
k is 0, 1, 2 or 3;
Hal is fluoro, bromo, chloro or iodo.

In another one embodiment, the present invention relates to the compound of formula I, wherein the fragment

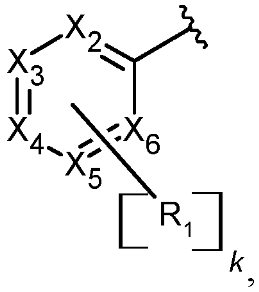

is selected from group, comprising:

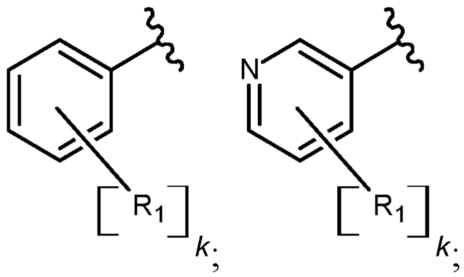

wherein $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
k is 0, 1, 2 or 3;
Hal is fluoro, bromo or chloro.

In another one embodiment, the present invention relates to the compound of formula I, wherein the fragment

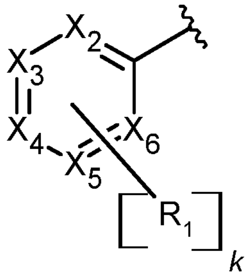,

is selected from group, comprising:

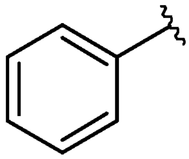; 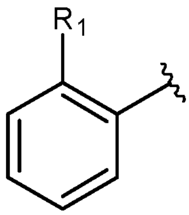; 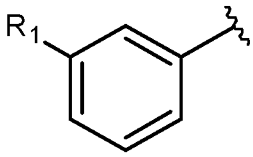; 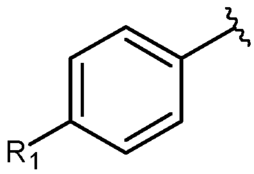; 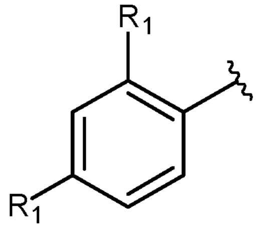;

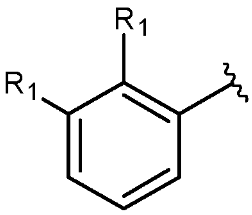; 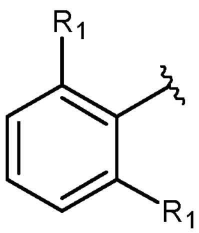; 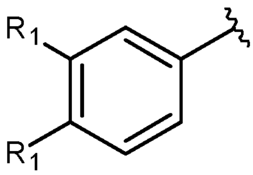; 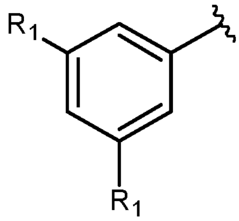;

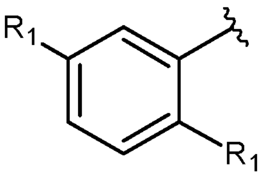; 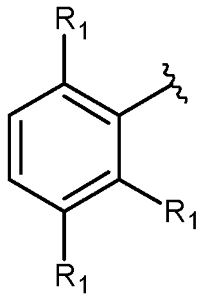; 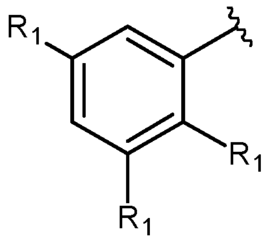; 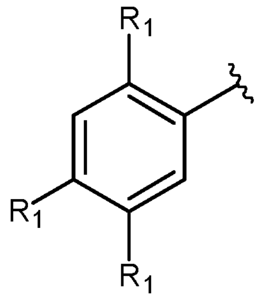;

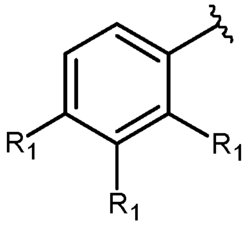; 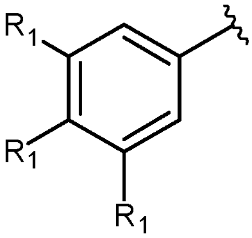; 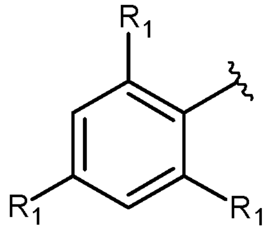; 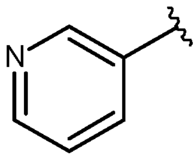; 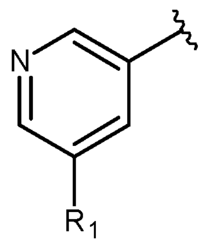;

wherein each $R_1$ is independently hydrogen; Hal; cyano; nitro; hydroxy group; $C_1$-$C_6$ alkyl, unsubstituted or substituted by one or several radicals selected from Hal, hydroxy group, $-NR_2R_3$; $C_1$-$C_6$ alkoxy group, unsubstituted or substituted by one or several radicals selected from Hal, $-NR_2R_3$, hydroxy group, $C_1$-$C_6$ alkyloxy, aryl, unsubstituted or substituted by one or several radicals selected from Hal, hydroxy group, $-NR_2R_3$; aryloxy, unsubstituted or substituted by one or several radicals selected from Hal, $C_1$-$C_6$ alkyl, hydroxy group, $-NR_2R_3$; $C_3$-$C_6$ cycloalkyloxy, unsubstituted or substituted by one or several radicals selected from Hal, hydroxy group, $-NR_2R_3$; $C_1$-$C_6$ alkyloxy

$C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
Hal is fluoro, bromo or chloro.

Compounds, described in the present invention, may be formed as, and/or used as, pharmaceutically acceptable salts. The type of pharmaceutical acceptable salts, include, but are not limited to: acid addition salts, formed by reacting the free base form of the compound with a pharmaceutically acceptable inorganic acid such as hydrochloric acid, hydrobromic acid, sulfuric acid, nitric acid, phosphoric acid, metaphosphoric acid, and the like; or with an organic acid such as acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-3-hydroxy-2-ene-1-carboxylic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, and the like.

The corresponding counterions of the pharmaceutically acceptable salts may be analyzed and identified using various methods including, but not limited to, ion exchange chromatography, ion chromatography, capillary electrophoresis, inductively coupled plasma, atomic absorption spectroscopy, mass spectrometry, or any combination thereof.

The salts are recovered by using at least one of the following techniques: filtration, precipitation with a non-solvent followed by filtration, evaporation of the solvent, or, in the case of aqueous solutions, lyophilization. It should be understood that a reference to a pharmaceutically acceptable salt includes the solvent addition forms or crystal forms thereof, particularly solvates or polymorphs. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol. Solvates of compounds described herein can be conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

Compounds described herein may be in various forms, including but not limited to, amorphous forms, milled forms and nano-particulate forms. In addition, compounds described herein include crystalline forms, also known as polymorphs. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and solubility. Various factors such as the recrystallization solvent, rate of crystallization, and storage temperature may cause one crystal form to dominate.

The screening and characterization of the pharmaceutically acceptable salts, polymorphs and/or solvates may be accomplished using a variety of techniques including, but not limited to, thermal analysis, x-ray diffraction, spectroscopy, vapor sorption, and microscopy. Thermal analysis methods address to analysis of thermo chemical degradation or thermo physical processes including, but not limited to, polymorphic transitions, and such methods are used to analyze the relationships between polymorphic forms, to determine weight loss, to find the glass transition temperature, or for excipient compatibility studies. Such methods include, but are not limited to, Differential scanning calorimetry (DSC), Modulated Differential Scanning Calorimetry (MDCS), Thermogravimetric analysis (TGA), Thermogravimetric and Infrared analysis (TG/IR). X-ray diffraction methods include, but are not limited to, single crystal and powder diffractometers and synchrotron sources. The various spectroscopic techniques used include, but are not limited to, Raman, FTIR, UVIS, and NMR (liquid and solid state). The various microscopy techniques include, but are not limited to, polarized light microscopy, Scanning Electron Microscopy (SEM) with Energy Dispersive X-Ray Analysis (EDX), Environmental Scanning Electron Microscopy with EDX (in gas or water vapor atmosphere), IR microscopy, and Raman microscopy.

In another embodiment of the present invention relates to the compounds selected from the group including:

| Formula | Name | Code |
|---|---|---|
| 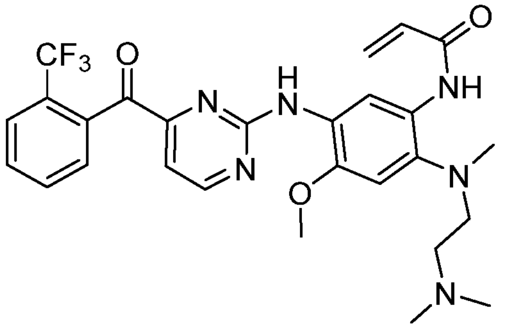 | N-(2-((2-(dimethylamino)ethyl) (methyl)amino)-4-methoxy-5-((4-(2-(trifluoromethyl) benzoyl) pyrimidin-2-yl) amino)phenyl)acrylamide | EGFR_ 3365 |
| 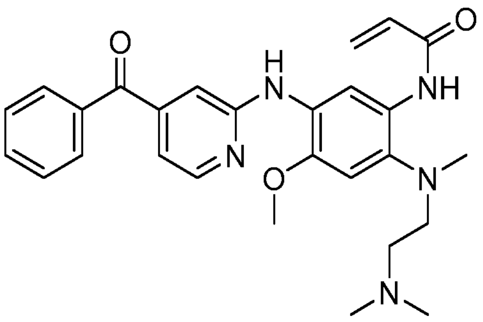 | N-(5-((4-benzoylpyridin-2-yl) amino)-2-((2-(dimethylamino) ethyl)(methyl)amino)-4-methoxyphenyl)acrylamide | EGFR_ 3365 _3 |
| 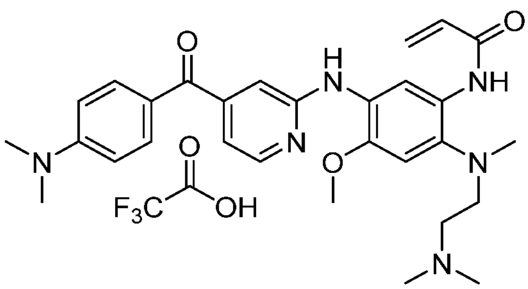 | N-(5-((4-(4-(dimethylamino) benzoyl)pyridin-2-yl)amino)-2-((2-(dimethylamino) ethyl) (methyl) amino)-4-methoxyphenyl)acrylamide 2,2,2-trifluoroacetate | EGFR_ 3365 _4 |
| 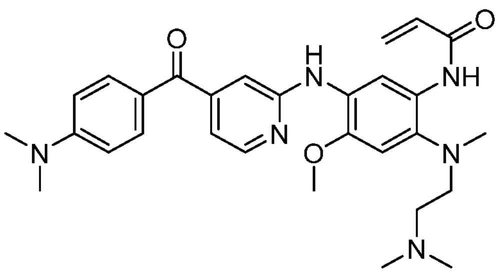 | N-(5-((4-(4-(dimethylamino) benzoyl)pyridin-2-yl)amino)-2-((2-(dimethylamino) ethyl)(methyl) amino)-4-methoxyphenyl)acrylamide | EGFR_ 3365 _4a |
| 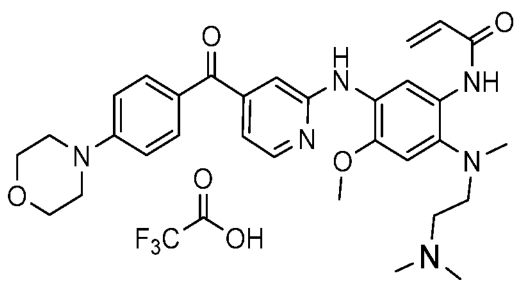 | N-(2-((2-(dimethylamino) ethyl)(methyl)amino)-4-methoxy-5-((4-(4-morpholinobenzoyl)pyridin-2-yl)amino)phenyl)acrylamide 2,2,2-trifluoroacetate | EGFR_ 3365 _5 |

(continued)

| Formula | Name | Code |
|---|---|---|
| 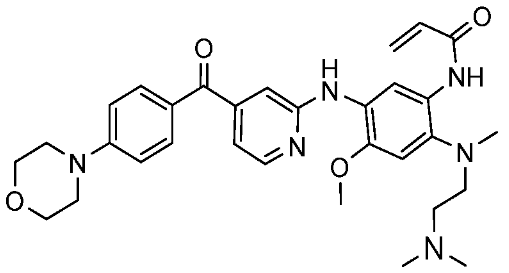 | N-(2-((2-(dimethylamino) ethyl)(methyl)amino)-4-methoxy-5-((4-(4-morpholinobenzoyl)pyridin-2-yl)amino)phenyl)acrylamide | EGFR_3365_5a |
| 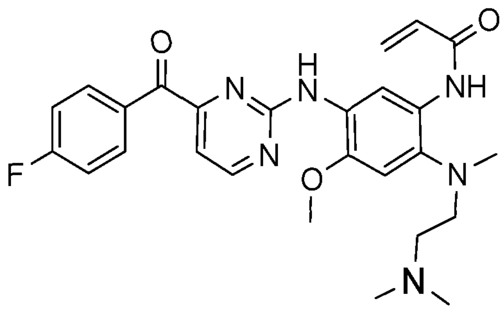 | N-(2-((2-(dimethylamino) ethyl)(methyl)amino)-5-((4-(4-fluorobenzoyl)pyrimidin-2-yl) amino)-4-methoxyphenyl) acrylamide | EGFR_3365_10 |
| 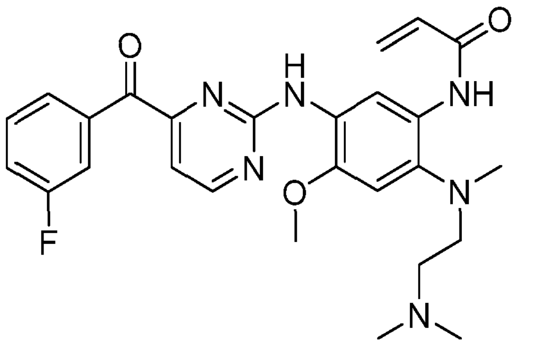 | N-(2-((2-(dimethylamino) ethyl)(methyl)amino)-5-((4-(3-fluorobenzoyl)pyrimidin-2-yl) amino)-4-methoxyphenyl) acrylamide | EGFR_3365_11 |
| 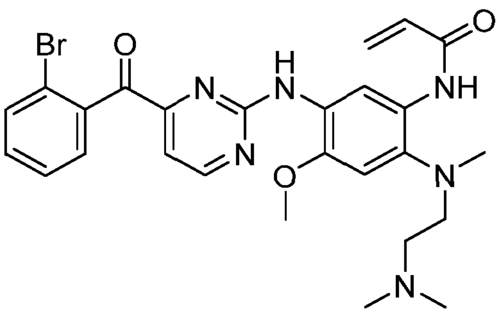 | N-(5-((4-(2-bromobenzoyl) pyrimidin-2-yl) amino)-2-((2-(dimethylamino)ethyl) (methyl) amino)-4-methoxyphenyl)acrylamide | EGFR_3365_12 |
| 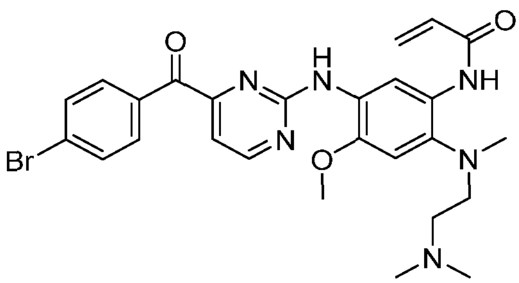 | N-(5-((4-(4-bromobenzoyl) pyrimidin-2-yl) amino)-2-((2-(dimethylamino)ethyl) (methyl) amino)-4-methoxyphenyl)acrylamide | EGFR_3365_13 |

(continued)

| Formula | Name | Code |
|---|---|---|
| 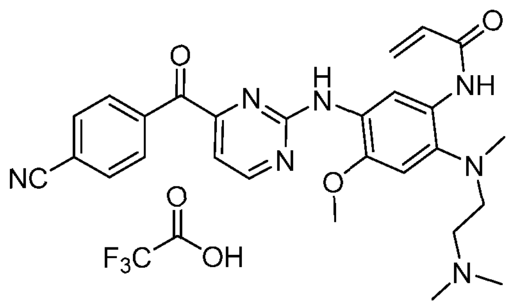 | N-(5-((4-(4-cyanobenzoyl) pyrimidin-2-yl) amino)-2-((2-(dimethylamino)ethyl) (methyl) amino)-4-methoxyphenyl)acry lamide 2,2,2-trifluoroacetate | EGFR_ 3365 _14 |
| 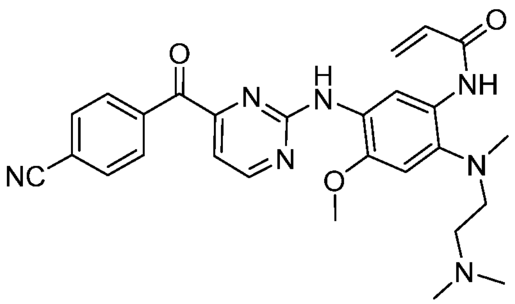 | N-(5-((4-(4-cyanobenzoyl) pyrimidin-2-yl) amino)-2-((2-(dimethylamino)ethyl) (methyl) amino)-4-methoxyphenyl)acrylamide | EGFR_ 3365 _14a |
| 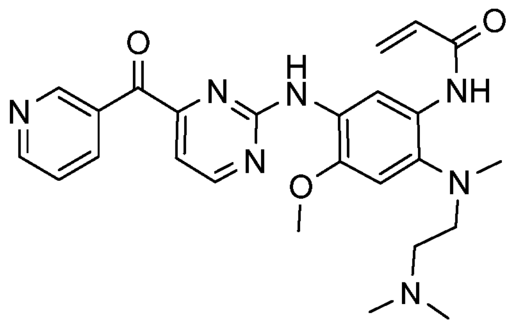 | N-(2-((2-(dimethylamino) ethyl)(methyl)amino)-4-methoxy-5-((4-nicotinoylpyrimidin-2-yl) amino) phenyl)acrylamide | EGFR_ 3365 _15 |
| 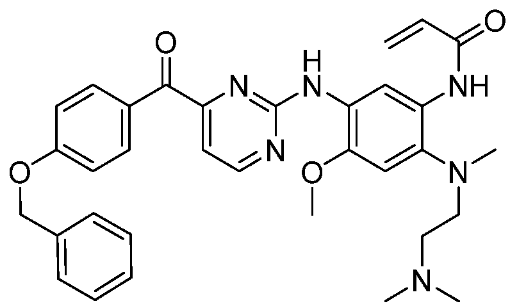 | N-(5-((4-(4-(benzyloxy) benzoyl)pyrimidin-2-yl) amino)-2-((2-(dimethylamino) ethyl)(methyl) amino)-4-methoxyphenyl)acrylamide | EGFR_ 3365 _16 |
| 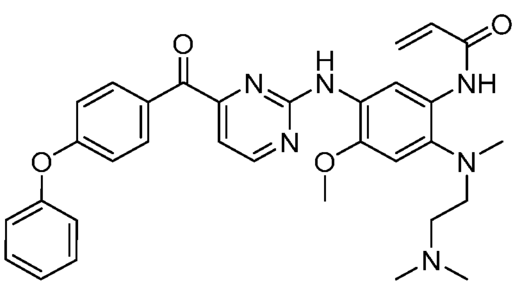 | N-(2-((2-(dimethylamino) ethyl)(methyl)amino)-4-methoxy-5-((4-(4-phenoxybenzoyl)pyrimidin-2-yl)amino)phenyl)acrylamide | EGFR_ 3365 _17 |

(continued)

| Formula | Name | Code |
|---|---|---|
| 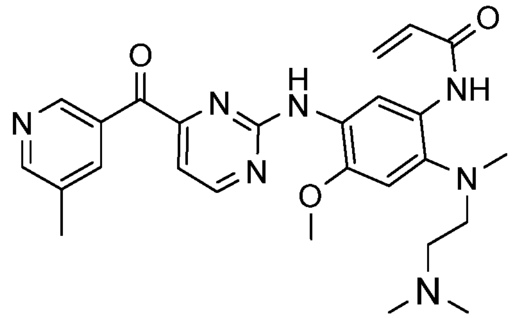 | N-(2-((2-(dimethylamino) ethyl)(methyl)amino)-4-methoxy-5-((4-(5-methylnicotinoyl)pyrimidin-2-yl)amino)phenyl)acrylamide | EGFR_3365_26 |
| 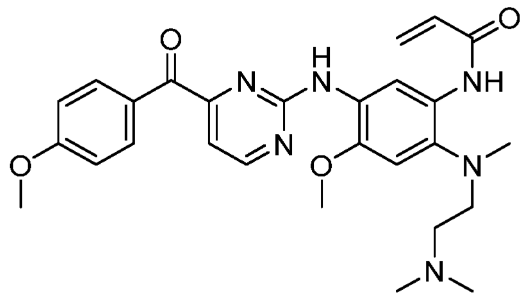 | N-(2-((2-(dimethylamino) ethyl)(methyl)amino)-4-methoxy-5-((4-(4-methoxybenzoyl)pyrimidin-2-yl)amino)phenyl)acrylamide | EGFR_3365_28 |
| 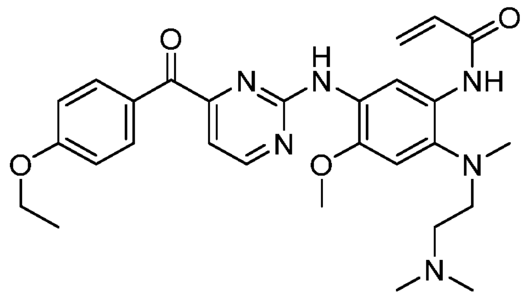 | N-(2-((2-(dimethylamino) ethyl)(methyl)amino)-5-((4-(4-ethoxybenzoyl)pyrimidin-2-yl) amino)-4-methoxyphenyl) acrylamide | EGFR_3365_29 |
| 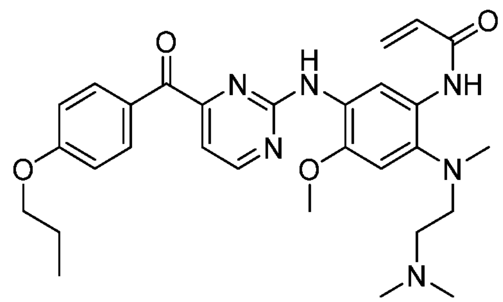 | N-(2-((2-(dimethylamino) ethyl)(methyl)amino)-4-methoxy-5-((4-(4-propoxybenzoyl)pyrimidin-2-yl)amino)phenyl)acrylamide | EGFR_3365_30 |
| 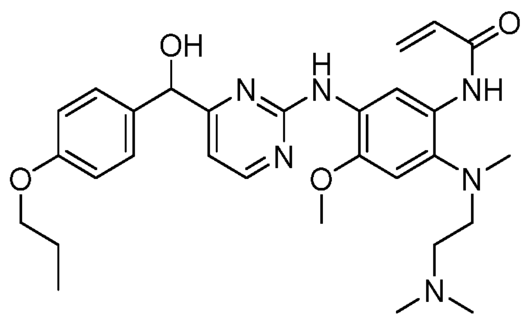 | N-(2-((2-(dimethylamino) ethyl)(methyl)amino)-5-((4-(hydroxy(4 -propoxyphenyl) methyl) pyrimidin-2-yl)amino)-4-methoxyphenyl) acrylamide | EGFR_3365_30a |

(continued)

| Formula | Name | Code |
|---|---|---|
| 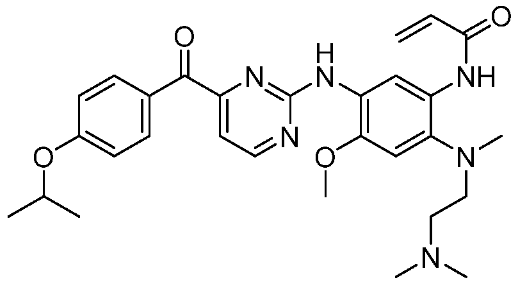 | N-(2-((2-(dimethylamino) ethyl)(methyl)amino)-5-((4-(4-isopropoxybenzoyl) pyrimidin-2-yl) amino)-4-methoxyphenyl) acrylamide | EGFR_3365_31 |
| 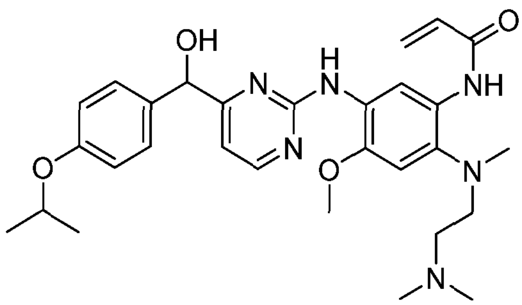 | N-(2-((2-(dimethylamino) ethyl)(methyl)amino)-5-((4-(hydroxy(4-isopropoxyphenyl) methyl) pyrimidin-2-yl)amino)-4-methoxyphenyl) acrylamide | EGFR_3365_31a |
| 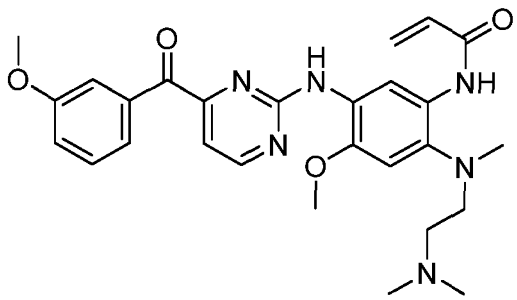 | N-(2-((2-(dimethylamino) ethyl)(methyl)amino)-4-methoxy-5-((4-(3-methoxybenzoyl)pyrimidin-2-yl)amino)phenyl)acrylamide | EGFR_3365_32 |
| 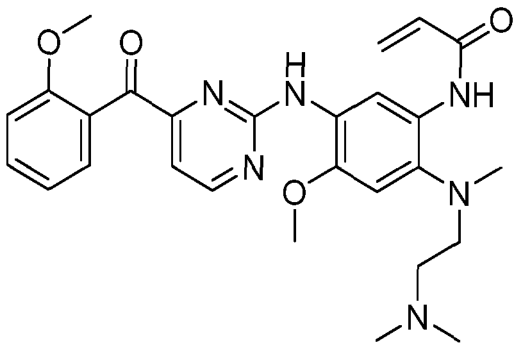 | N-(2-((2-(dimethylamino) ethyl)(methyl)amino)-4-methoxy-5-((4-(2-methoxybenzoyl)pyrimidin -2-yl)amino)phenyl)acrylamide | EGFR_3365_33 |
| 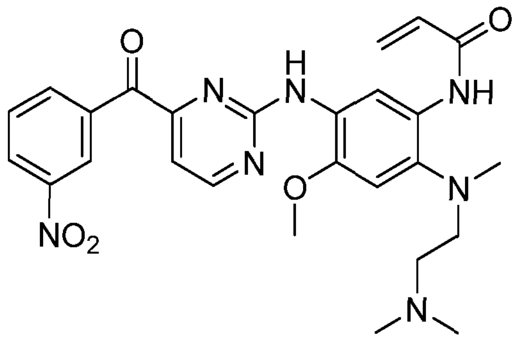 | N-(2-((2-(dimethylamino) ethyl)(methyl)amino)-4-methoxy-5-((4-(3-nitrobenzoyl)pyrimidin-2-yl) amino)phenyl)acrylamide | EGFR_3365_34 |

(continued)

| Formula | Name | Code |
|---|---|---|
| 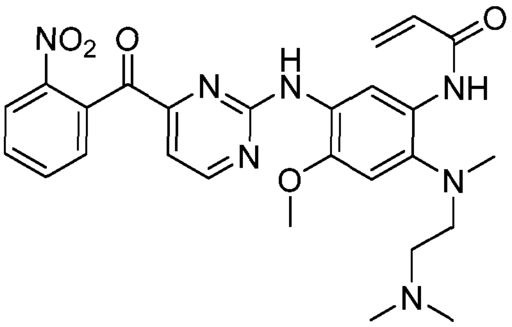 | N-(2-((2-(dimethylamino) ethyl)(methyl)amino)-4-methoxy-5-((4-(2-nitrobenzoyl)pyrimidin-2-yl) amino)phenyl)acrylamide | EGFR_3365_36 |
| 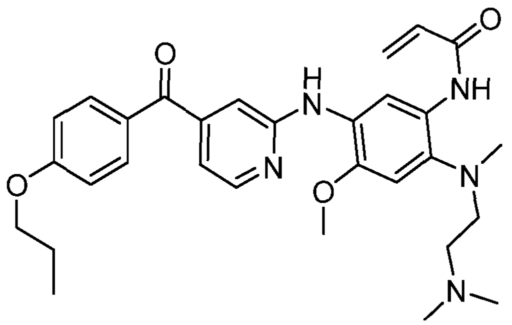 | N-(2-((2-(dimethylamino) ethyl)(methyl)amino)-4-methoxy-5-((4-(4-propoxybenzoyl)pyridin-2-yl) amino)phenyl)acrylamide | EGFR_3365_50 |
| 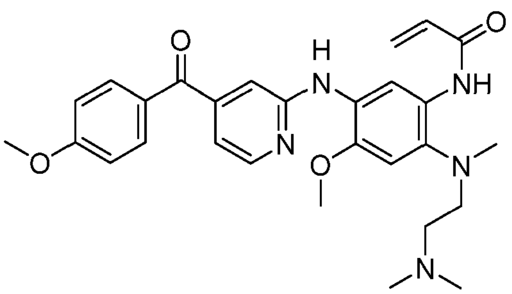 | N-(2-((2-(dimethylamino) ethyl)(methyl)amino)-4-methoxy-5-((4-(4-methoxybenzoyl)pyridin-2-yl)amino)phenyl)acrylamide | EGFR_3365_51 |
| 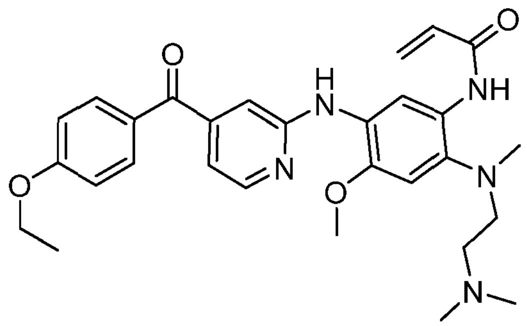 | N-(2-((2-(dimethylamino) ethyl)(methyl)amino)-5-((4-(4-ethoxybenzoyl)pyridin-2-yl) amino)-4-methoxyphenyl) acrylamide | EGFR_3365_52 |
| 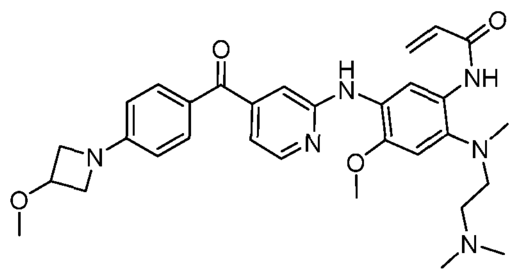 | N-(2-((2-(dimethylamino) ethyl)(methyl)amino)-4-methoxy-5-((4-(4-(3-methoxyazetidin-1-yl) benzoyl)pyridin-2-yl)amino) phenyl)acrylamide | EGFR_3365_53 |

(continued)

| Formula | Name | Code |
|---|---|---|
| 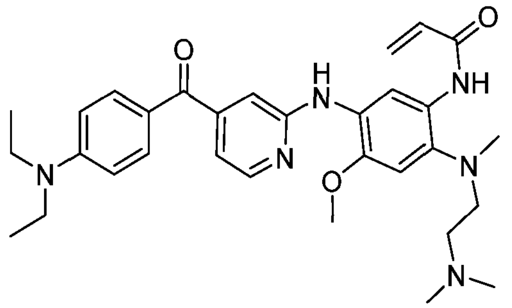 | N-(5-((4-(4-(diethylamino) benzoyl)pyridin-2-yl) amino)-2-((2-(dimethyl amino) ethyl)(methyl) amino)-4-methoxyphenyl)acrylamide | EGFR_ 3365 _54 |
| 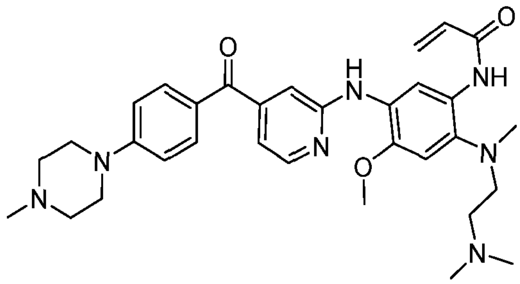 | N-(2-((2-(dimethylamino) ethyl)(methyl)amino)-4-methoxy-5-((4-(4-(4-methylpiperazin-1-yl) benzoyl)pyridin-2-yl) amino)phenyl)acrylamide | EGFR_ 3365 _55 |
| 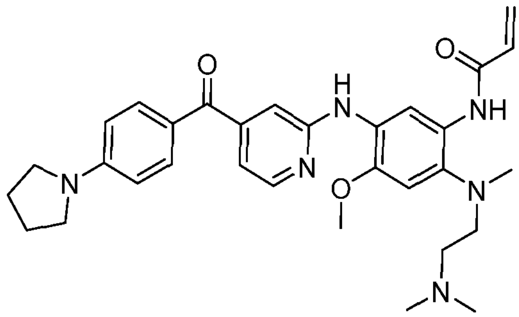 | N-(2-((2-(dimethylamino) ethyl)(methyl)amino)-4-methoxy-5-((4-(4-(pyrrolidin-1-yl)benzoyl) pyridin-2-yl)amino)phenyl)acrylamide | EGFR_ 3365 _56 |
| 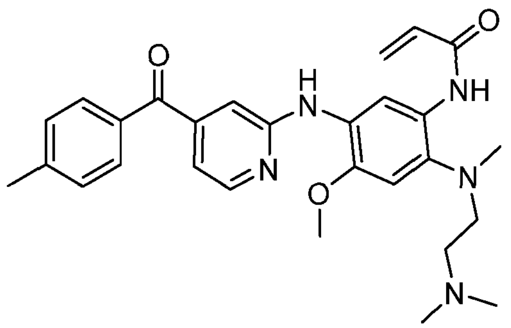 | N-(2-((2-(dimethylamino) ethyl)(methyl)amino)-4-methoxy-5-((4-(4-methylbenzoyl)pyridin-2-yl) amino)phenyl)acrylamide | EGFR_ 3365 _57 |
| 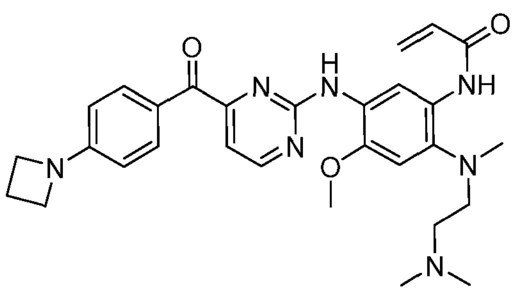 | N-(5-((4-(4-(azetidin-1-yl) benzoyl)pyrimidin-2-yl)amino)-2-((2-(dimethylamino) ethyl)(methyl) amino)-4-methoxyphenyl)acrylamide | EGFR_ 3365 _58 |

(continued)

| Formula | Name | Code |
|---|---|---|
| 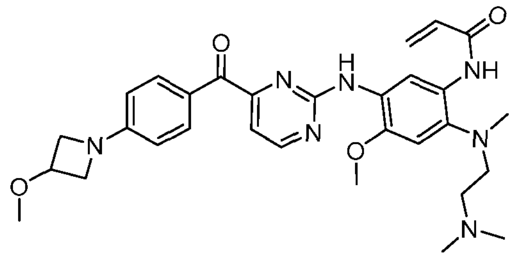 | N-(2-((2-(dimethylamino) ethyl)(methyl)amino)-4-methoxy-5-((4-(4-(3-methoxyazetidin-1-yl) benzoyl)pyrimidin-2-yl) amino)phenyl) acrylamide | EGFR_3365_61 |
| 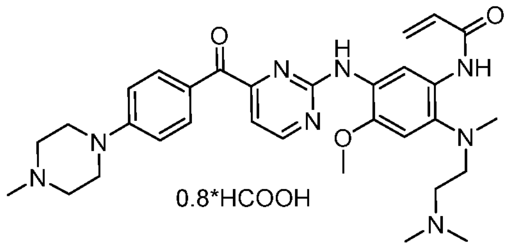 0.8*HCOOH | N-(2-((2-(dimethylamino) ethyl)(methyl)amino)-4-methoxy-5-((4-(4-(4-methylpiperazin-1-yl) benzoyl)pyrimidin-2-yl)amino) phenyl) acrylamide formate | EGFR_3365_62 |
| 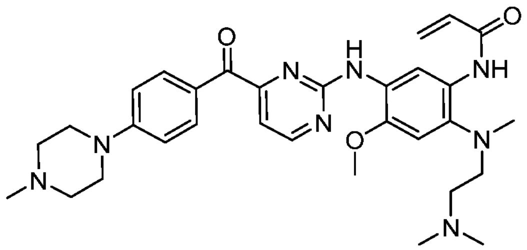 | N-(2-((2-(dimethylamino) ethyl)(methyl)amino)-4-methoxy-5-((4-(4-(4-methylpiperazin-1-yl) benzoyl)pyrimidin-2-yl)amino) phenyl) acrylamide | EGFR_3365_62a |
| 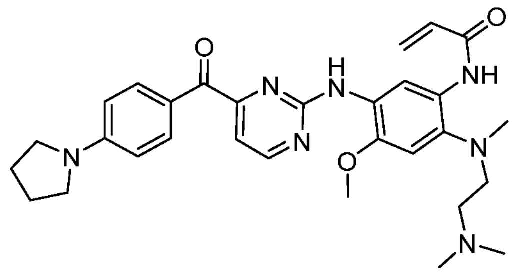 | N-(2-((2-(dimethylamino) ethyl)(methyl)amino)-4-methoxy-5-((4-(4-(pyrrolidin-1-yl)benzoyl) pyrimidin-2-yl) amino)phenyl)acrylamide | EGFR_3365_63 |
| 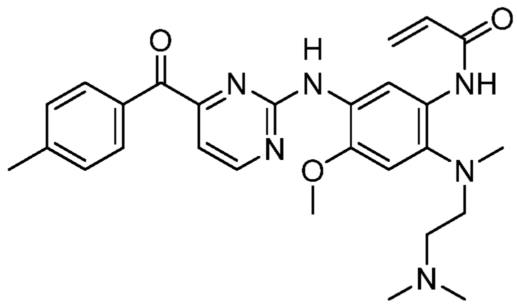 | N-(2-((2-(dimethylamino) ethyl)(methyl)amino)-4-methoxy-5-((4-(4-methylbenzoyl)pyrimidin-2-yl) amino)phenyl)acrylamide | EGFR_3365_64 |

(continued)

| Formula | Name | Code |
|---|---|---|
| 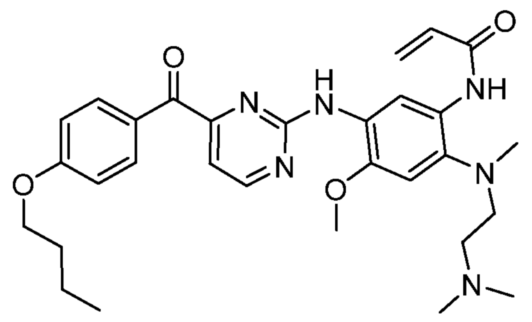 | N-(5-((4-(4-butoxybenzoyl) pyrimidin-2-yl) amino)-2-((2-(dimethylamino) ethyl)(methyl) amino)-4-methoxyphenyl)acrylamide | EGFR_ 3365 _66 |
| 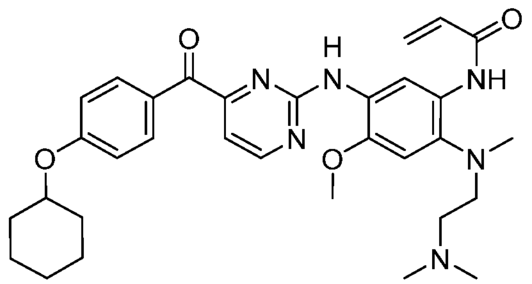 | N-(5-((4-(4-(cyclohexyloxy) benzoyl)pyrimidin-2-yl)amino)-2-((2-(dimethylamino) ethyl)(methyl) amino)-4-methoxyphenyl)acrylamide | EGFR_ 3365 _67 |
| 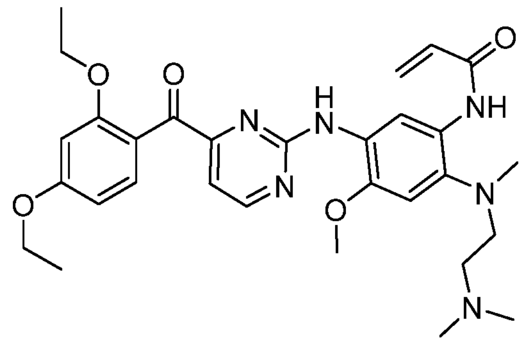 | N-(5-((4-(2,4-diethoxybenzoyl) pyrimidin-2-yl) amino)-2-((2-(dimethylamino)ethyl) (methyl) amino)-4-methoxyphenyl)acrylamide | EGFR_ 3365 _68 |
| 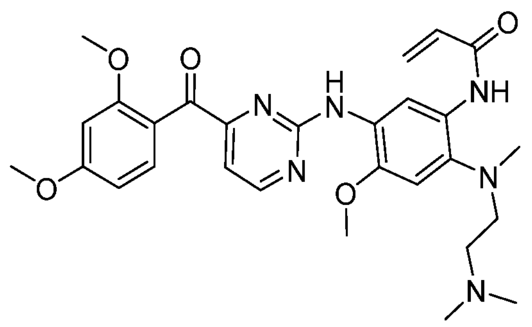 | N-(5-((4-(2,4-dimethoxy benzoyl)pyrimidin-2-yl) amino)-2-((2-(dimethylamino) ethyl)(methyl) amino)-4-methoxyphenyl)acrylamide | EGFR_ 3365 _69 |

(continued)

| Formula | Name | Code |
|---|---|---|
| 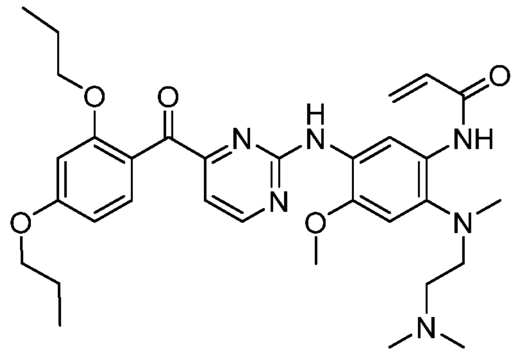 | N-(2-((2-(dimethylamino) ethyl)(methyl)amino)-5-((4-(2,4-dipropoxybenzoyl) pyrimidin-2-yl) amino)-4-methoxyphenyl)acrylamide | EGFR_3365_70 |
| 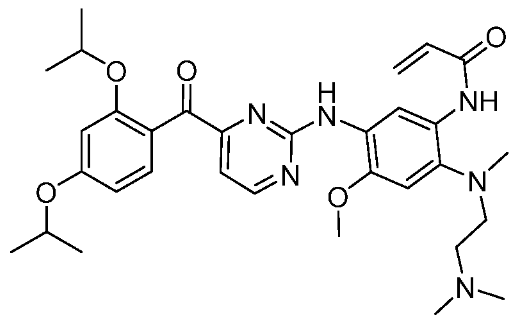 | N-(5-((4-(2,4-diisopropoxy benzoyl)pyrimidin-2-yl)amino)-2-((2-(dimethyl amino) ethyl)(methyl) amino)-4-methoxyphenyl)acrylamide | EGFR_3365_71 |
| 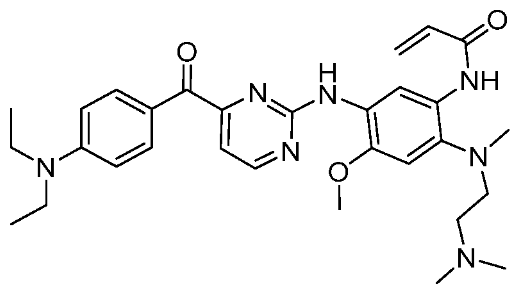 | N-(5-((4-(4-(diethylamino) benzoyl)pyrimidin-2-yl)amino)-2-((2-(dimethylamino) ethyl)(methyl) amino)-4-methoxyphenyl)acrylamide | EGFR_3365_72 |
| 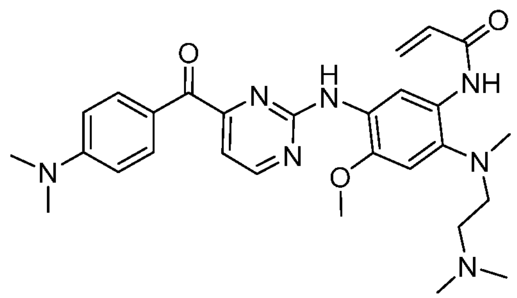 | N-(5-((4-(4-(dimethylamino) benzoyl)pyrimidin-2-yl)amino)-2-((2-(dimethylamino)ethyl) (methyl) amino)-4-methoxyphenyl)acrylamide | EGFR_3365_73 |
| 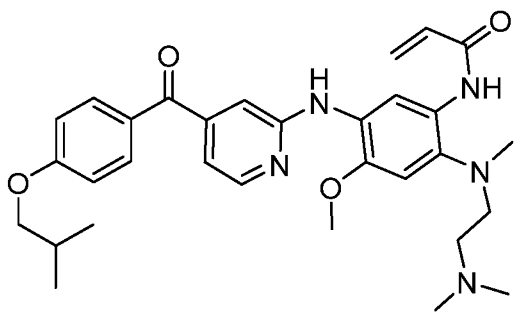 | N-(2-((2-(dimethylamino) ethyl)(methyl)amino)-5-((4-(4-isobutoxybenzoyl) pyridin-2-yl) amino)-4-methoxyphenyl) acrylamide | EGFR_3365_77 |

(continued)

| Formula | Name | Code |
|---|---|---|
| 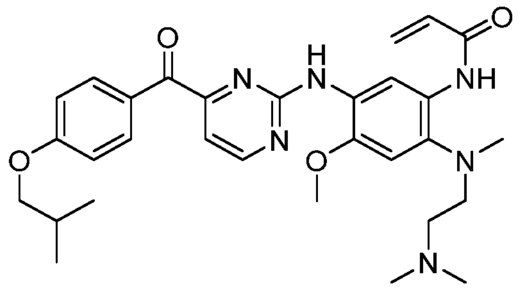 | N-(2-((2-(dimethylamino) ethyl)(methyl)amino)-5-((4-(4-isobutoxybenzoyl)pyrimidin-2-yl) amino)-4-methoxyphenyl) acrylamide | EGFR_3365_78 |
| 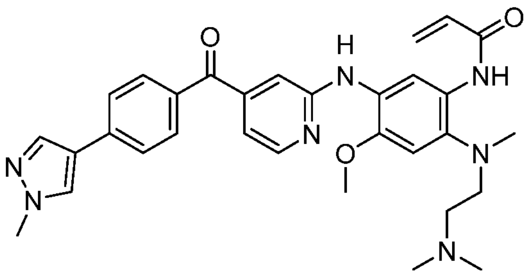 | N-(2-((2-(dimethylamino) ethyl)(methyl)amino)-4-methoxy-5-((4-(4-(1-methyl-1H-pyrazol-4-yl) benzoyl) pyridin-2-yl)amino)phenyl) acrylamide | EGFR_3365_85 |
| 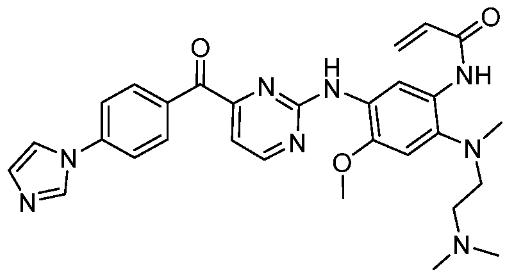 | N-(5-((4-(4-(1H-imidazol-1-yl) benzoyl)pyrimidin-2-yl)amino)-2-((2-(dimethylamino)ethyl) (methyl) amino)-4-methoxyphenyl)acrylamide | EGFR_3365_86 |
| 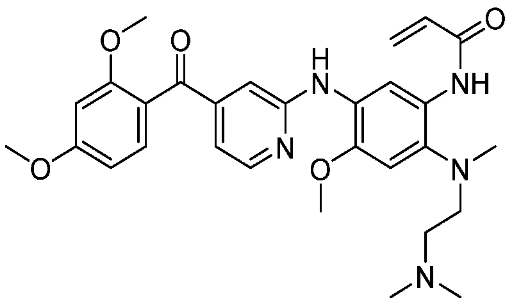 | N-(5-((4-(2,4-dimethoxy benzoyl)pyridin-2-yl) amino)-2-((2-(dimethylamino) ethyl)(methyl) amino)-4-methoxyphenyl)acrylamide | EGFR_3365_87 |
| 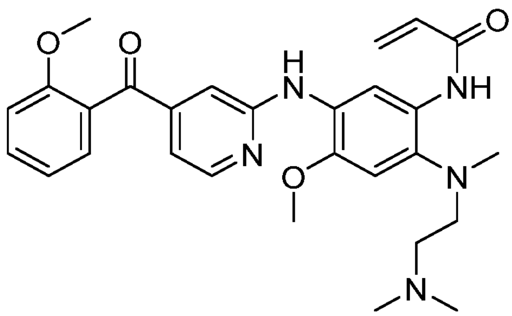 | N-(2-((2-(dimethylamino) ethyl)(methyl)amino)-4-methoxy-5-((4-(2-methoxybenzoyl)pyridin-2-yl) amino)phenyl)acrylamide | EGFR_3365_88 |

(continued)

| Formula | Name | Code |
|---|---|---|
| 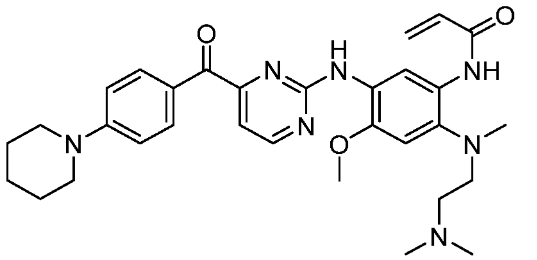 | N-(2-((2-(dimethylamino) ethyl)(methyl)amino)-4-methoxy-5-((4-(4-(piperidin-1-yl)benzoyl) pyrimidin-2-yl)amino)phenyl)acrylamide | EGFR_3365_90 |
| 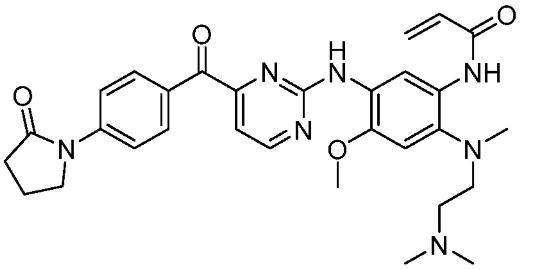 | N-(2-((2-(dimethylamino) ethyl)(methyl)amino)-4-methoxy-5-((4-(4-(2-oxopyrrolidin-1-yl) benzoyl)pyrimidin-2-yl) amino)phenyl) acrylamide | EGFR_3365_91 |
| 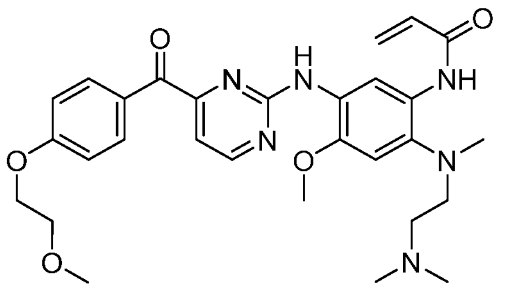 | N-(2-((2-(dimethylamino) ethyl)(methyl)amino)-4-methoxy-5-((4-(4-(2-methoxyethoxy)benzoyl) pyrimidin-2-yl)amino)phenyl) acrylamide | EGFR_3365_92 |
| 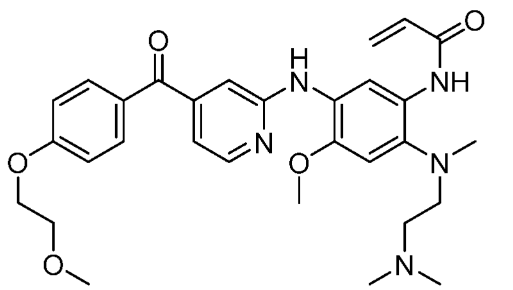 | N-(2-((2-(dimethylamino) ethyl)(methyl)amino)-4-methoxy-5-((4-(4-(2-methoxyethoxy)benzoyl) pyridin-2-yl)amino)phenyl) acrylamide | EGFR_3365_93 |
| 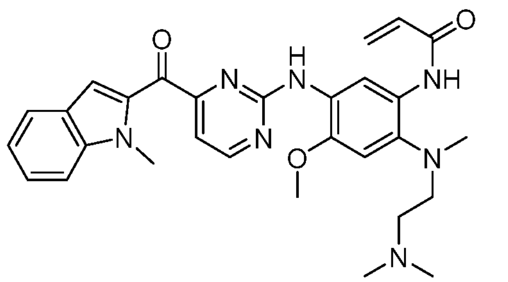 | N-(2-((2-(dimethylamino)ethyl) (methyl)amino)-4-methoxy-5-((4-(1-methyl-1H-indole-2-carbonyl)pyrimidin-2-yl) amino)phenyl) acrylamide | EGFR_3365_94 |

(continued)

| Formula | Name | Code |
|---|---|---|
| 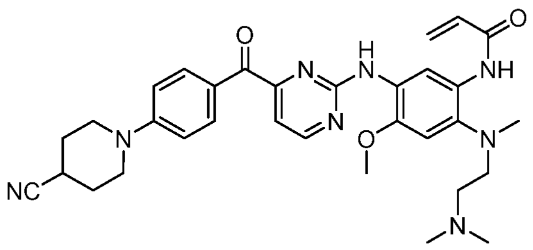 | N-(5-((4-(4-(4-cyanopiperidin-1-yl)benzoyl) pyrimidin-2-yl) amino)-2-((2-(dimethylamino) ethyl)(methyl)amino)-4-methoxyphenyl) acrylamide | EGFR_3365_97 |
| 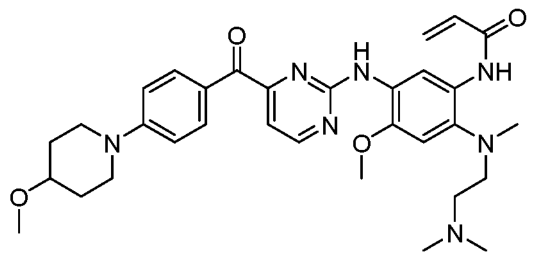 | N-(2-((2-(dimethylamino) ethyl)(methyl)amino)-4-methoxy-5-((4-(4-(4-methoxypiperidin-1-yl) benzoyl)pyrimidin- 2-yl) amino)phenyl) acrylamide | EGFR_3365_98 |
| 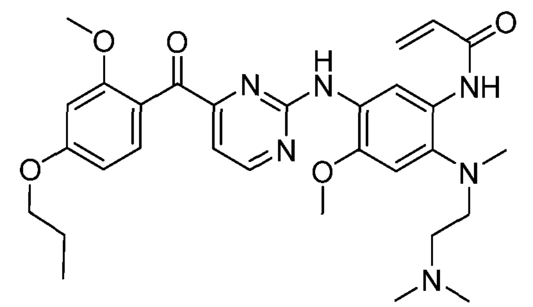 | N-(2-((2-(dimethylamino) ethyl)(methyl)amino)-4-methoxy-5-((4-(2-methoxy-4-propoxybenzoyl) pyrimidin-2-yl)amino)phenyl)acrylamide | EGFR_3365_101 |
| 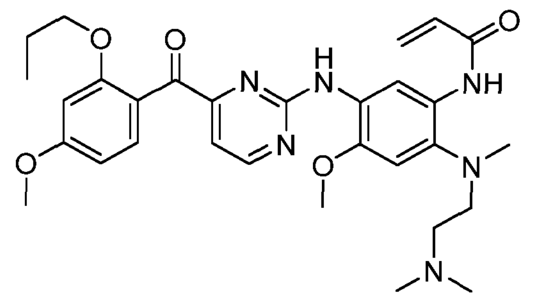 | N-(2-((2-(dimethylamino) ethyl)(methyl)amino)-4-methoxy-5-((4-(4-methoxy-2-propoxybenzoyl) pyrimidin-2-yl)amino)phenyl)acrylamide | EGFR_3365_102 |
| 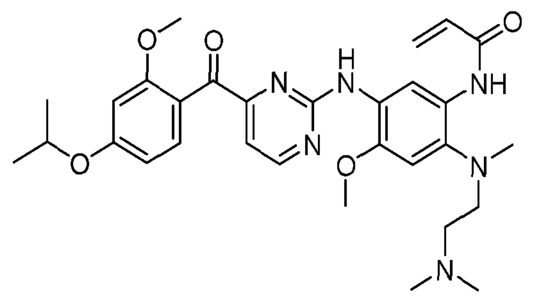 | N-(2-((2-(dimethylamino) ethyl)(methyl)amino)-5-((4-(4-isopropoxy-2-methoxybenzoyl) pyrimidin-2-yl)amino)-4-methoxyphenyl) acrylamide | EGFR_3365_103 |

(continued)

| Formula | Name | Code |
|---|---|---|
| 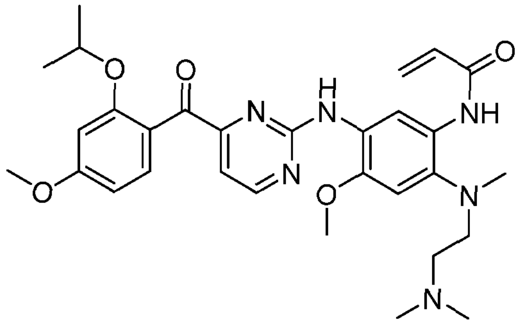 | N-(2-((2-(dimethylamino) ethyl)(methyl)amino)-5-((4-(2-isopropoxy -4-methoxybenzoyl) pyrimidin-2-yl)amino)-4-methoxyphenyl) acrylamide | EGFR_3365_104 |
| 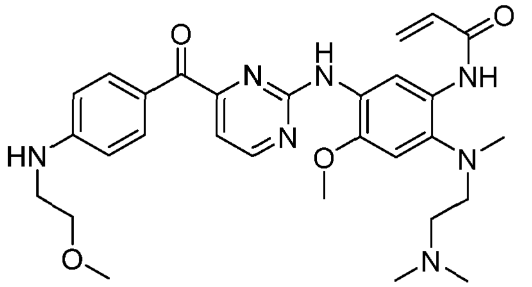 | N-(2-((2-(dimethylamino) ethyl)(methyl)amino)-4-methoxy-5-((4-(4-((2-methoxyethyl)amino) benzoyl) pyrimidin-2-yl)amino) phenyl) acrylamide | EGFR_3365_105 |
| 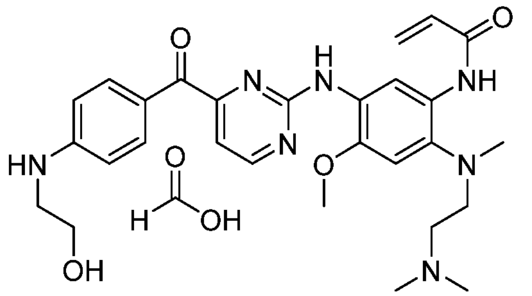 | N-(2-((2-(dimethylamino) ethyl)(methyl)amino)-5-((4-(4-((2-hydroxyethyl)amino) benzoyl) pyrimidin-2-yl)amino)-4-methoxyphenyl) acrylamide formate | EGFR_3365_106 |
| 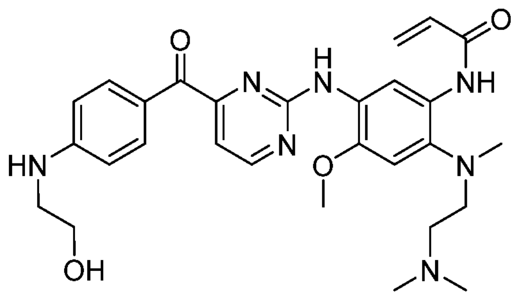 | N-(2-((2-(dimethylamino) ethyl)(methyl)amino)-5-((4-(4-((2-hydroxyethyl)amino) benzoyl) pyrimidin-2-yl)amino)-4-methoxyphenyl) acrylamide | EGFR_3365_106a |
| 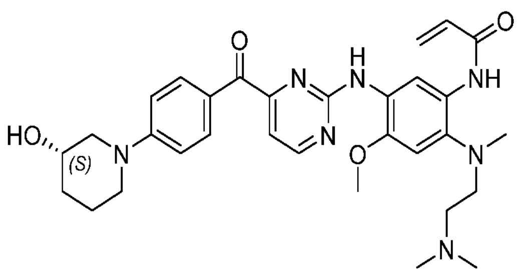 | (S)-N-(2-((2-(dimethylamino) ethyl)(methyl) amino)-5-((4-(4-(3 -hydroxypiperidin- 1 -yl) benzoyl)pyrimidin-2-yl)amino)-4-methoxyphenyl)acrylamide | EGFR_3365_108 |

(continued)

| Formula | Name | Code |
|---|---|---|
| 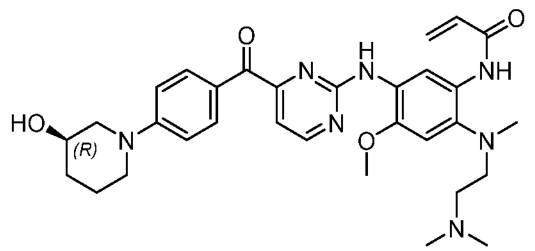 | (R)-N-(2-((2-(dimethylamino) ethyl)(methyl) amino)-5-((4-(4-(3 -hydroxypiperidin- 1 -yl) benzoyl)pyrimidin-2-yl)amino)-4-methoxyphenyl)acrylamide | EGFR_3365_109 |
| 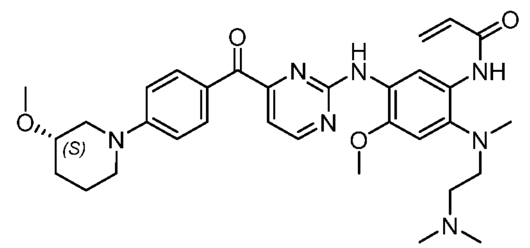 | (S)-N-(2-((2-(dimethylamino) ethyl)(methyl) amino)-4-methoxy-5-((4-(4-(3-methoxypiperidin-1-yl) benzoyl)pyrimidin-2-yl) amino)phenyl) acrylamide | EGFR_3365_110 |
| 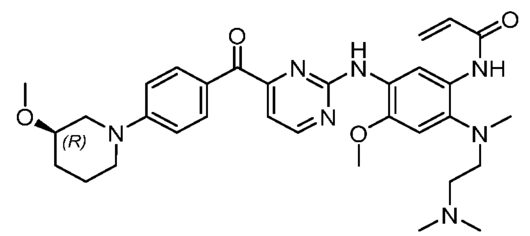 | (R)-N-(2-((2-(dimethylamino) ethyl)(methyl) amino)-4-methoxy-5-((4-(4-(3-methoxypiperidin-1-yl) benzoyl)pyrimidin- 2-yl) amino)phenyl) acrylamide | EGFR_3365_111 |
| 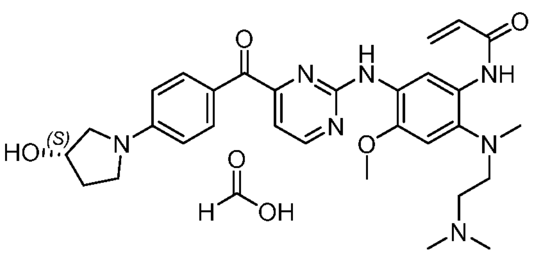 | (S)-N-(2-((2-(dimethylamino) ethyl)(methyl) amino)-5-((4-(4-(3 -hydroxypyrrolidin- 1 -yl) benzoyl)pyrimidin-2-yl)amino)-4-methoxyphenyl)acrylamide formate | EGFR_3365_112 |
| 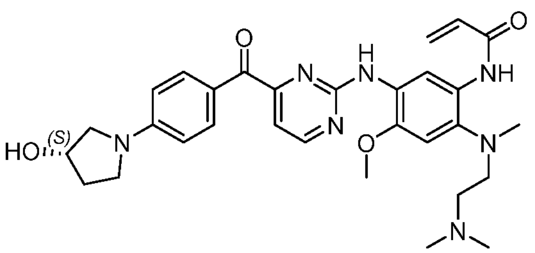 | (S)-N-(2-((2-(dimethylamino) ethyl)(methyl) amino)-5-((4-(4-(3 -hydroxypyrrolidin- 1 -yl) benzoyl)pyrimidin-2-yl)amino)-4-methoxyphenyl)acrylamide | EGFR_3365_112a |
| 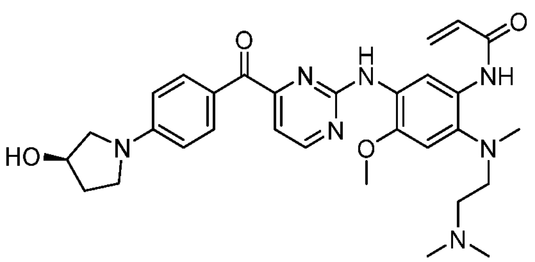 | (R)-N-(2-((2-(dimethylamino) ethyl)(methyl) amino)-5-((4-(4-(3 -hydroxypyrrolidin- 1 -yl) benzoyl)pyrimidin-2-yl)amino)-4-methoxyphenyl)acrylamide | EGFR_3365_113 |

(continued)

| Formula | Name | Code |
|---|---|---|
| 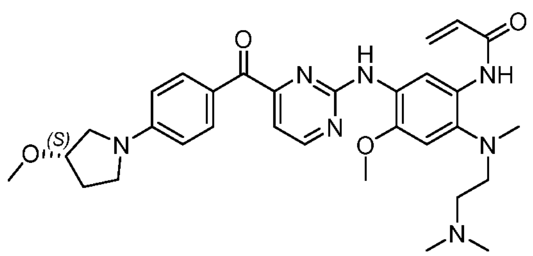 | (S)-N-(2-((2-(dimethylamino) ethyl)(methyl) amino)-4-methoxy-5-((4-(4-(3-methoxypyrrolidin-1-yl) benzoyl)pyrimidin-2-yl) amino)phenyl)acrylamide | EGFR_3365_114 |
| 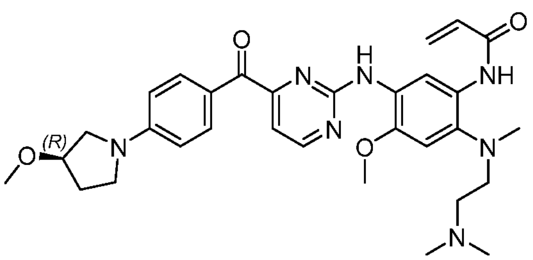 | (R)-N-(2-((2-(dimethylamino) ethyl)(methyl) amino)-4-methoxy-5-((4-(4-(3-methoxypyrrolidin-1-yl) benzoyl)pyrimidin-2-yl) amino)phenyl)acrylamide | EGFR_3365_115 |
| 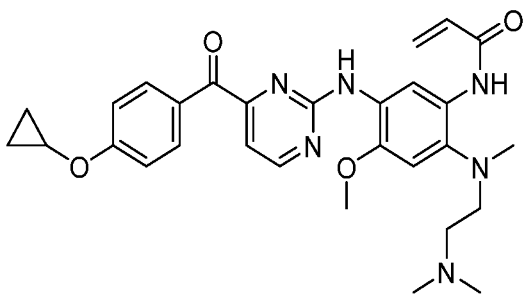 | N-(5-((4-(4-cyclopropoxy benzoyl)pyrimidin-2-yl) amino)-2-((2-(dimethylamino) ethyl)(methyl) amino)-4-methoxyphenyl)acrylamide | EGFR_3365_116 |
| 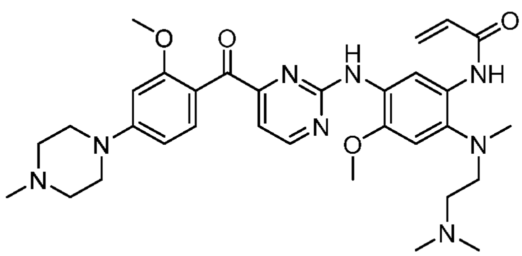 | N-(2-((2-(dimethylamino) ethyl)(methyl)amino)-4-methoxy-5-((4-(2-methoxy-4-(4-methylpiperazin-1-yl) benzoyl)pyrimidin-2-yl) amino)phenyl)acrylamide | EGFR_3365_120 |
| 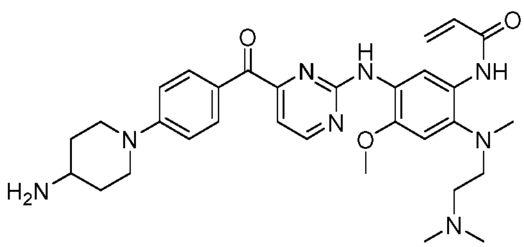 | N-(5-((4-(4-(4-aminopiperidin-1-yl)benzoyl) pyrimidin-2-yl) amino)-2-((2-(dimethylamino) ethyl)(methyl)amino)-4-methoxyphenyl) acrylamide | EGFR_3365_121 |
| 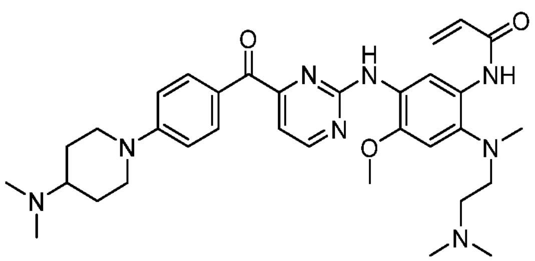 | N-(2-((2-(dimethylamino) ethyl)(methyl)amino)-5-((4-(4-(4-(dimethylamino) piperidin-1-yl) benzoyl)pyrimidin-2-yl) amino)-4-methoxyphenyl) acrylamide | EGFR_3365_121a |

(continued)

| Formula | Name | Code |
|---|---|---|
| 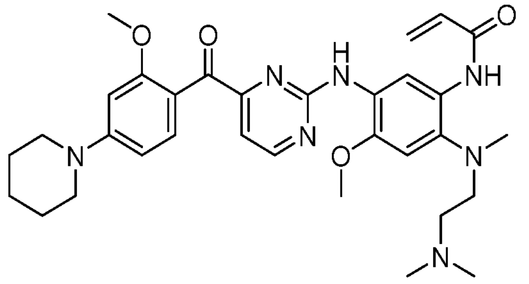 | N-(2-((2-(dimethylamino) ethyl)(methyl)amino)-4-methoxy-5-((4-(2-methoxy-4-(piperidin-1 -yl) benzoyl) pyrimidin-2-yl)amino) phenyl) acrylamide | EGFR_ 3365 _122 |
| 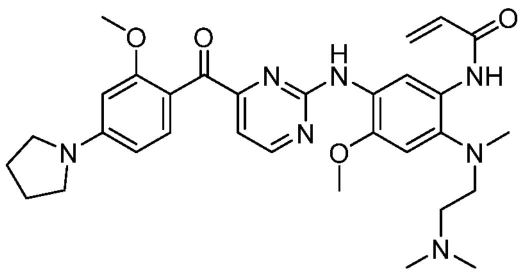 | N-(2-((2-(dimethylamino) ethyl)(methyl)amino)-4-methoxy-5-((4-(2-methoxy-4-(pyrrolidin-1-yl) benzoyl) pyrimidin-2-yl)amino) phenyl) acrylamide | EGFR_ 3365 _123 |
| 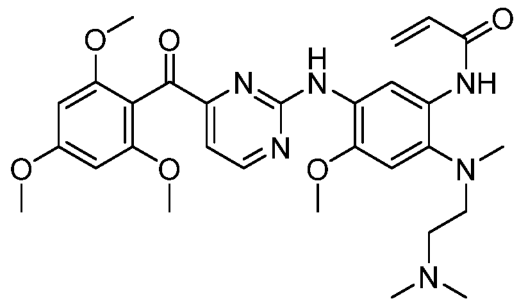 | N-(2-((2-(dimethylamino) ethyl)(methyl)amino)-4-methoxy-5-((4-(2,4,6-trimethoxybenzoyl) pyrimidin-2-yl)amino)phenyl)acrylamide | EGFR_ 3365 _124 |
| 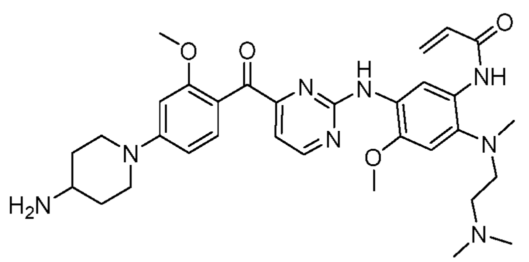 | N-(5-((4-(4-(4-aminopiperidin-1-yl)-2-methoxybenzoyl) pyrimidin-2-yl)amino)-2-((2-(dimethylamino)ethyl)(methyl) amino)-4-methoxyphenyl) acrylamide | EGFR_ 3365 _126 |
| 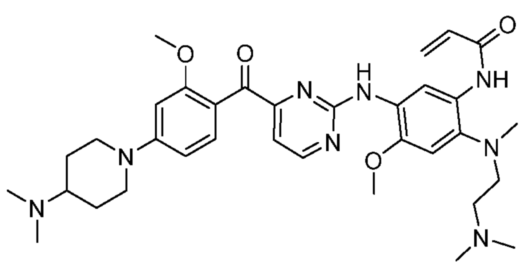 | N-(2-((2-(dimethylamino) ethyl)(methyl)amino)-5-((4-(4-(4-(dimethylamino) piperidin-1 -yl)-2-methoxybenzoyl) pyrimidin-2-yl)amino)-4-methoxyphenyl) acrylamide | EGFR_ 3365 _127 |

The present invention also relates to a method for inhibiting of biological activity of EGFR in a subject, comprising contacting EGFR with the compound described herein.

Compounds, that inhibit EGFR, can be used to manufacture drugs intended for treating any of the pathological conditions described herein, for example, compounds of formula I, pharmaceutically acceptable salts, solvates or stereoisomers will be useful in the treatment of diseases or medical conditions mediated, alone or partially, by EGFR activity, for example, oncological diseases. Examples of oncological disease that may be treated using the present compounds

include, but are not limited to, bladder cancer, ovarian cancer, cervical cancer, colorectal cancer, breast cancer, pancreatic cancer, head and neck cancer, glioma, glioblastoma, melanoma, prostate cancer, leucosis, lymphoma, non-Hodgkin lymphoma, Hodgkin's lymphoma, lung cancer (for example, non-small cell lung cancer), hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

In one embodiment, the present invention relates to a pharmaceutical composition that comprises a therapeutically effective amount of at least one of the compounds described herein, or pharmaceutically acceptable salt, solvate thereof, and one or more pharmaceutically acceptable excipients. In another one embodiment, the pharmaceutical composition comprising compounds of the present invention is intended to prevent or treat a disease or disorder mediated by the activation of EGFR.

In another one embodiment, the pharmaceutical composition comprising compounds of the present invention is intended to prevent or treat a disease or disorder mediated by the activation of EGFR with a L858R mutation and/or a T790M mutation and/or an exon 19 deletion and/or a C797S mutation.

In another one embodiment, the pharmaceutical composition comprising compounds of the present invention is intended to prevent or treat oncological disease including bladder cancer, ovarian cancer, cervical cancer, colorectal cancer, breast cancer, pancreatic cancer, head and neck cancer, glioma, glioblastoma, melanoma, prostate cancer, leucosis, lymphoma, non-Hodgkin lymphoma, Hodgkin's lymphoma, lung cancer (for example, non-small cell lung cancer), hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

In another one embodiment, the pharmaceutical composition comprising compounds of the present invention is intended to prevent or treat oncological disease, wherein the oncological disease is non-small cell lung cancer.

The pharmaceutical composition of the present invention comprises, by way of example, from about 5% to about 100% of active ingredients, preferably from about 10% to about 60% of active ingredients. It is to be understood that each dosage unit may not comprise an effective amount of an active ingredient or ingredients, because the sufficient effective amount can be achieved by multiple dosing.

A typical composition is prepared by mixing the compound described herein with a carrier, diluent or excipient. Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which compound of the present invention is being applied. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The compositions may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., compound of the invention or pharmaceutical composition thereof) or aid in the manufacturing of the pharmaceutical product (i.e., medicament). The pharmaceutical compositions should preferably be manufactured in compliance with the GMP (Good Manufacturing Practice) requirements.

The pharmaceutical compositions also may contain salts, solvates and hydrates of compounds of the present invention, or stabilized form of the compound (e.g., complex with a cyclodextrin derivative or other known complexation agent).

The pharmaceutical compositions of the invention may be formulated for an oral route administration. Oral administration is a route of administration, where a medicine is taken through the mouth, by virtue of swallowing. The compounds of the present invention may also be administered by buccal, lingual, or sublingual route by which the compound enters the blood stream directly from the mouth.

Formulations suitable for oral, buccal, lingual, or sublingual administration include solid, semi-solid and liquid systems such as tablets; granules; soft or hard capsules containing multi- or nano-particulates, liquids, or powders; lozenges (including liquid-filled); chews; gels; fast dispersing dosage forms; films; ovules; sprays; and buccal/mucoadhesive patches. More preferred formulations for oral administration are tablets, granules and capsules.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The pharmaceutical compositions of the invention could be used for parenteral administration. As used herein,

"parenteral administration" of a pharmaceutical composition includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intravenous, intraarterial, intrathecal, intraventricular, intraurethral, intracranial, intrasynovial injection or infusions; and kidney dialytic infusion techniques. Intratumoral delivery, e.g. intratumoral injection, may also be advantageous. Regional perfusion is also contemplated.

Formulations of a pharmaceutical composition suitable for parenteral administration typically comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules or in multi-dose containers containing a preservative. Formulations for parenteral administration include, but are not limited to, suspensions, solutions, emulsions in oily or aqueous vehicles, pastes, and the like.

The compounds of the invention can also be administered intranasally or by inhalation, typically in the form of a dry powder (either alone, as a mixture, or as a mixed component particle, for example, mixed with a suitable pharmaceutically acceptable excipient) from a dry powder inhaler, as an aerosol spray from a pressurised container, pump, spray, atomiser (preferably an atomiser using electrohydrodynamics to produce a fine mist), or nebuliser, with or without the use of a suitable propellant, or as nasal drops.

The pressurised container, pump, spray, atomizer, or nebuliser generally contains a solution or suspension of a compound of the invention comprising, for example, a suitable agent for dispersing, solubilising, or extending release of the active, a propellant(s) as solvent.

Prior to use in a dry powder or suspension formulation, the drug product is generally micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules, blisters and cartridges for use in an inhaler or insufflator may be formulated to contain a powder mix of the compound of the invention, a suitable powder base and a performance modifier.

A suitable solution formulation for use in an atomiser using electrohydrodynamics to produce a fine mist may contain a suitable dose of the compound of the invention per actuation and the actuation volume may for example vary from 1 $\mu$L to 100 $\mu$L.

Suitable flavours, such as menthol and levomenthol, or sweeteners, such as saccharin or saccharin sodium, may be added to those formulations of the invention intended for inhaled/intranasal administration.

Formulations may be formulated to be immediate and/or modified release. Modified release formulations include delayed-, sustained-, pulsed-, controlled-, targeted and programmed release.

In one embodiment, the present invention relates to a method for treating a disease or disorder mediated by the activation of EGFR comprising administering a therapeutically effective amount of the compound described herein, or the present pharmaceutical composition in a subject in need thereof.

In another one embodiment, the present invention relates to the method for treating, described herein, wherein the disease or disorder is the disease or disorder mediated by the activation of EGFR with a L858R mutation and/or a T790M mutation and/or an exon 19 deletion and/or a C797S mutation.

In another one embodiment, the present invention relates to the method for treating, described herein, wherein the disease or disorder mediated by the activation of EGFR is oncological disease. In another one embodiment, the present invention relates to the method for treating, described herein, wherein oncological disease is selected from the group comprising bladder cancer, ovarian cancer, cervical cancer, colorectal cancer, breast cancer, pancreatic cancer, head and neck cancer, glioma, glioblastoma, melanoma, prostate cancer, leucosis, lymphoma, non-Hodgkin lymphoma, Hodgkin's lymphoma, lung cancer (for example, non-small cell lung cancer), hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

In another one embodiment, the present invention relates to the method for treating, described herein, wherein oncological disease is non-small cell lung cancer.

In one embodiment, the present invention relates to use of the present compound or a pharmaceutical composition described herein for the treatment of a disease or disorder mediated by the activation of EGFR in a subject in need thereof.

In one embodiment, the present invention relates to the use described herein, wherein the disease or disorder is the disease or disorder mediated by the activation of EGFR with a L858R mutation and/or a T790M mutation and/or

an exon 19 deletion and/or a C797S mutation.

In one embodiment, the present invention relates to the use described herein, wherein the disease or disorder mediated by the activation of EGFR is oncological disease. In one embodiment, the present invention relates to the use described herein, wherein oncological disease is selected from the group comprising bladder cancer, ovarian cancer, cervical cancer, colorectal cancer, breast cancer, pancreatic cancer, head and neck cancer, glioma, glioblastoma , melanoma, prostate cancer, leucosis, lymphoma, non-Hodgkin lymphoma, Hodgkin's lymphoma, lung cancer (for example, non-small cell lung cancer), hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

In one embodiment, the present invention relates to the use described herein, wherein oncological disease is non-small cell lung cancer.

The compounds of the invention may be administered alone or in combination with one or more other preparations or antibodies (or any combination thereof). Thus, the pharmaceutical compositions, methods and uses of the invention also encompass embodiments of combinations (co-administration) with other active agents.

As used herein, the terms "co-administration", "co-administered" and "in combination with" referring to the compounds with one or more other therapeutic agents, is intended to mean, and does refer to and include the following:

- simultaneous administration of such combination of compound of the invention and therapeutic agent(s) to a patient in need of treatment, when such components are formulated together into a single dosage form which releases said components at substantially the same time to said patient,
- substantially simultaneous administration of such combination of compound of the invention and therapeutic agent(s) to a patient in need of treatment, when such components are formulated apart from each other into separate dosage forms which are taken at substantially the same time by said patient, whereupon said components are released at substantially the same time to said patient,
- sequential administration of such combination of compound of the invention and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound of the invention and therapeutic agent(s) to a patient in need of treatment, when such components are formulated together into a single dosage form which releases said components in a controlled manner whereupon they are concurrently, consecutively, and/or overlappingly released at the same and/or different times to said patient, where each part may be administered by either the same or a different route.

As well known to those skilled in the art, therapeutically effective dosages may vary when the drugs are used in combination treatment. Methods for experimentally determining therapeutically effective dosages of drugs and other agents for use in combination treatment regimens are described in the literature. For example, the use of metronomic dosing, i.e., providing more frequent, lower doses in order to minimize toxic side effects, has been described in the literature. Combination treatment further includes periodic treatments that start and stop at various times to assist with the clinical management of the patient. For combination therapies described herein, dosages of the co-administered compounds will of course vary depending on the type of co-drug employed, on the specific drug employed, on the condition or disorder being treated and so forth.

The antitumor treatment described above can be used either as a stand-alone therapy, or in combination with surgery, or radiotherapy, or drug therapy. Such therapy may be administered concurrently, simultaneously, sequentially or separately with treatment with a compound of the invention and may include one or more of the following categories of anti-tumour substances: antiproliferative/antineoplastic drugs and combinations thereof, as used in medical oncology, such as alkylating agents (for example, cisplatin, oxaliplatin, carboplatin, cyclophosphamide, chlormethine, melphalan, chlorambucil, busulfan, treosulfan, temozolomide, bendamustine, prospidine, spirobromine, prednimustine, estramustine, paphencyl, lofenal, ifosfamide, mafosfamide, trofosfamide, glufosfamide and nitrosoureas, including carmustine, lomustine, nimustine, fotemustine, aranose, streptozocin); antimetabolites (for example, gemcitabine, fluorouracil, floxuridine, tegafur, raltitrexed, methotrexate, trimetrexate, pemetrexed, pralatrexate, calcium levofolinate, cytosine arabinoside, hydroxyurea, azathioprine, cladribine, fludarabine, pentostatin, mercaptopurine, nelarabine, thioguanine, fopurin, azacitidine, capecitabine, fludarabine, cladribine, nelarabine, azathioprine, clofarabine, cytarabine, enocitabine, carmofur, gemcitabine, sapacitabine, elacytarabine, doxifiuridine); anticancer antibiotics (for example, bleomycin, doxorubicin, daunomycin, epirubicin, idarubicin, mitomycin, dactinomycin, mitramycin, daunurobicin, carubicin, epirubicin, valrubicin, zorubicin, aclarubicin, pirarubicin, nemorubicin, amrubicin, zinostatin, streptozocin, митоксантрон ); antimitotic agents (for example, vinca alkaloids, such as vincristine, vinblastine, vinflunine, vindesine and vinorelbine, taxoids, such

as paclitaxel and docetaxel, cabazitaxel, tezetaxel, polo kinase inhibitors); and topoisomerase inhibitors (for example, epipodophyllotoxins, such as etoposide and teniposide, amsacrine, topotecan, irinotecan, belotecan, voreloxin, amonafide and camptothecin); cytostatic agents such as anti-estrogens (for example, tamoxifen, clostilbegyt, fulvestrant, toremifene, raloxifene, droloxifene and iodoxifen), antiandrogens (for example, bicalutamide, flutamide, nilutamide, topilutamide, enzalutamide and cyproterone acetate, chlormadinone), luteinizing hormone-releasing hormone (LHRH) antagonists or LHRH agonists (for example, goserelin, leuprorelin and buserelin), progestogens (for example, chlormadinone, gestonorone caproate, medroxyprogesterone, megestrol acetate), aromatase inhibitors (for example, anastrozole, letrozole, vorazole and exemestane) and inhibitors of 5$\alpha$-reductase (for example, finasteride, dutasteride, epristeride); anti-invasive agents (for example, c-Src family kinase inhibitors (for example, saracatinib, dasatinib and bosutinib), metalloproteinase inhibitors (for example, marimastat), inhibitors of urokinase activator receptor function (for example, plasminogen or anti-heparanase antibodies); growth factor inhibitors: for example, such inhibitors include anti-growth factor antibodies and anti-growth factor receptor antibodies (for example, trastuzumab, panitumumab, cetuximab, and any anti-growth factor/anti-growth factor receptor antibodies disclosed by Stern et al. Critical reviews in oncology/haematology, 2005, Vol. 54, p. 11-29); such inhibitors also include tyrosine kinase inhibitors, including inhibitors of the epidermal growth factor family (for example, EGFR tyrosine kinase inhibitors, such as gefitinib, erlotinib, canertinib (CI 1033), afatinib, osimertinib, rociletinib, icotinib, dacomitinib; erbB2 tyrosine kinase inhibitors, such as lapatinib); inhibitors of the hepatocyte growth factor family; inhibitors of the insulin-like growth factor family; inhibitors of the platelet-derived growth factor family, such as imatinib, nilotinib; serine/threonine-kinase inhibitors (for example, Ras/Raf pathway inhibitors, such as farnesyl transferase inhibitors, for example sorafenib, tipifarnib, and lonafarnib), MEK- and/or AKT-kinase pathway inhibitors, c-Kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (for example, barasertib (AZD1152), danusertib (PHA-739358), tozasertib (VX-680), MLN8054, R763, MP235, MP529, VX-528 and AX39459) and cyclin dependent kinase inhibitors such as CDK2 and/or CDK4 inhibitors; antiangiogenic agents such as those which inhibit the effects of vascular endothelial growth factor (for example, bevacizumab, vandetanib, vatalanib, sunitinib, axitinib, pazopanib, crizotinib and cediranib (AZD2171), linomide, integrin avp3 function inhibitors, angiostatin, endostatin, thalidomide, everolimus, sirolimus, itraconazole, suramin, semaxanib, thrombospondin, ramucirumab, tasquinimod, ranibizumab, sorafenib, compounds disclosed in international applications WO 97/22596, WO 97/30035, WO 97/32856 and WO 98/13354); vascular-damaging agents (for example, combretastatin A4, ombrabulin, and compounds disclosed in international applications WO 99/02166, WO 00/40529, WO 00/41669, WO 01/92224, WO 02/04434 and WO 02/08213; an endothelin receptor antagonist (for example, bosentan, sitaxentan, ambrisentan, BQ-123, BQ-788, macitentan, tezosentan, zibotentan, atrasentan); antisense therapies (for example, those which are directed to the targets listed above, such as ISIS 2503, anti-ras antisense, anti-EGFR antisense, custirsen, apatorsen, ISIS-STAT3Rx (ISIS 481464/AZD9150), ISIS-ARRx (AZD5312), Trabedersen (AP 12009), EZN-2968, LErafAON-ETU); gene therapy approaches, including, for example, approaches to replace aberrant genes (for example, aberrant p53 or aberrant BRCA1 or BRCA2, GDEPT (gene-directed enzyme pro-drug therapy) approaches, such as those using cytosine deaminase, thymidine kinase or a bacterial nitroreductase enzyme), and approaches to increase patient tolerance to chemotherapy or radiotherapy such as multi-drug resistance gene therapy; and immunotherapy approaches, including, for example, checkpoint inhibitors, such as PD-1/PD-L1 (nivolumab, pembrolizumab, atezolizumab, durvalumab, avelumab, pidilizumab, etc.), and drugs that target CTLA-4 (including ipilimumab, tremelimumab), OX-40, VISTA, ICOS, TIGIT, LAG-3, 4-1BB, GITR, CD40, CCR4, etc.; other ex-vivo and in-vivo approaches to increase the immunogenicity of patient tumour cells, such as transfection with cytokines such as interleukin 2, interleukin 4, interleukin 15 or granulocyte-macrophage colony stimulating factor, approaches to decrease T-cell anergy, approaches using transfected immune cells such as cytokine-transfected dendritic cells, approaches using cytokine-transfected tumour cell lines, approaches using anti-idiotypic antibodies, approaches to reduce functions of immunosuppressive cells, such as regulatory T-cells, myeloid suppressor cells or IDO (indoleamine 2,3-deoxygenase)-expressing dendritic cells, and approaches using cancer vaccines consisting of proteins or peptides derived from tumour-associated antigens such as NY-ESO-1, MAGE-3, WT1 or Her2/neu.

Thus, according to another embodiment of the invention, there is provided a pharmaceutical product comprising a compound of formula I or pharmaceutically acceptable salt, solvate or stereoisomer thereof as defined hereinbefore, in combination with an anti-tumour substance as defined hereinbefore, intended for the conjoint treatment of cancer.

Dosage regimens may be adjusted to provide the optimum desired response. For example, a single dose may be administered, several divided doses may be administered over time or the dose may be proportionally reduced or increased as indicated by the exigencies of the therapeutic situation. It is especially advantageous to formulate oral compositions in dosage unit form for ease of administration and uniformity of dosage. Dosage unit form, as used herein, refers to physically discrete units suited as unitary dosages for the patients/subjects to be treated; each unit containing a predetermined quantity of active compound calculated to produce the desired therapeutic effect in association with the required pharmaceutical carrier. Specification for the unit dosage forms of the invention is typically dictated by and directly dependent on (a) the unique characteristics of a therapeutic agent and particular therapeutic or prophylactic effect to be achieved, and (b) the limitations inherent in the art of compounding such an active compound for the treatment

of sensitivity in the subjects.

Thus, a skilled artisan would appreciate, based upon the disclosure provided herein, that the doses and dosage regimen are adjusted in accordance with methods well known in the therapeutic arts. That is, the maximum tolerable dose can be readily established, and the effective amount providing a detectable therapeutic effect to a patient may also be determined, as can the temporal requirements for administering each agent to provide a detectable therapeutic effect to a patient. Thus, while certain dose and administration regimens are exemplified herein, these examples in no way limit the doses and administration regimen that may be provided to a patient in practicing the embodiments of the invention.

It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the embodied composition. Further, the dosage regimen with the compositions of this invention may be based on a variety of factors, including the type of disease, the age, weight, sex, medical condition of the patient, the severity of the condition, the route of administration, and the particular compound employed. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Thus, the present invention encompasses intra-patient dose-escalation as determined by the person skilled in the art. Methods for determining appropriate dosages and regimens are well known in the art and would be understood by a skilled artisan once provided the ideas disclosed herein.

Generally, standard daily dosage for an adult human is in the range from 0.02 mg to 5000 mg or from about 1 mg to about 1500 mg.

Once improvement of the patient's conditions has occurred, a maintenance dose is administered, if necessary. Subsequently, the dosage or the frequency of administration, or both, can be reduced, as a function of the symptoms, to a level at which the improved disease or disorder is retained. Patients may be required periodic treatment on a long-term basis upon any relapse of symptoms.

The foregoing ranges are merely suggestive, as the number of variables in regard to an individual treatment regime is large, and considerable excursions from these recommended values are not uncommon. Such dosages may be altered depending on a number of variables, not limited to the activity of the compound used, the disorder or condition to be treated, the method of administration, the requirements of the individual subject, the severity of the disorder or condition being treated, and the judgment of the physician.

In order that this invention may be better understood, the following examples are set forth. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

## Examples

**Example 1.** Method for preparation of compound **4b.**

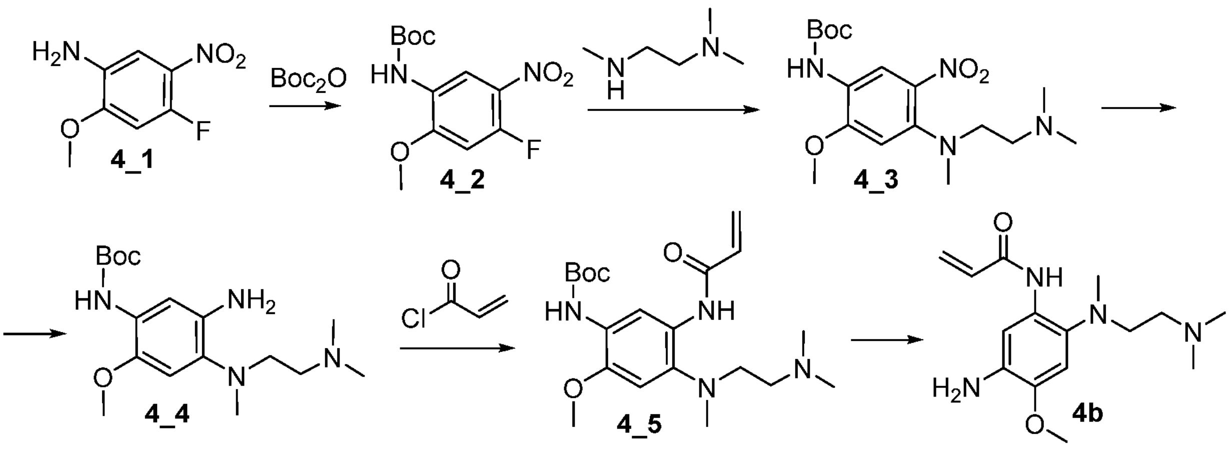

4_1 4_2 4_3 4_4 4_5 4b

**Step 1.** Preparation of compound **4_2.**

Solution of di-tert-butyl dicarbonate (16.2 g, 74.3 mmol) in 30 mL of dichloromethane was added dropwise at 0 °C for 30 minutes to a solution of 4-fluoro-2-methoxy-5-nitroaniline **4_1** (10.0 g, 53.7 mmol) and DMAP (0.33 g, 2.66 mmol) in dichloromethane (100 ml). Reaction mixture was brought to room temperature and stirred for 24 hours. Resulting mixture was concentrated under vacuum, product was isolated by column chromatography on silica gel using dichloromethane/hexane (gradient from 1:1 to 1:0) as eluent. Yield of the compound **4_2** was 6.40 g (42%).

**Step 2.** Preparation of compound **4_3.**

*N,N,N'*-trimethylethylenediamine (3.59 mL, 27.2 mmol) and DIPEA (5.24 mL, 31.3 mmol) were added to a solution of aniline **4_2** (6.10 g, 20.9 mmol) in DMF (10 mL) at room temperature. Reaction mixture was stirred at 60 °C for 2 hours. Resulting mixture was poured into water, product was extracted with ethyl acetate. The combined organic layers were washed with water and saturated NaCl solution, dried with $Na_2SO_4$, filtered and concentrated under vacuum. Yield of the compound **4_3** was 7.62 g (99%).

**Step 3.** Preparation of compound **4_4.**

Pd/C (1.30 g, 3.02 mmol) was added to a solution of compound **4_3** (7.50 g, 20.2 mmol) in methanol (90 mL) and was hydrogenated under pressure of hydrogen (2 atm) for 1 hour. Reaction mixture was filtered and concentrated under vacuum. Yield of the compound **4_4** was 6.69 g (98%).

**Step 4.** Preparation of compound **4_5.**

Solution of acryloyl chloride (3.59 mL, 27.2 mmol) in dichloromethane (80 mL) was added dropwise at 0 °C for 30 minutes to a mixture of compound **4_4** (6.69 g, 19.8 mmol) and DIPEA (3.59 mL, 27.2 mmol) in dichloromethane (150 mL). Resulting mixture was stirred at room temperature. After 3.5 hours, further amounts of acryloyl chloride (0.38 mL, 4.60 mmol) and DIPEA (0.80 mL, 4.60 mmol) were added. Reaction mixture was stirred at room temperature for 2 hours. Saturate solution of $Na_2SO_4$ was added to a resulting mixture, organic layer was washed with saturated NaCl solution, dried with $Na_2SO_4$, filtered and concentrated under vacuum. Product was isolated by column chromatography on silica gel using ethyl acetate/hexane/triethylamine (gradient from 8:2:0 to 8:2:0.05) as eluent. Yield of the compound **4_5** was 5.04 g (65%).

**Step 5.** Preparation of compound **4b.**

Compound **4_5** (5.04 g, 12.8 mmol) was dissolved in 30 mL of trifluoroacetic acid and stirred at room temperature for 1 hour. Reaction mixture was added dropwise to a saturated $Na_2CO_3$ solution, product was extracted with ethyl acetate. The combined organic layers were washed with water and saturated NaCl solution, dried on $Na_2SO_4$, filtered and concentrated under vacuum. Product was isolated by column chromatography on silica gel using ethyl acetate/hexane/triethylamine (gradient from 8:2:0 to 8:2:0.07) as eluent. Yield of the compound **4b** was 3.23 g (85%).

**Example 2.** Method of preparation of compound **EGFR_3365_3, EGFR_3365_4, EGFR_3365_50, EGFR_3365_51, EGFR_3365_52, EGFR_3365_54, EGFR_3365_56, EGFR_3365_57, EGFR_3365_77, EGFR_3365_85, EGFR_3365_87, EGFR_3365_88, EGFR_3365_93.**

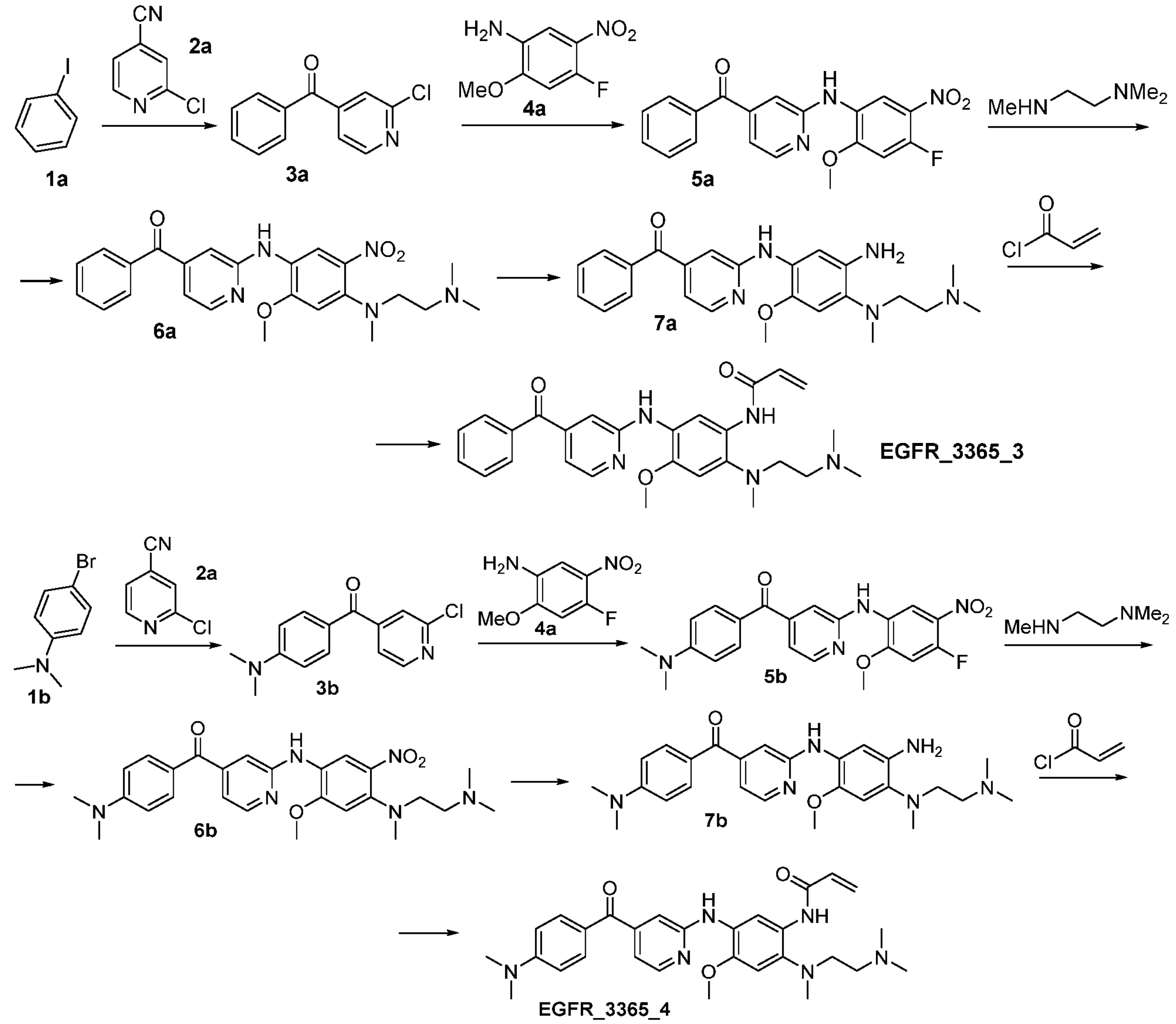
1a 2a 3a 4a 5a 6a 7a EGFR_3365_3
1b 2a 3b 4a 5b 6b 7b EGFR_3365_4

**Step 1.** Preparation of compound **3a.**

2.5 M n-Butyl lithium solution in hexane (15.9 mL, 39.7 mmol) was added dropwise under nitrogen atmosphere at -78°C for 15 minutes to a solution of iodobenzene **1a** (8.35 g, 39.7 mmol) in 350 mL of diethyl ether. Reaction mixture was brought to 0°C temperature and stirred for 30 minutes. Then, reaction mixture was cooled to -78°C and a solution of nitrile **2a** (5.00 g, 36.1 mmol) in 50 mL of diethyl ether was slowly added to it. Resulting mixture was stirred at -78°C for 1 hour, then mixture was brought to -30°C and 100 mL of 2M HCl was added. Reaction mixture was stirred for 1 hour, neutralized with 1M NaOH solution, product was extracted with ethyl acetate. The combined organic layers were dried with $Na_2SO_4$, filtered and concentrated under vacuum. Product was isolated by column chromatography on silica gel using ethyl acetate/hexane (1:4) as eluent. Yield of the compound **3a** was 7.04 g (90%).

Compound **3b** was prepared in a similar manner using the corresponding initial reagent **1b.**

**Step 2.** Preparation of compound **5a.**

$CS_2C0_3$ (0.300 g, 0.92 mmol), BINAP (0.057 g, 0.09 mmol) and aniline **4a** (0.094 g, 0.51 mmol) were added at room temperature to a solution of compound **3a** (0.100 g, 0.46 mmol) in 2 mL of 1,4-dioxane. Resulting solution was

degassed with nitrogen for 10 minutes. Then, $Pd(OAc)_2$ (0.010 g, 0.05 mmol) was added under stirring to reaction mixture. Resulting mixture was boiled under nitrogen atmosphere for 3 hours. Mixture was then diluted with 10 mL of dichloromethane and filtered through Celite. Filtrate was concentrated under vacuum, product was isolated by column chromatography on silica gel using dichloromethane as eluent. Yield of the compound 5a was 0.112 g (66%).

Compound **5b** was prepared in a similar manner using the corresponding intermediate compound **3b.**

**Step 3.** Preparation of compound **6a.**

*N*,*N*,*N'*-Trimethylethane-1,2-diamine (0.034 g, 0.33 mmol) and DIPEA (0.077 g, 0.6 mmol) were added to a solution of compound **5a** (0.110 g, 0.3 mmol) in 3 mL of dimethylformamide. Reaction mixture was stirred at room temperature for 12 hours. Resulting mixture was concentrated, product was isolated by column chromatography on silica gel using dichloromethane/ethyl acetate (8:1) as eluent with gradient of triethylamine (from 0% to 10%). Yield of the compound 6a was 0.127 g (95%).

Compound **6b** was prepared in a similar manner using the corresponding intermediate compound **5b.**

**Step 4.** Preparation of compound **7a.**

$NaHCO_3$ (0.214 g, 2.54 mmol) was added to a solution of compound **6a** (0.127 g, 0.28 mmol) in 9 mL of tetrahydrofuran/methanol/water (3:1:5) mixture. $Na_2S_2O_4$ (0.442 g, 2.54 mmol) was added in parts at 0 °C for 30 minutes to resulting mixture. Resulting mixture was stirred at room temperature for 15 minutes. Mixture was then diluted with water, product was extracted with ethyl acetate. The combined organic layers were dried with $Na_2SO_4$, filtered and concentrated under vacuum. Product was isolated by column chromatography on silica gel using dichloromethane/ethyl acetate (3:1) as eluent with gradient triethylamine (from 0.5% to 5%). Yield of the compound **7a** was 0.107 g (90%).

Compound **7b** was prepared in a similar manner using the corresponding intermediate compound **6b.**

**Step 5.** Preparation of compound **EGFR_3365_3.**

DIPEA (0.035 g, 0.27 mmol) was added to a solution of compound **7a** (0.107 g, 0.26 mmol) in 5 mL of dichloromethane. Solution of acryloyl chloride (0.024 g, 0.26 mmol) in 3 mL of dichloromethane was added dropwise at -70 °C for 1 hour to resulting mixture. Reaction mixture was brought to -30 °C and stirred at this temperature for 30 minutes. Saturated $NaHCO_3$ solution was added to resulting mixture, product was extracted with dichloromethane. The combined organic layers were washed with saturated NaCl solution, dried with $Na_2SO_4$, filtered and concentrated under vacuum. Product was isolated by column chromatography on silica gel using dichloromethane/hexane/ethyl acetate (3:1:1) as eluent with gradient triethylamine (from 0.5% to 3%). Yield of the compound **EGFR_3365_3** was 0.055 g (45%).

Compound **EGFR_3365_4** was prepared in a similar manner using the corresponding intermediate compound **7b** (product was additionally purified by preparative chromatography). Compound **EGFR_3365_4a** was prepared using repeated lyophilization of compound **EGFR_3365_4**.

Candidates **EGFR_3365_50, EGFR_3365_51, EGFR_3365_52, EGFR_3365_54, EGFR_3365_56, EGFR_3365_57, EGFR_3365_77, EGFR_3365_85, EGFR_3365_87, EGFR_3365_88, EGFR_3365_93** were prepared in a similar manner to compound **5a** using aniline **4b** instead of **4a** and the corresponding initial reagents via intermediate compounds shown in Table 1.

**Table 1.**

| Initial reagent | Intermediate compound | Candidates |
|---|---|---|
| 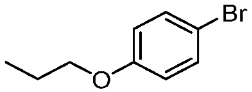 **1ah** | 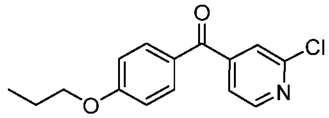 **3ah** | 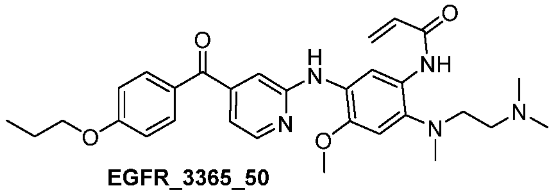 **EGFR_3365_50** |
| 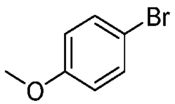 **1ai** | 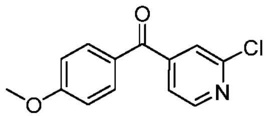 **3ai** | 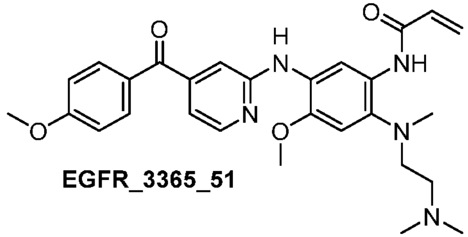 **EGFR_3365_51** |
| 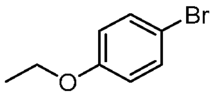 1aj | 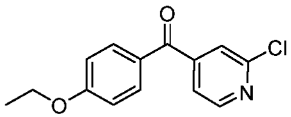 **3aj** | 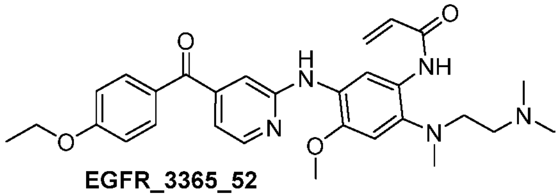 **EGFR_3365_52** |
| 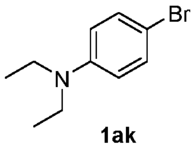 **1ak** | 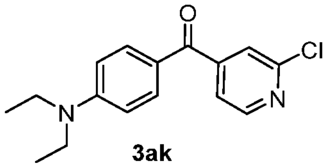 **3ak** | 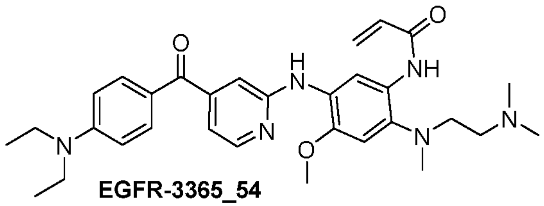 **EGFR-3365_54** |

(continued)

| Initial reagent | Intermediate compound | Candidates |
|---|---|---|
| 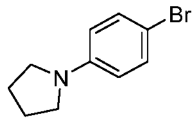 1al | 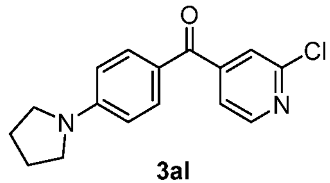 3al | 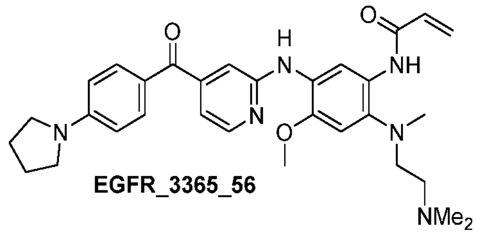 EGFR_3365_56 |
| 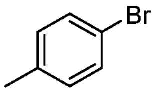 1am | 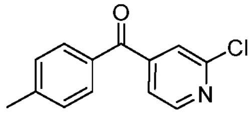 3am | 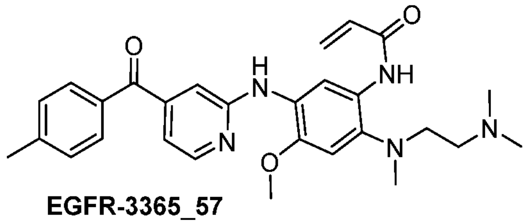 EGFR-3365_57 |
| 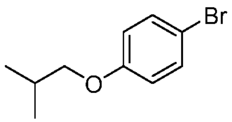 1an | 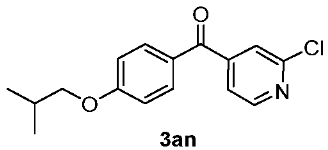 3an | 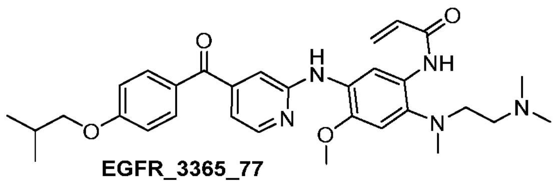 EGFR_3365_77 |
| 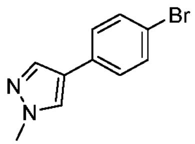 1ao | 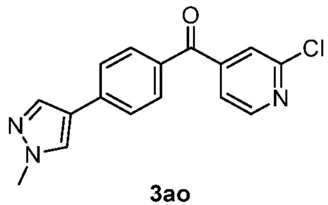 3ao | 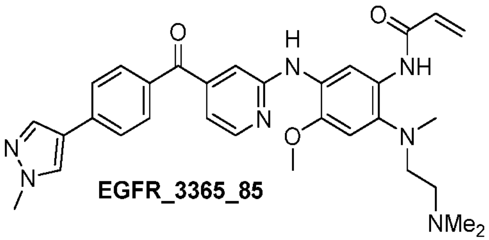 EGFR_3365_85 |

(continued)

| Initial reagent | Intermediate compound | Candidates |
|---|---|---|
| 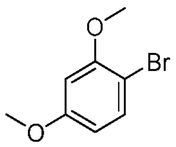 1ap | 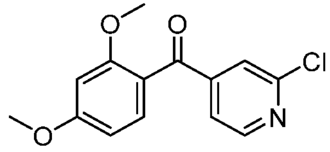 3ap | 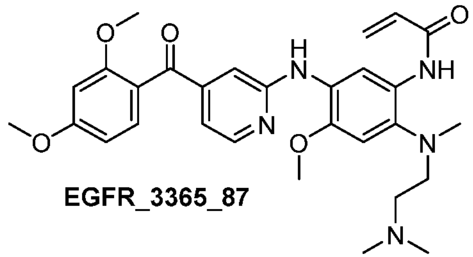 EGFR_3365_87 |
| 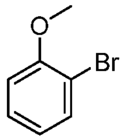 1aq | 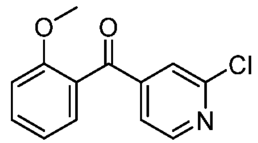 3aq | 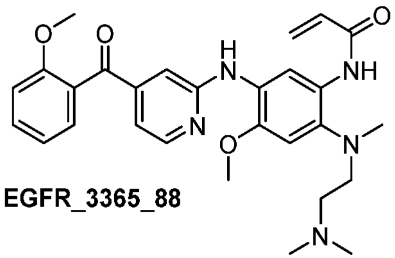 EGFR_3365_88 |
| 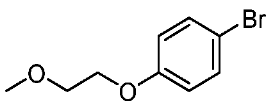 1ar | 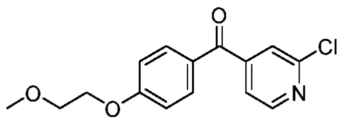 3ar | 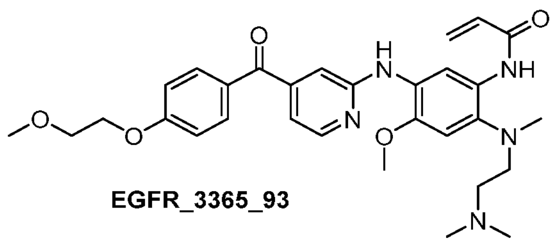 EGFR_3365_93 |

**Example 3.** Method of preparation of compound **EGFR_3365_5, EGFR_3365_15, EGFR_3365_26, EGFR_3365_73, EGFR_3365_101, EGFR_3365_102, EGFR_3365_103, EGFR_3365_104, EGFR_3365_116, EGFR_3365_124**.

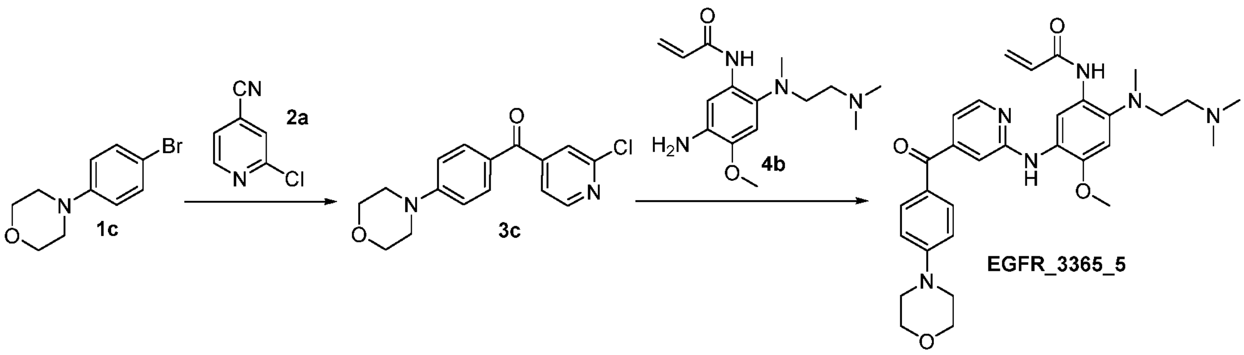
1c 2a 3c 4b EGFR_3365_5

**Step 1.** Preparation of compound **3c.**

Aryl bromide **1c** (0.5 g, 2.06 mmol) and catalytic amount of dibromoethane were added to a suspension of magnesium (0.061 g, 2.52 mmol) in 20 mL of tetrahydrofuran. Resulting suspension was boiled for 2.5 hours, cooled to room temperature, and nitrile **2a** (0.277 g. 2.0 mmol) was added to it. Reaction mixture was stirred at room temperature for 2 days, diluted with saturated $NH_4Cl$ solution, organic layer was separated and concentrated. Residue was dissolved in 10 mL of diethyl ether, 40 mL of 1M HCl was added to a solution, resulting mixture was stirred at room temperature for 30 minutes. Water layer was isolated and neutralized with saturated $NaHCO_3$ solution, product was extracted with dichloromethane, the combined organic layers were washed with saturated NaCl solution, dried with $Na_2SO_4$, filtered and concentrated under vacuum. Product was isolated by column chromatography on silica gel using ethyl acetate/hexane (1:4) as eluent. Yield of the compound **3c** was 0.407 g (65%).

**Step 2.** Preparation of compound **EGFR_3365_5.**

Compound **EGFR_3365_5** was prepared in a similar manner to compound **5a** (step 2) using compound **3c** instead of compound **3a** and aniline **4b** instead of **4a.** Compound **EGFR_3365_5a** was prepared using repeated lyophilization of compound **EGFR_3365_5.**

Compounds **EGFR_3365_15, EGFR_3365_26, EGFR_3365_73, EGFR_3365_101, EGFR_3365_102, EGFR_3365_103, EGFR_3365_104, EGFR_3365_116, EGFR_3365_124** were prepared in a similar manner using the corresponding initial reagents via the corresponding intermediate compounds shown in Table 2.

**Table 2.**

| Initial reagent | Intermediate compound | Candidate |
|---|---|---|
| 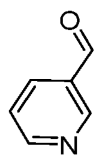 **1i** | 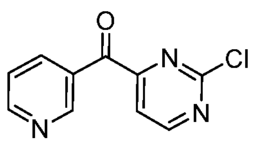 **3i** | 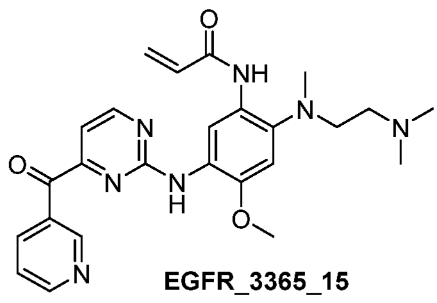 **EGFR_3365_15** |

(continued)

| Initial reagent | Intermediate compound | Candidate |
|---|---|---|
| 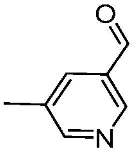 1l | 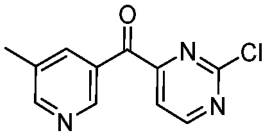 3l | 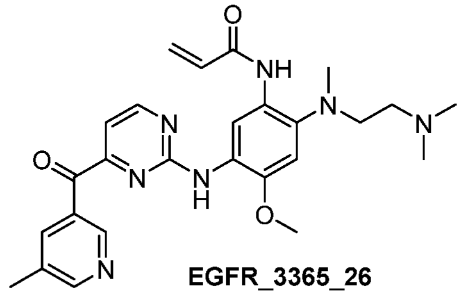 EGFR_3365_26 |
| 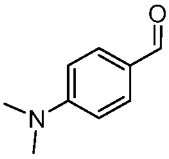 1as | 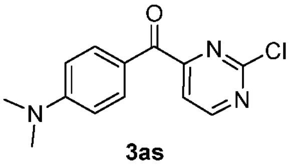 3as | 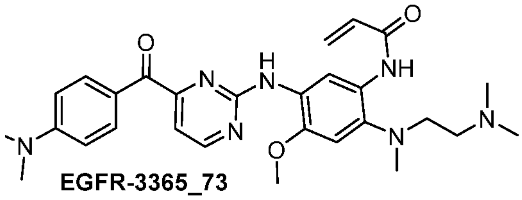 EGFR-3365_73 |
| 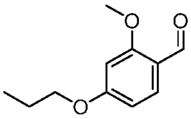 1at | 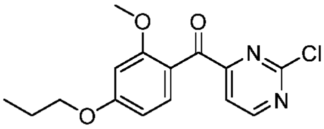 3at | 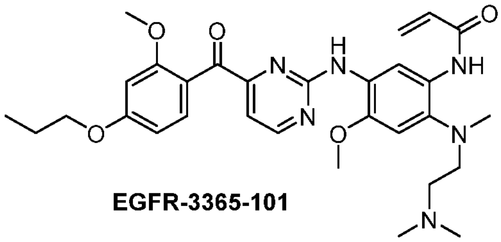 EGFR-3365-101 |
| 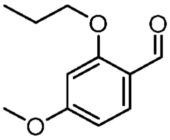 1au | 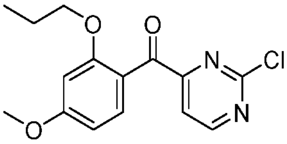 3au | 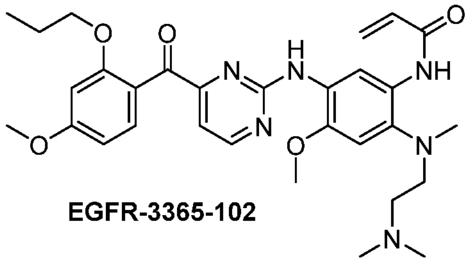 EGFR-3365-102 |
| 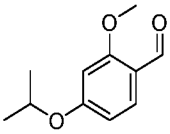 1av | 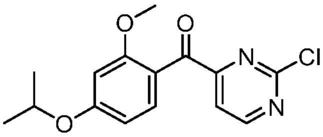 3av | 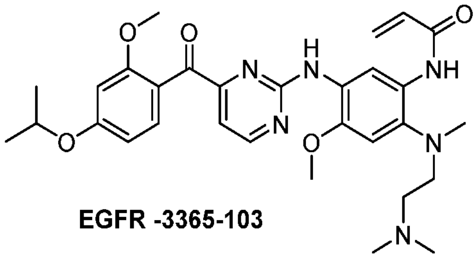 EGFR -3365-103 |
| 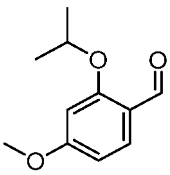 1aw | 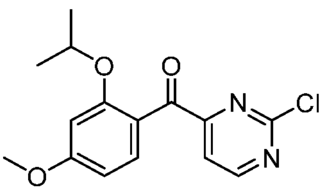 3aw | 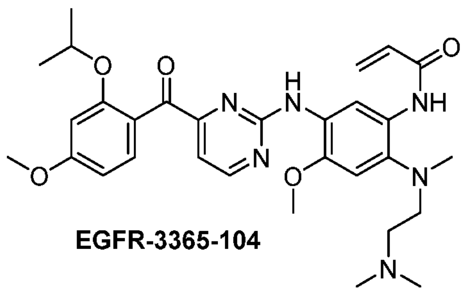 EGFR-3365-104 |

(continued)

| Initial reagent | Intermediate compound | Candidate |
|---|---|---|
| 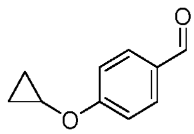 **1ax** | 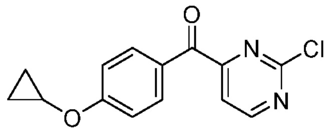 **3ax** | 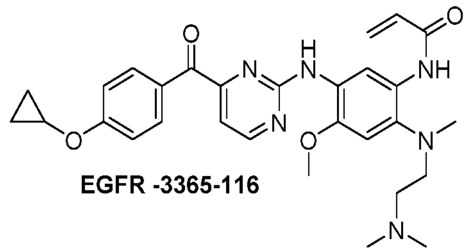 **EGFR -3365-116** |
| 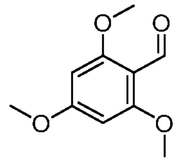 **1ay** | 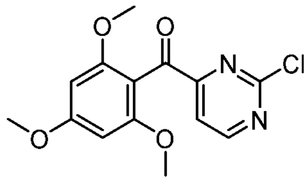 **3ay** | 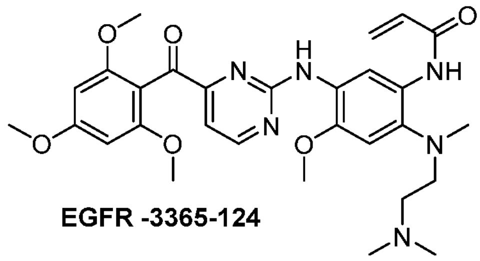 **EGFR -3365-124** |

**Example 4.** Method of preparation of compound **EGFR_3365_10, EGFR_3365_11, EGFR_3365_12, EGFR_3365_13, EGFR_3365_14, EGFR_3365_14a, EGFR_3365_16, EGFR_3365_17, EGFR_3365_28, EGFR_3365_29, EGFR_3365_30, EGFR_3365_31, EGFR_3365_32, EGFR_3365_33, EGFR_3365_34, EGFR_3365_36, EGFR_3365, EGFR_3365_64, EGFR_3365_66, EGFR_3365_67, EGFR_3365_68, EGFR_3365_69, EGFR_3365_70, EGFR_3365_71, EGFR_3365_78, EGFR_3365_86, EGFR_3365_91, EGFR_3365_92, EGFR_3365_94.**

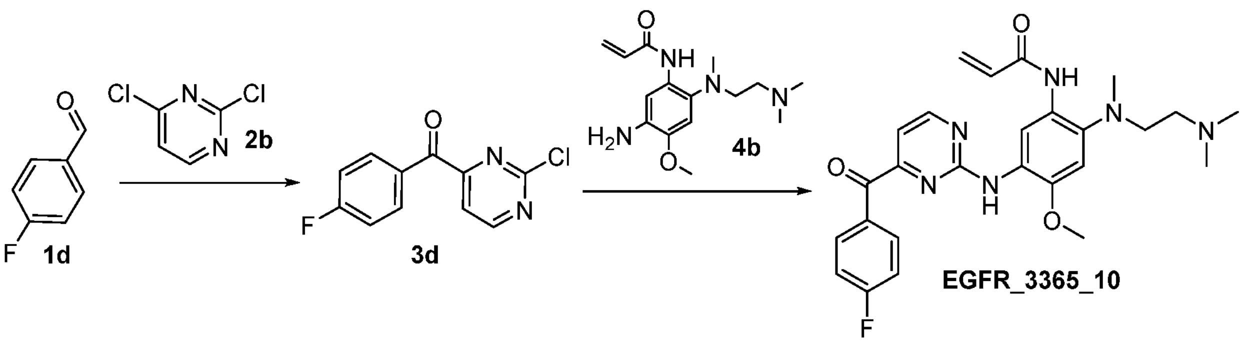
**1d** **2b** **3d** **4b** **EGFR_3365_10**

**Step 1.** Preparation of compound **3d.**

2,4-dichloropyrimidine **2b** (0.500 g, 3.35 mmol) and N,N'-dimethylimidazolium iodide (0.375 g, 1.67 mmol) were added at room temperature to a solution of aldehyde **1d** (0.478 g, 3.85 mmol) in 10 mL of DMF. Resulting solution was degassed with nitrogen for 10 minutes, NaH (60%, 0.201 g, 5.02 mmol) was then added. Reaction mixture was stirred at room temperature for 4 hours. Resulting mixture was then poured into cold water, product was extracted with ethyl acetate. The combined organic layers were washed with water and saturated NaCl solution, dried with $Na_2SO_4$, filtered and concentrated under vacuum. Product was isolated by column chromatography on silica gel using ethyl acetate/hexane (1:4) as eluent. Yield of the compound **3d** was 0.166 g (21%).

Compounds **3e-3u** were prepared in a similar manner (Table 2). Mixture of 1,4-dioxane and DMSO (10:1) was used as a solvent for preparation of compounds **3f** and **3g.**

**Step 2.** Preparation of compound **EGFR_3365_10.**

Aniline **4b** (0.115 g, 0.53 mmol) and trifluoroacetic acid (0.185 mL, 2.64 mmol) were added at room temperature

to a solution of compound **3d** (0.114 g, 0.48 mmol) in 2 mL of isopropyl alcohol. Reaction mixture was boiled for 24 hours. Saturated $NaHCO_3$ solution and dichloromethane were added to a reaction mixture. Organic layer was washed with saturated NaCl solution, dried with $Na_2SO_4$, filtered and concentrated under vacuum. Product was isolated by column chromatography on silica gel using dichloromethane/hexane/ethyl acetate (3:1:1) as eluent with gradient triethylamine (from 0.5% to 3%). Yield of the compound **EGFR_3365_10** was 54 mg (23%). Product was additionally purified by preparative chromatography.

Compounds **EGFR_3365_11, EGFR_3365_12, EGFR_3365_13, EGFR_3365_14, EGFR_3365_14a, EGFR_3365_16, EGFR_3365_17, EGFR_3365_28, EGFR_3365_29, EGFR_3365_30, EGFR_3365_31, EGFR_3365_32, EGFR_3365_33, EGFR_3365_34, EGFR_3365_36, EGFR 3365, EGFR_3365_64, EGFR_3365_66, EGFR_3365_67, EGFR_3365_68, EGFR_3365_69, EGFR_3365_70, EGFR_3365_71, EGFR_3365_78, EGFR_3365_86, EGFR_3365_91, EGFR_3365_92, EGFR_3365_94** were prepared in a similar manner using the corresponding initial reagents via the corresponding intermediate compounds shown in Table 3.

**Table 3.**

| Initial reagent | Intermediate compound | Candidate |
|---|---|---|
| 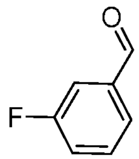 **1e** | 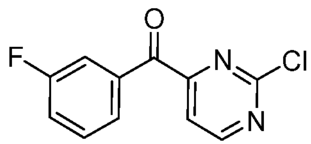 **3e** | 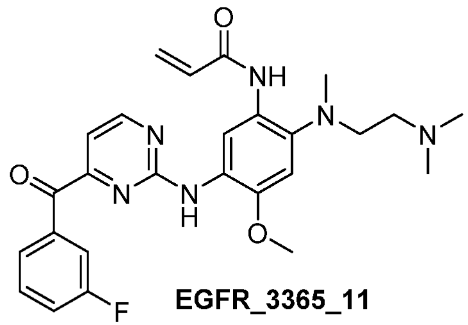 **EGFR_3365_11** |
| 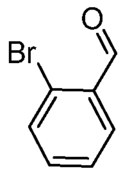 **1f** | 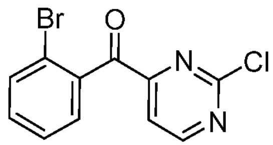 **3f** | 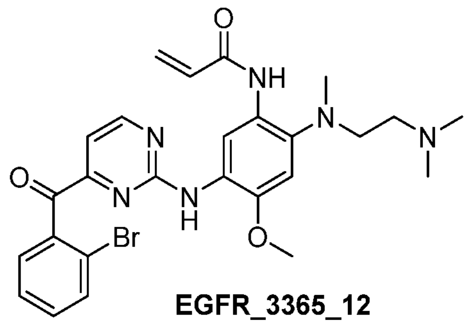 **EGFR_3365_12** |
| 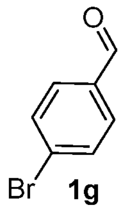 **1g** | 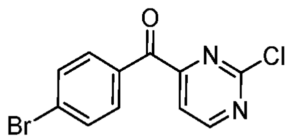 **3g** | 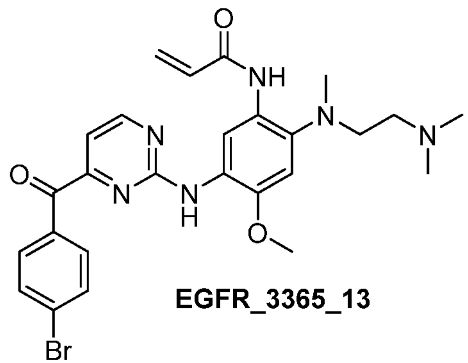 **EGFR_3365_13** |

(continued)

| Initial reagent | Intermediate compound | Candidate |
|---|---|---|
| 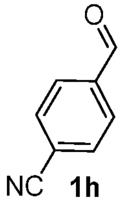 **1h** | 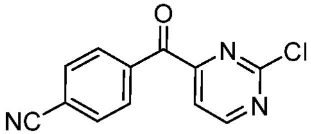 **3h** | 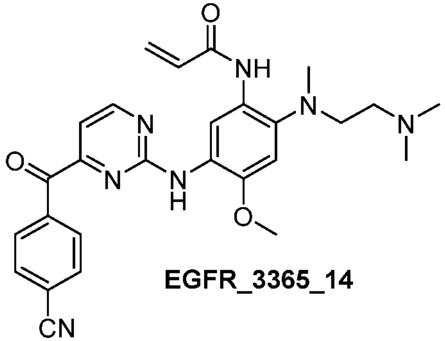 **EGFR_3365_14** |
| 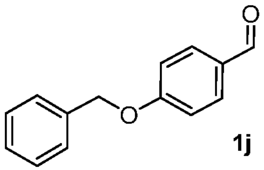 **1j** | 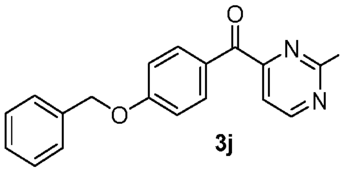 **3j** | 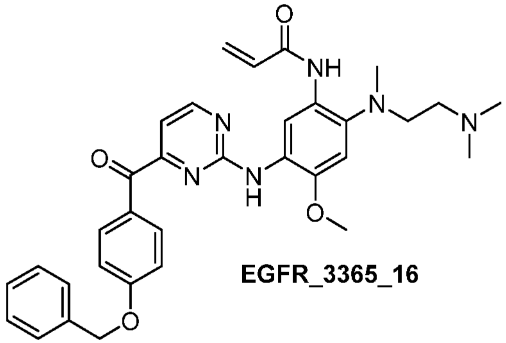 **EGFR_3365_16** |

(continued)

| Initial reagent | Intermediate compound | Candidate |
|---|---|---|
| 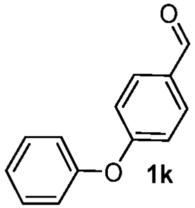 1k | 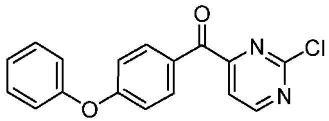 3k | 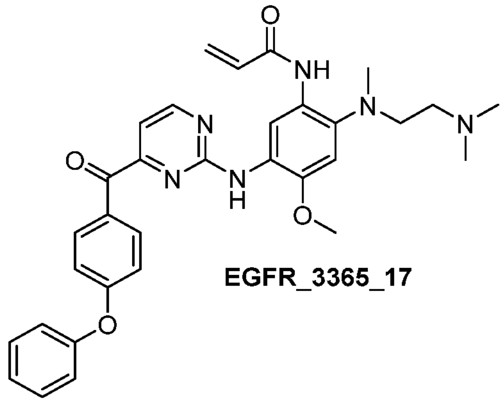 EGFR_3365_17 |
| 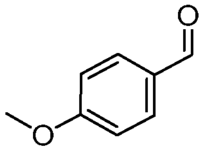 1m | 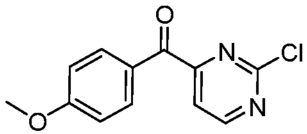 3m | 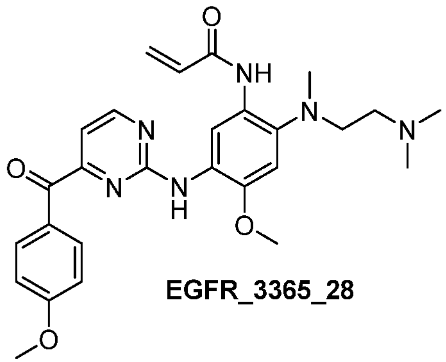 EGFR_3365_28 |

(continued)

| Initial reagent | Intermediate compound | Candidate |
|---|---|---|
| 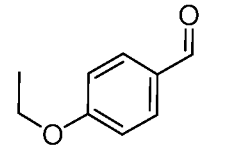 1n | 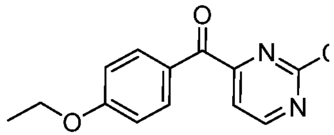 3n | 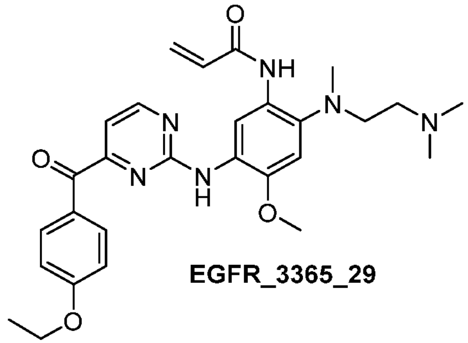 EGFR_3365_29 |
| 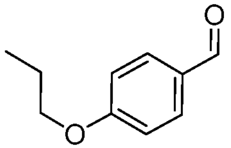 1o | 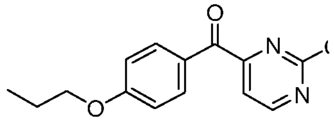 3o | 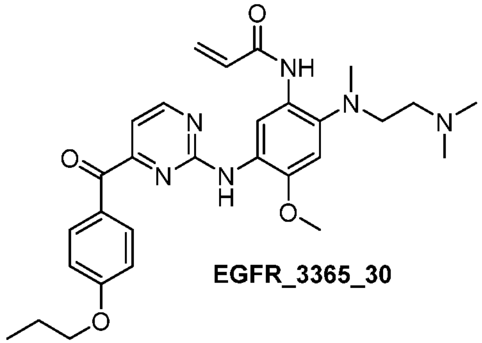 EGFR_3365_30 |
| 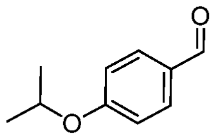 1p | 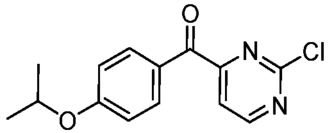 3p | 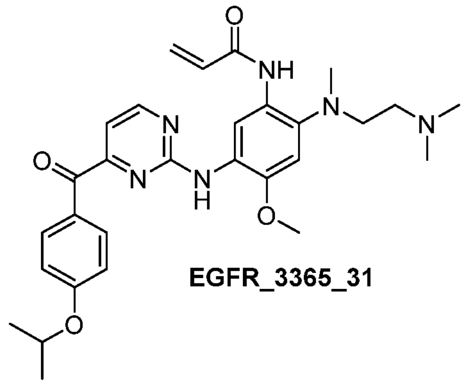 EGFR_3365_31 |

(continued)

| Initial reagent | Intermediate compound | Candidate |
|---|---|---|
| 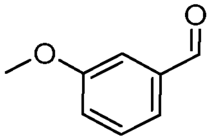 **1q** | 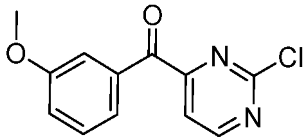 **3q** | 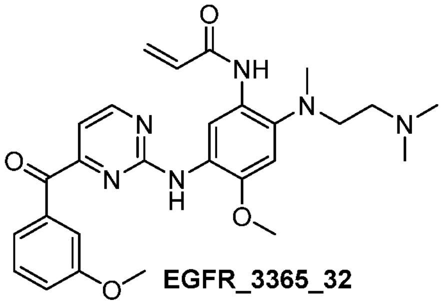 **EGFR_3365_32** |
| 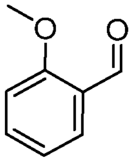 **1r** | 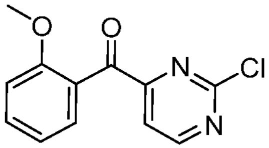 **3r** | 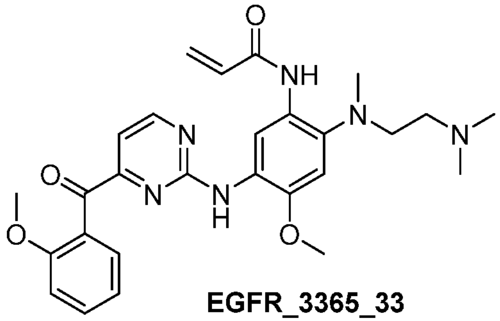 **EGFR_3365_33** |
| 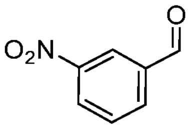 **1s** | 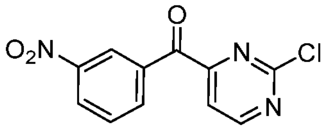 **3s** | 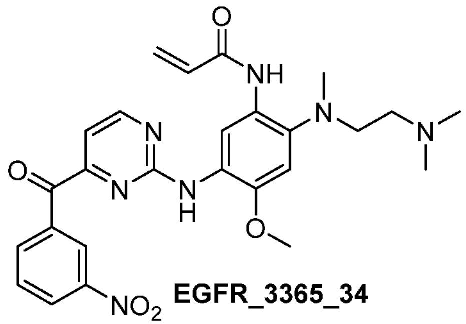 **EGFR_3365_34** |

(continued)

| Initial reagent | Intermediate compound | Candidate |
|---|---|---|
| 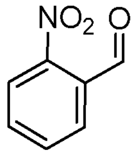 1t | 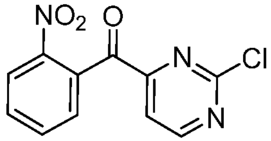 3t | 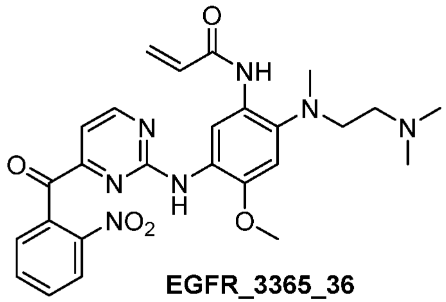 EGFR_3365_36 |
| 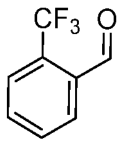 1u | 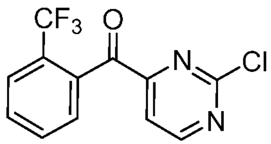 3u | 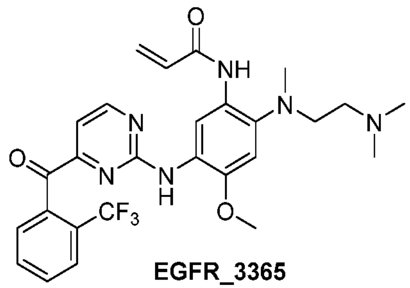 EGFR_3365 |
| 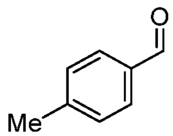 1v | 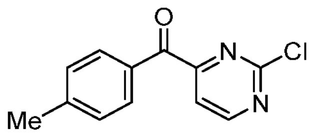 3v | 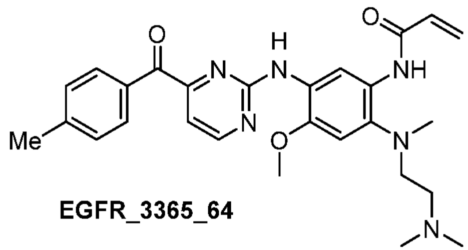 EGFR_3365_64 |
| 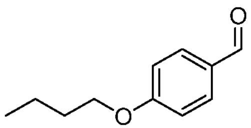 1w | 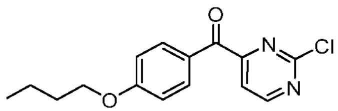 3w | 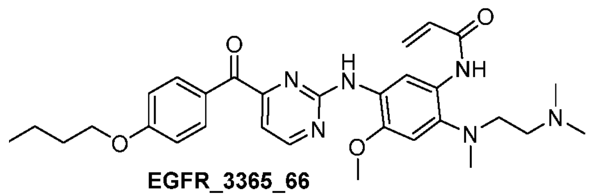 EGFR_3365_66 |

(continued)

| Initial reagent | Intermediate compound | Candidate |
|---|---|---|
| 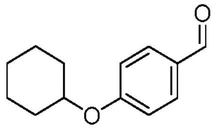 1x | 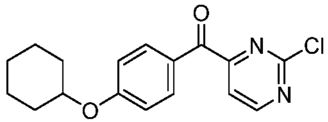 3x | 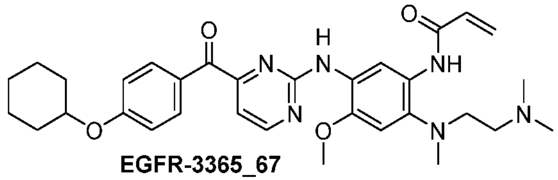 EGFR-3365_67 |
| 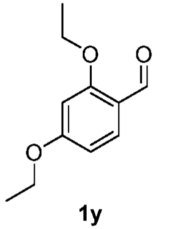 1y | 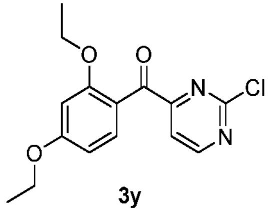 3y | 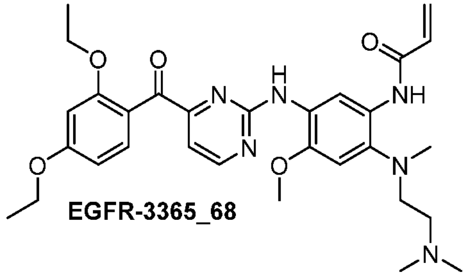 EGFR-3365_68 |
| 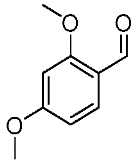 1z | 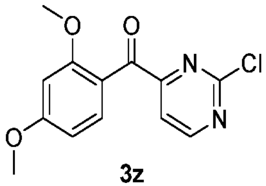 3z | 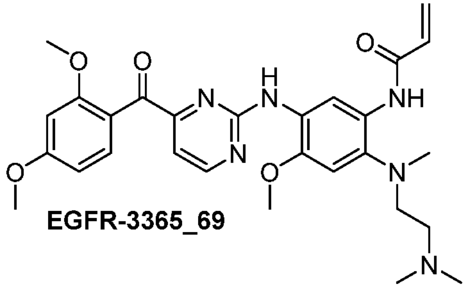 EGFR-3365_69 |
| 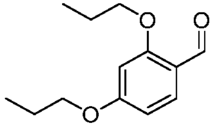 1aa | 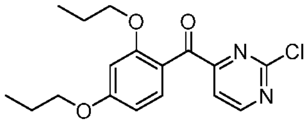 3aa | 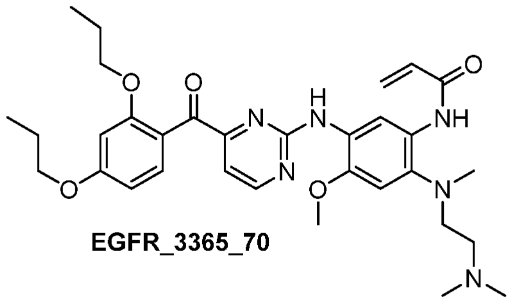 EGFR_3365_70 |

(continued)

| Initial reagent | Intermediate compound | Candidate |
|---|---|---|
| 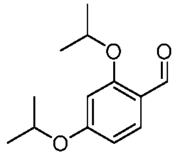 1ab | 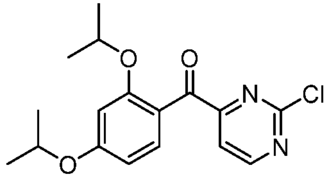 3ab | 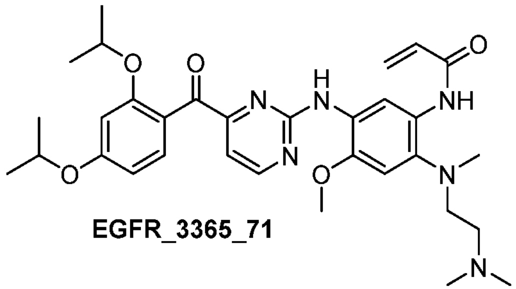 EGFR_3365_71 |
| 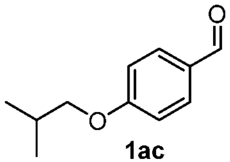 1ac | 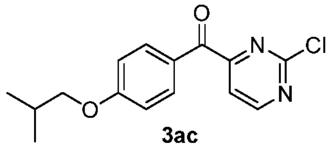 3ac | 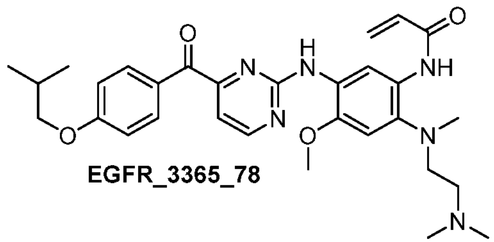 EGFR_3365_78 |
| 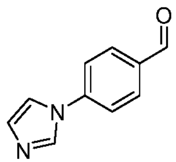 1ad | 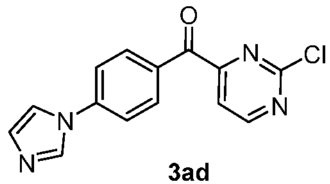 3ad | 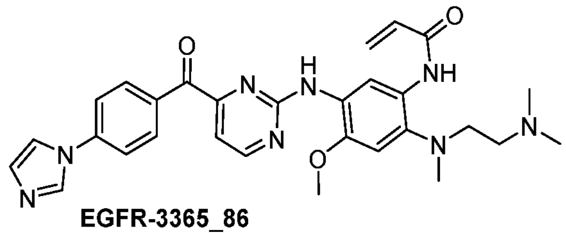 EGFR-3365_86 |
| 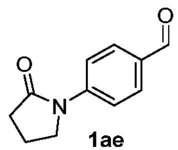 1ae | 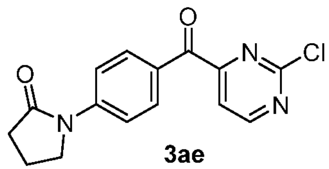 3ae | 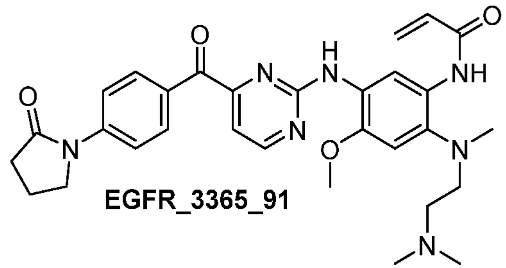 EGFR_3365_91 |

(continued)

| Initial reagent | Intermediate compound | Candidate |
|---|---|---|
| 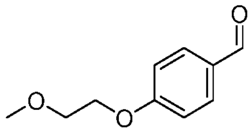 **1af** | 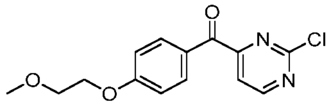 **3af** | 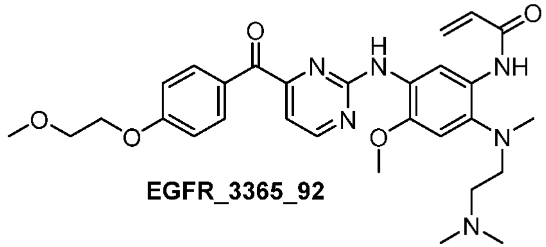 **EGFR_3365_92** |
| 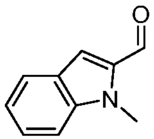 **1ag** | 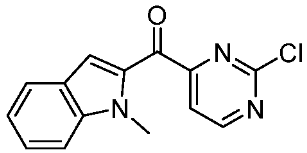 **3ag** | 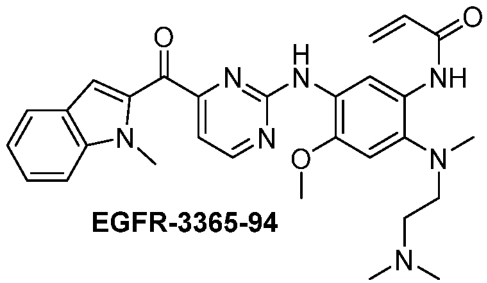 **EGFR-3365-94** |

**Example 5.** Method of preparation of compound **EGFR_3365_31a, EGFR_3365_30a.**

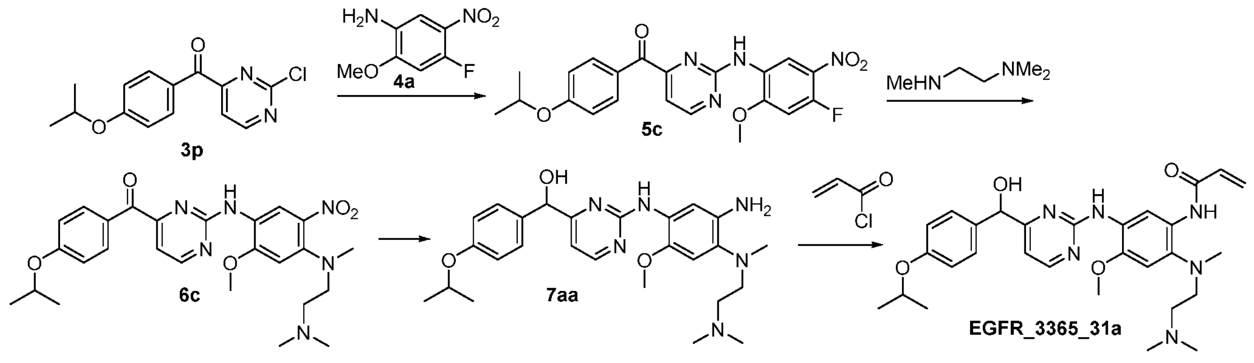

3p, 5c, 6c, 7aa, EGFR_3365_31a

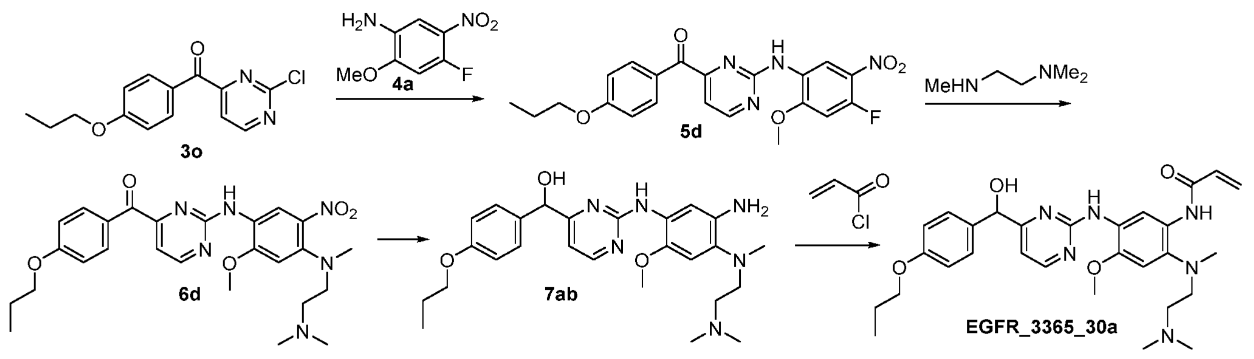

3o, 5d, 6d, 7ab, EGFR_3365_30a

**Step 1.** Preparation of compound **5c.**

Compound **5c** was prepared in the same manner to compound **EGFR_3365_10** using compound **3p** instead of compound **3d** and aniline **4a** instead of aniline **4b.**

Compound **5d** was prepared in a similar manner using the corresponding initial compound **3o.**

**Step 2.** Preparation of compound **6c.**

Compound **6c** was prepared in the same manner to compound **6a** using compound **5c** instead of compound **5a.**

Compound **6d** was prepared in a similar manner using the corresponding intermediate compound **5d.**

**Step 3.** Preparation of compound **7aa.**

$Na_2S_2O_4$ (13.75 g, 78.2 mmol) was added to a solution of compound **6c** (4.1 g, 7.82 mmol) in 120 mL of methanol/water (1:2) mixture. Reaction mixture was stirred at 40 °C for 1 hour. Resulting mixture was concentrated, product was extracted with dichloromethane, the combined organic layers were dried with $Na_2SO_4$, filtered and concentrated under vacuum. Product was isolated by column chromatography on silica gel using dichloromethane/methanol (gradient from 9:1 to 1:1) as eluent. Yield of the compound **7aa** was 3.6 g (96%) obtained as brown oily substance.

Compound **7ab** was prepared in a similar manner using the corresponding intermediate compound **6d.**

**Step 4.** Preparation of compound **EGFR_3365_31a.**

Solution of *N,N*-diisopropylethylamine (0.63 g, 4,84 mmol) in 5 mL of dichloromethane was added dropwise under nitrogen atmosphere at -70°C to a solution of **7aa** (2.0 g, 4.03 mmol) in 40 mL of dichloromethane, solution of acryloyl chloride (0.33 g, 3.63 mmol) in 5 mL of dichloromethane was then added. Reaction mixture was stirred at -40°C for 2 hours, water was then added at the same temperature, product was extracted with dichloromethane, the combined organic layers were dried with $Na_2SO_4$, filtered and concentrated under vacuum. Product was isolated by column chromatography on silica gel using dichloromethane/methanol (gradient from 99:1 to 90:1) as eluent. Yield of the compound **EGFR_3365_31a** was 0.81 g (37%) obtained as white powder.

Compound **EGFR_3365_30a** was prepared in a similar manner using the corresponding intermediate compound

**7ab.**

**Example 6.** Method of preparation of compound **EGFR_3365_63, EGFR_3365_58, EGFR_3365_61, EGFR_3365_62, EGFR_3365_62a, EGFR_3365_72, EGFR_3365_90, EGFR_3365_97, EGFR_3365_98, EGFR_3365_105, EGFR_3365_106, EGFR_3365_106a, EGFR_3365_108, EGFR_3365_109, EGFR_3365_110, EGFR_3365_111, EGFR_3365_112, EGFR_3365_112a, EGFR_3365_113, EGFR_3365_114, EGFR_3365_115, EGFR_3365_121a.**

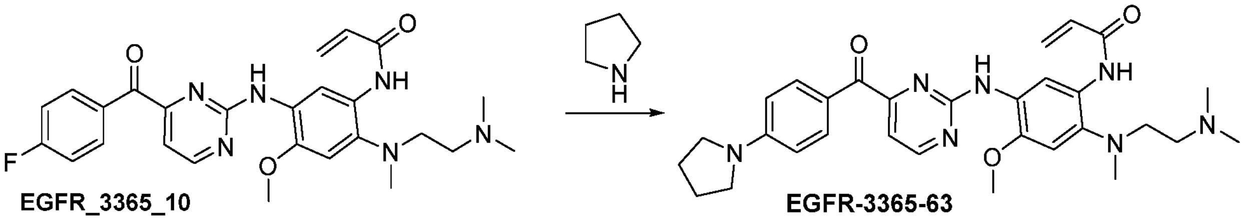

**EGFR_3365_10** **EGFR-3365-63**

$K_2CO_3$ (36 mg, 0.258 mmol, 1.25 eq) was added to a solution of compound **EGFR_3365_10** (102 mg, 0.21 mmol) and pyrrolidine (17 μl, 0.21 mmol, 1 eq) in DMF (1 mL). Resulting suspension was stirred at 80°C for 12 hours. Reaction mixture was poured into water, product was extracted with ethyl acetate. The combined organic layers were dried with $Na_2SO_4$, filtered and concentrated under vacuum. Product was isolated by column chromatography on silica gel using ethyl acetate/triethylamine (1/0.075) as eluent. Yield of the compound **EGFR_3365_63** was 72 mg. Product was additionally purified by preparative HPLC, as a result 48 mg (43%) was obtained.

Compounds **EGFR_3365_58, EGFR_3365_61, EGFR_3365_62, EGFR_3365_62a, EGFR_3365_72, EGFR_3365_90, EGFR_3365_97, EGFR_3365_98, EGFR_3365_105, EGFR_3365_106, EGFR_3365_106a, EGFR_3365_108, EGFR_3365_109, EGFR_3365_110, EGFR_3365_111, EGFR_3365_112, EGFR_3365_112a, EGFR_3365_113, EGFR_3365_114, EGFR_3365_115, EGFR_3365_121a** were prepared in a similar manner using the corresponding initial reagents shown in Table 4.

**Table 4.**

| Initial reagent | Candidate |
|---|---|
| 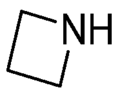 | 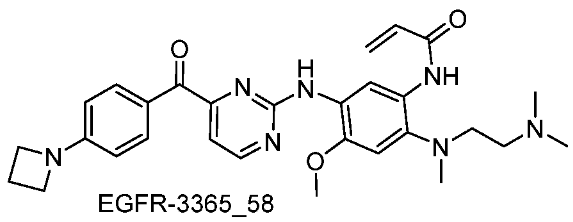 EGFR-3365_58 |
| 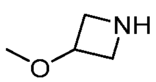 | 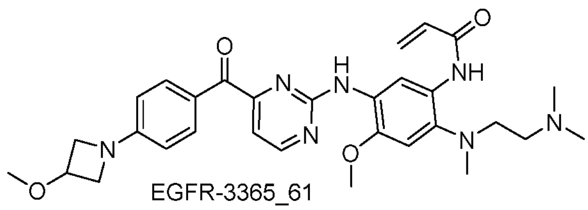 EGFR-3365_61 |
| 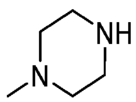 | 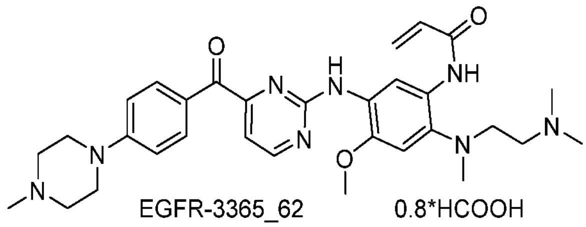 EGFR-3365_62 0.8*HCOOH |

(continued)

| Initial reagent | Candidate |
|---|---|
| 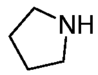 | 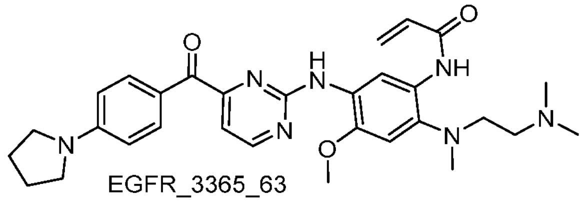 EGFR_3365_63 |
| 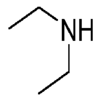 | 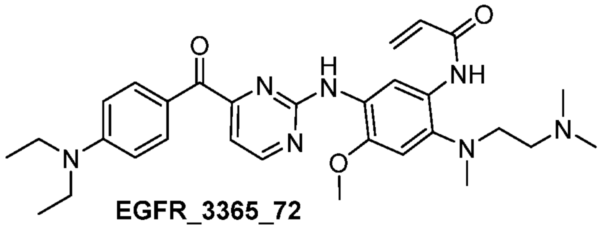 **EGFR_3365_72** |
| 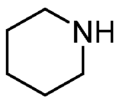 | 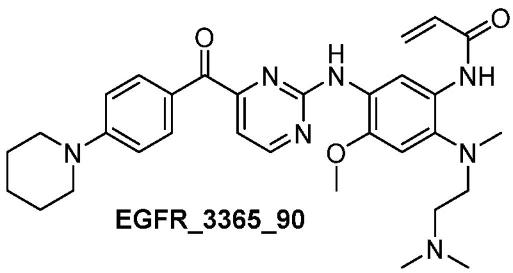 **EGFR_3365_90** |
| 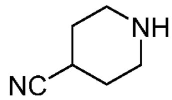 | 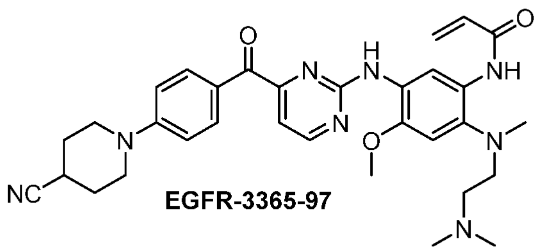 **EGFR-3365-97** |
| 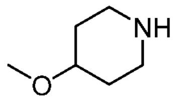 | 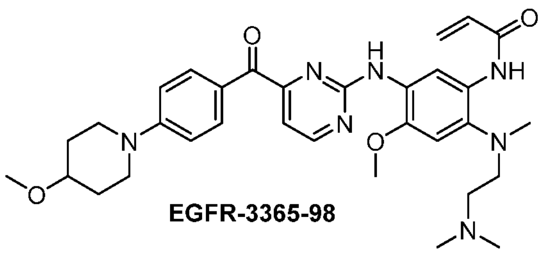 **EGFR-3365-98** |
| 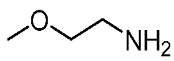 | 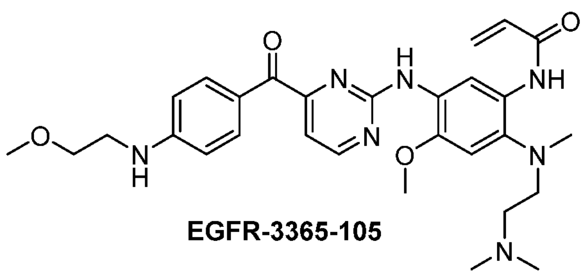 **EGFR-3365-105** |

(continued)

| Initial reagent | Candidate |
|---|---|
| 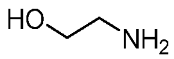 | 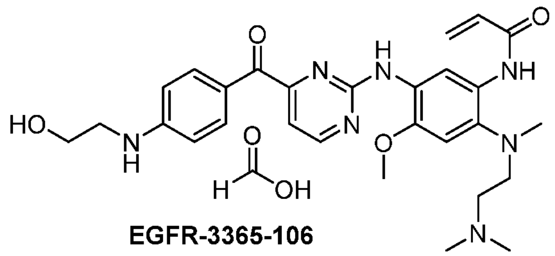 EGFR-3365-106 |
| 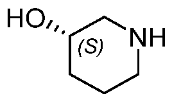 | 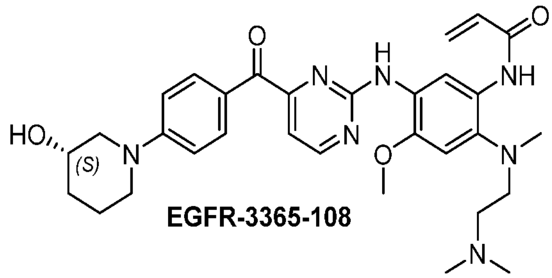 EGFR-3365-108 |
| 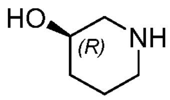 | 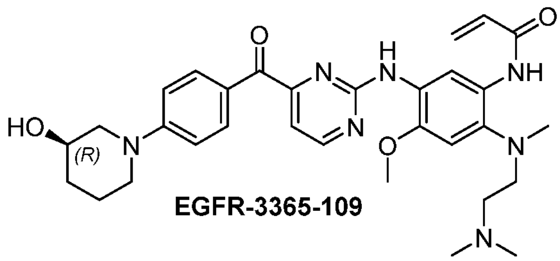 EGFR-3365-109 |
| 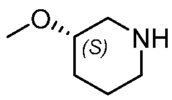 | 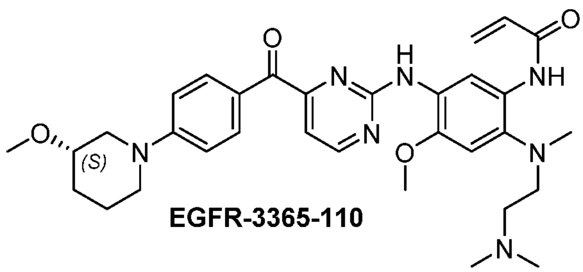 EGFR-3365-110 |
| 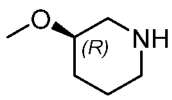 | 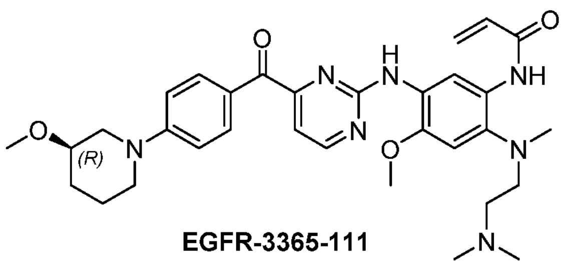 EGFR-3365-111 |

(continued)

| Initial reagent | Candidate |
|---|---|
| 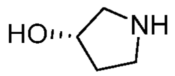 | 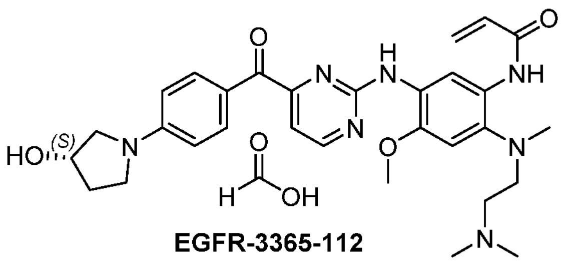 EGFR-3365-112 |
| 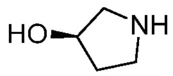 | 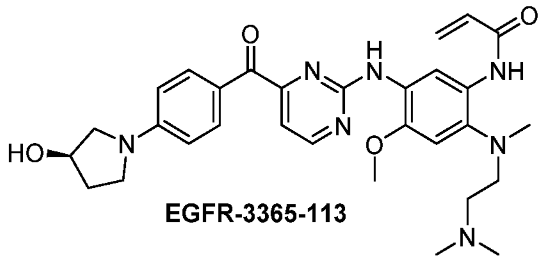 EGFR-3365-113 |
| 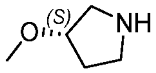 | 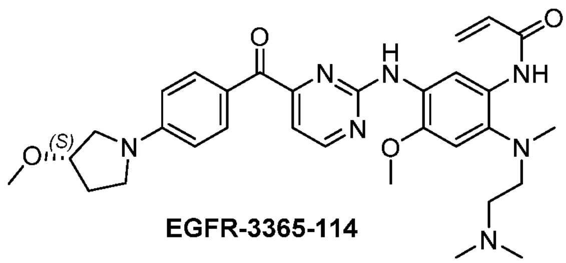 EGFR-3365-114 |
| 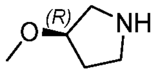 | 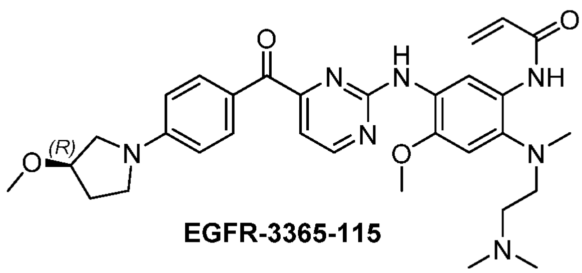 EGFR-3365-115 |
| 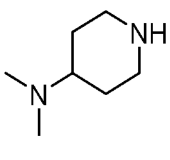 | 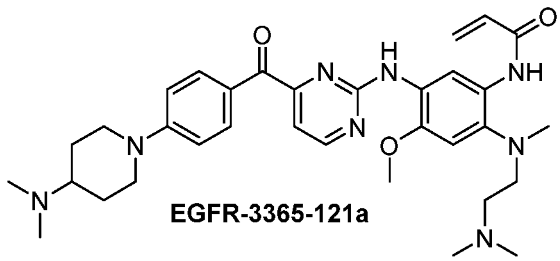 EGFR-3365-121a |

**Example 7.** Method of preparation of compound **EGFR_3365_120, EGFR_3365_122, EGFR-3365_123, EGFR_3365_127.**

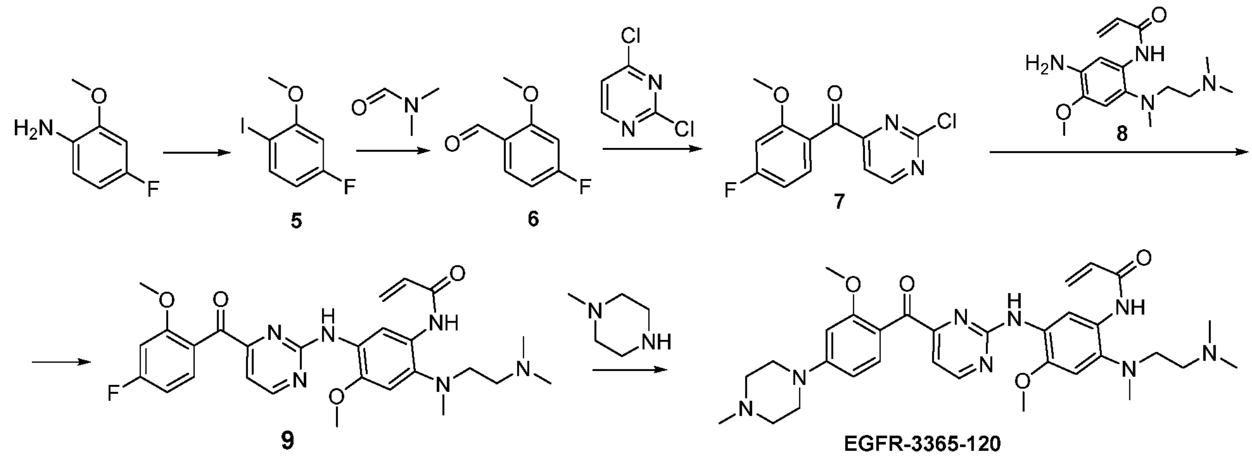

5 6 7 8

9 EGFR-3365-120

**Step 1.** Preparation of compound **5.**

4-Fluoro-2-methoxyaniline (2.50 g, 17.5 mmol) was dissolved in 14 mL of concentrated HCl, cooled to 0°C, solution of $NaNO_2$ (1.45 g, 21.0 mmol) in 13 mL of water was then added under stirring. It was stirred for 40 minutes, resulting solution was added dropwise at room temperature to a solution of KI (8.73 g, 52.6 mmol) in 30 mL of water. Resulting mixture was stirred at 35-40°C for 1 hour, product was then extracted with ethyl acetate (3x70 mL), extract was washed with $Na_2S_2O_3$ solution, dried with $Na_2SO_4$. Product was isolated by column chromatography on silica gel using hexane as eluent. Yield of 2-iodo-5-fluoroanysol **5** was 3.46 g (78%) obtained as colourless solid mass.

**Step 2.** Preparation of compound **6.**

2-Iodo-5-fluoroanisole **5** (3.46 g, 13.0 mmol) was dissolved in 70 mL of THF, reaction mixture was cooled to -10°C under nitrogen atmosphere, 2 M isopropylmagnesium chloride solution in THF (8.50 mL, 17.0 mmol) was added, stirred for 30 minutes, DMF (2.89 g, 39.1 mmol) was added, stirred for 30 minutes, reaction mixture was brought to toom temperature, 20 mL of saturated $NH_4Cl$ water solution was added. Resulting mixture was extracted with ethyl acetate (3x70 mL), extract was washed with water (5x20 mL), dried with $Na_2SO_4$. Product was isolated by column chromatography on silica gel using ethyl acetate-hexane (1:9) as eluent. Yield of 2-methoxy-4-fluorobenzaldehyde **6** was 1.9 g (94%).

**Step 3.** Preparation of compound **7.**

2-methoxy-4-fluorobenzaldehyde (0.40 g, 2.60 mmol), 2,4-dichloropyrimidine (0.59 g, 3.89 mmol), 1,3-dimethylimidazolium iodide (0.31 g, 1.30 mmol) were dissolved in 9 mL of DMF, nitrogen was passed through reaction mixture for 2 minutes, NaH (0.13 g, 2.24 mmol, 60% suspension in paraffin) was added, resulting mixture was stirred under nitrogen atmosphere at 75°C for 4 hours. 2,4-Dichloropyrimidine (0.3 g, 1.95 mmol), 1,3-dimethylimidazolium iodide (0.16 g, 0.65 mmol) and NaH (0.065 g, 1.12 mmol) were added to the reaction mixture and heated under stirring for 1 hour, NaH (0.065 g, 1.12 mmol) was added, then heated for 2 more hours. 20 mL of water was added to the resulting mixture, product was extracted with ethyl acetate (3x50 mL), the extract was washed with water (4x10 mL), dried with $Na_2SO_4$. Product was isolated by column chromatography on silica gel using ethyl acetate-hexane (1:9) as eluent. Yield of the compound 7 was 0.21 g (30%).

**Step 4.** Preparation of compound **9.**

Compound **7** (0.12 g, 0.45 mmol), aniline **8** (0.28 g, 0.90 mmol), BINAP (0.057 g, 0.09 mmol), $CS_2CO_3$ (0.44 g, 1.35 mmol) and $Pd(OAc)_2$ (10 mg, 0.05 mmol, 10 mmol %) were dissolved in 3.6 mL of dioxane and placed in a screw-cap vessel. Reaction mixture was degassed by passing nitrogen through it for 5 minutes, stirred under nitrogen atmosphere at 90°C for 4 hours. Product was isolated by column chromatography on silica gel using ethyl triethylamine-ethyl acetate-hexane (2:40:10) as eluent. Yield of the compound **9** was 0.18 g (69%).

**Step 5.** Preparation of compound **EGFR_3365_120.**

Compound **9** (0.10g, 0.17 mmol), $K_2CO_3$ (0.029 g, 0.21 mmol), 1 mL of DMSO and N-methylpiperazine (0.019 g, 0.19 mmol) were placed in a screw-cap vessel in the respective order. Reaction mixture was stirred at 80°C for 5

hours. 5 mL of water was added to the resulting mixture, product was extracted with ethyl acetate (3x30 mL), the extract was washed with water (3x5 mL), dried with $Na_2SO_4$. Product was isolated by column chromatography on silica gel using methanol and methanol-triethylamine (100:1) subsequently as eluent. Yield of the compound **EGFR_3365_120** was 0.054 g (52%).

Compounds **EGFR_3365_122, EGFR_3365_123 И EGFR_3365_127** were prepared in a similar manner using the corresponding initial reagents shown in Table 5.

**Table 5.**

| Initial reagent | Candidate |
|---|---|
| 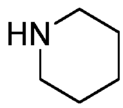 | 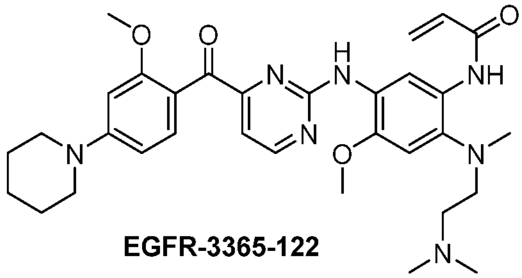 **EGFR-3365-122** |
| 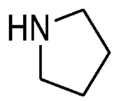 | 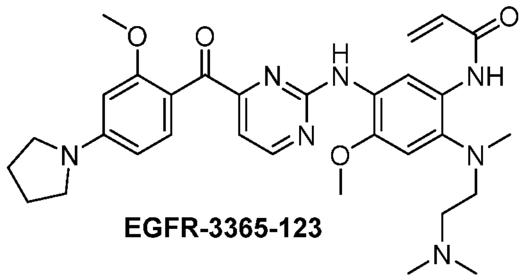 **EGFR-3365-123** |
| 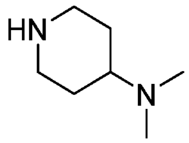 | 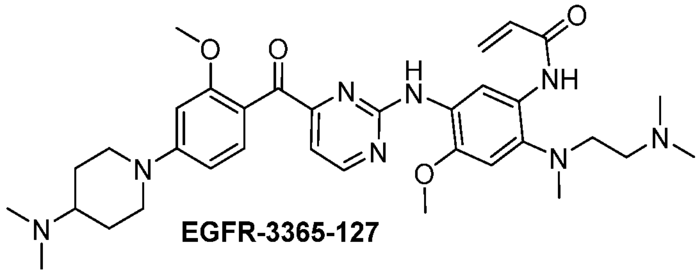 **EGFR-3365-127** |

**Example 8.** Method of preparation of compound **EGFR_3365_121, EGFR 3365_126.**

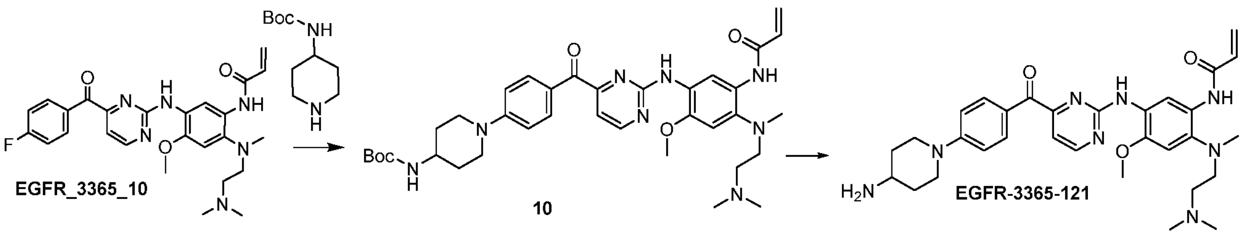
EGFR_3365_10
10
EGFR-3365-121

**Step 1.** Preparation of compound **10.**

Compound **10** was prepared in a similar manner to compound **EGFR_3365_63** using compound *tert*-butylpiperidin-4-yl carbamate instead of pyrrolidine.

**Step 2.** Preparation of compound **EGFR_3365_121.**

Trifluoroacetic acid (1.8 mL) was added at room temperature to a solution of compound **9** (0.051 g, 0.07 mmol) in dichloromethane (7 mL). Reaction mixture was stirred at room temperature for 1 hour. Resulting mixture was carefully poured into saturated $Na_2CO_3$ solution (degassed). Product was extracted with ethyl acetate (3x15 mL), the combined organic layers were washed with water (2x10 mL), saturated NaCl solution (1×10 mL), dried with $Na_2SO_4$, filtered and concentrated under vacuum. Resulting product (0.032 g, 80%) was purified by preparative HPLC. The obtained product was treated with saturated $Na_2CO_3$ solution (degassed) to an alkalescent medium, product was extracted with ethyl acetate (3x15 mL) (degassed). The combined organic layers were washed with saturated NaCl solution (1×10 mL). The obtained extract was dried with $Na_2SO_4$, filtered and concentrated under vacuum. After lyophilization, 0.03 g (75%) of a yellow crystalline substance was obtained.

Compound **EGFR_3365_126** was prepared in a similar manner using the corresponding intermediate compound 9 and *tert*-butylpiperidin-4-yl carbamate.

**Example 9.** Method of preparation of compound **EGFR_3365_53.**

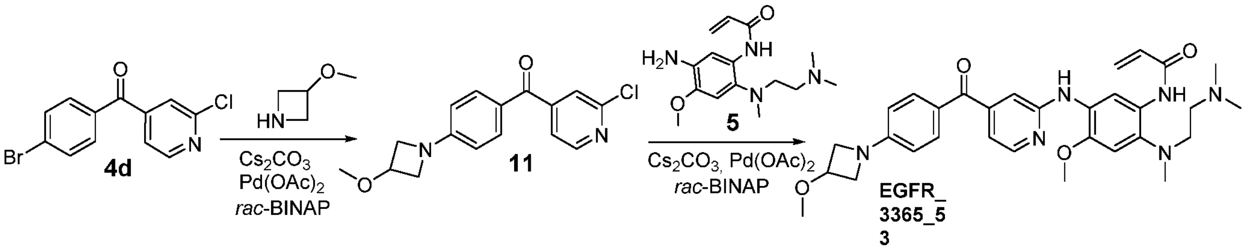


**Step 1.** Preparation of compound **11.**

$Pd(OAc)_2$ (15 mg, 0.06 mmol, 0.1 eq) was added under a steam of argon to a mixture of ketone **4d** (200 mg, 0.65 mmol), 3-methoxyazetidine hydrochloride (74 mg, 0.58 mmol, 0.9 eq), rac-BINAP (82 mg, 0.013 mmol, 0.2 eq), (646 mg, 1.96 mmol, 3 eq) in in dry 1,4-dioxane (14 mL). Reaction mixture was stirred at 90°C for 3 hours. 45 mL of water was added to the resulting mixture, product was extracted with ethyl acetate (3x15 mL). The combined organic layers were dried with $Na_2SO_4$, filtered and concentrated under vacuum. Product was isolated by column chromatography on silica gel using ethyl acetate/hexane (10/90) as eluent. Yield of the compound **7** was 110 mg (45%) obtained as yellow powder.

**Step 2.** Preparation of compound **EGFR_3365_53.**

$Pd(OAc)_2$ (7 mg, 0.03 mmol, 0.1 eq) was added under a steam of argon to a mixture of compound **11** (100 mg, 0.29 mmol), compound **5** (79 mg, 0.27 mmol, 0.9 eq), *rac*-BINAP (37 mg, 0.059 mmol, 0.2 eq), (293 mg, 0.89 mmol, 3 eq) in in dry 1,4-dioxane (7 mL). Reaction mixture was stirred at 90°C for 4 hours. 30 mL of water was added to the resulting mixture, product was extracted with ethyl acetate (3x10 mL). The combined organic layers were dried with $Na_2SO_4$, filtered and concentrated under vacuum. Product was isolated by column chromatography on silica gel using ethyl acetate/triethylamine (98/2) as eluent. Yield of the compound **EGFR_3365_53** was 110 mg (45%) obtained as yellow powder.

**Example 10.** Analysis of prepared compounds.

Purity and structure of the prepared compounds were confirmed by liquid chromatography-mass spectrometry (LC-MS) and $^1H$ NMR spectroscopy (Table 6).

**Equipment Data:**

**Table 6.** Liquid chromatography-mass spectrometry

| Name | Manufacturer, country |
|---|---|
| Agilent Triple Quad liquid chromatography/mass spectrometry (LC/MS) system | Agilent, USA |
| Agilent 1200 Autosampler | |
| Agilent 1200 Thermostatted Column | |
| Agilent 1200 Degasser | |
| Agilent 1200 Autosampler Thermostat | |
| Agilent 6410 QQQ MS Detector | |
| Agilent 1200 UV Detector | |
| Agilent 1200 Pump | |

**Table 7.** NMR spectrometer

| Name | Manufacturer, country | Model, main characteristics |
|---|---|---|
| NMR spectrometer | Germany | AVANCE III, 400 MHz |

**Table 8.** Analytical data for examples of compounds

| Code | ESI-MS $[M+H]^+$ | $^1H$ NMR (400 MHz, DMSO-$d_6$), δ |
|---|---|---|
| **EGFR_3365** | 543.2 | $^1H$ Я MP (400 MHz, $CDCl_3$), δ 9.11 (s, 1H), 8.77 (d, *J* = 8.8 Hz, 1H), 7.74 - 7.72 (m, 1H), 7.62 - 7.59 (m, 4H), 7.4 (d, *J* = 7.4 Hz 1H), 7.28 (s, 1H), 6.68 (s, 2H), 6.46 (d, *J* = 6.5 Hz, 1H), 5.75 (d, *J* = 5.8 Hz, 1H), 3.88 (s, 3H), 2.89 - 2.86 (m, 2H), 2.71 (s, 3H), 2.31 - 2.27 (m, 8H) |
| **EGFR_ 3365_3** | 474.3 | 10.05 (s, 1H), 8.77 (s, 1H), 8.42 (s, 1H), 8.25 (d, J = 5.1 Hz, 1H), 7.84 - 7.78 (m, 2H), 7.71 (t, J = 7.4 Hz, 1H), 7.58 (t, J = 7.6 Hz, 2H), 7.09 (s, 1H), 6.96 (s, 1H), 6.88 (dd, J = 5.1, 1.1 Hz, 1H), 6.37 (dd, J = 17.0, 10.1 Hz, 1H), 6.22 (dd, J = 16.9, 1.9 Hz, 1H), 5.73 (d, J = 11.7 Hz, 1H), 3.80 (s, 3H), 2.85 (t, J = 5.6 Hz, 2H), 2.69 (s, 4H), 2.28 (t, J = 5.7 Hz, 2H), 2.19 (s, 7H) |
| **EGFR_ 3365_4** | 474.3 | 9.57 (s, 1H), 9.28 (s, 1H), 8.68 (s, 1H), 8.44 (s, 1H), 8.19 (d, J = 5.3 Hz, 1H), 7.69 (d, J = 9.0 Hz, 2H), 7.10 (s, 1H), 6.96 (s, 1H), 6.84 (d, J = 5.3 Hz, 1H), 6.78 (d, J = 9.1 Hz, 2H), 6.62 (dd, J = 16.9, 10.2 Hz, 1H), 6.30 (dd, J= 17.0, 1.8 Hz, 1H), 5.79 (dd, J = 10.2, 1.7 Hz, 1H), 3.86 (s, 3H), 3.33 - 3.20 (m, 4H), 3.05 (s, 7H), 2.80 (d, J = 4.6 Hz, 7H), 2.59 (s, 3H) |
| **EGFR_ 3365_4a** | 474.3 | - |
| **EGFR_ 3365_5** | 559.3 | 9.57 (s, 1H), 9.31 (s, 1H), 8.71 (s, 1H), 8.45 (s, 1H), 8.21 (d, *J* = 5.3 Hz, 1H), 7.72 (d, J = 9.0 Hz, 2H), 7.14 (s, 1H), 7.05 (d, *J* = 9.1 Hz, 2H), 6.97 (s, 1H), 6.87 (d, *J* = 6.5 Hz, 1H), 6.62 (dd, *J* = 16.9, 10.2 Hz, 1H), 6.31 (dd, *J* = 17.0, 1.8 Hz, 1H), 5.80 (dd, *J* = 10.2, 1.8 Hz, 1H), 3.87 (s, 3H), 3.79 - 3.71 (m, 4H), 3.40 - 3.33 (m, 4H), 3.32 - 3.21 (m, 4H), 2.80 (d, *J* = 4.7 Hz, 6H), 2.60 (s, 3H) |
| **EGFR_ 3365_5a** | 559.3 | - |
| **EGFR_ 3365_10** | 493.3 | 10.03 (s, 1H), 8.69 (d, *J* = 15.5 Hz, 2H), 8.48 (s, 1H), 8.16 - 8.10 (m, 2H), 7.30 (t, *J* = 8.9 Hz, 2H), 7.16 (d, *J* = 4.9 Hz, 1H), 7.00 (s, 1H), 6.37 (dd, *J* = 16.9, 10.1 Hz, 1H), 6.20 (d, *J* = 16.9 Hz, 1H), 5.74 (d, *J* = 12.0 Hz, 1H), 3.79 (s, 3H), 2.86 (t, *J* = 5.8 Hz, 2H), 2.70 (s, 3H), 2.29 (t, *J* = 5.8 Hz, 2H), 2.19 (s, 6H) |

(continued)

| Code | ESI-MS $[M+H]^+$ | $^1H$ NMR (400 MHz, DMSO-$d_6$), δ |
|---|---|---|
| **EGFR_ 3365_11** | 493.2 | 9.96 (s, 1H), 8.75 (s, 1H), 8.68 (d, *J* = 4.9 Hz, 1H), 8.43 (s, 1H), 7.89-7.77 (m, 2H), 7.58-7.45 (m, 2H), 7.19 (d, *J* = 4.9 Hz, 1H), 6.99 (s, 1H), 6.44-6.32 (m, 1H), 6.24-6.14 (m, 1H), 5.78-5.69 (m, 1H), 3.79 (s, 3H), 2.95-2,85 (m, 2H), 2.69 (s, 3H), 2.44-2.34 (m, 2H), 2.30 (s, 6H) |
| **EGFR_ 3365_12** | 555.1 | 9.93 (s, 1H), 8.70 (d, *J* = 4.9 Hz, 1H), 8.53 (s, 1H), 8.28 (s, 1H), 8.19 (s, 1H), 7.69 - 7.64 (m, 1H), 7.58 - 7.54 (m, 1H), 7.48 - 7.42 (m, 2H), 7.26 (d, *J* = 4.8 Hz, 1H), 6.91 (s, 1H), 6.43 (dd, *J* = 16.9, 10.1 Hz, 1H), 6.24 (dd, *J* = 17.0, 2.0 Hz, 1H), 5.76 (dd, *J* = 10.1, 1.9 Hz, 1H), 3.74 (s, 3H), 2.89 (t, *J* = 5.8Hz, 2H), 2.66 (s, 3H), 2.41 (t, *J* = 5.7 Hz, 2H), 2.28 (s, 6H) |
| **EGFR_ 3365_13** | 555.1 | 10.01 (s, 1H), 8.73 (s, 1H), 8.68 (d, *J* = 4.9 Hz, 1H), 8.46 (s, 1H), 8.01 - 7.94 (m, 2H), 7.72 - 7.65 (m, 2H), 7.18 (d, *J* = 4.9 Hz, 1H), 7.02 (s, 1H), 6.39 (dd, *J* = 16.9, 10.1 Hz, 1H), 6.22 (dd, *J* = 16.9, 2.0 Hz, 1H), 5.75 (dd, *J* = 10.0, 1.9 Hz, 1H), 3.79 (s, 3H), 2.90 (t, *J* = 5.7 Hz, 2H), 2.70 (s, 3H), 2.37 (s, 2H), 2.25 (s, 6H) |
| **EGFR_ 3365_14** | 500.2 | 9.43 (s, 1H), 9.22 (s, 1H), 8.74 (d, *J* = 4.8 Hz, 1H), 8.69 (s, 1H), 8.21 - 8.11 (m, 3H), 7.95 (d, *J* = 8.3 Hz, 2H), 7.28 (d, *J* = 4.8 Hz, 1H), 6.99 (s, 1H), 6.59 (dd, *J* = 17.0, 10.3 Hz, 1H), 6.28 (d, *J* = 15.9 Hz, 1H), 5.80 (d, *J* = 10.9 Hz, 1H), 3.85 (s, 3H), 3.40 - 3.19 (m, 4H), 2.80 (d, *J* = 4.6 Hz, 6H), 2.59 (s, 3H) |
| **EGFR_ 3365_14a** | 500.2 | - |
| **EGFR_ 3365_15** | 476.3 | 10.02 (s, 1H), 9.15 (d, *J* = 1.6 Hz, 1H), 8.78-8.55 (m, 2H), 8.70 (d, *J* = 4.9 Hz, 1H), 8.46 (s, 1H), 8.40 (dt, *J* = 8.0, 1.9 Hz, 1H), 7.48 (dd, *J* = 7.9, 4.8 Hz, 1H), 7.24 (d, *J* = 4.9 Hz, 1H), 6.99 (s, 1H), 6.4-6.25 (m, 1H), 6.18 (dd, *J* = 16.9, 2.0 Hz, 1H), 5.73 (dd, *J* = 10.1, 1.9 Hz, 1H), 3.79 (s, 3H), 2.86 (t, *J* = 5.7 Hz, 2H), 2.70 (s, 3H), 2.29 (t, *J* = 5.8 Hz, 2H), 2.20 (s, 6H) |
| **EGFR_ 3365_16** | 581.3 | 10.02 (s, 1H), 8.65 (s, 1H), 8.62 (d, J = 4.9 Hz, 1H), 8.50 (s, 1H), 8.05 - 8.01 (m, 1H), 7.49 - 7.31 (m, 1H), 7.10 (d, J = 9.0 Hz, 1H), 7.07 (d, J = 4.9 Hz, 1H), 6.99 (s, 1H), 6.37 (dd, J= 16.9, 10.1 Hz, 1H), 6.21 (dd, J = 16.9, 2.0 Hz, 1H), 5.72 (dd, J = 10.1, 1.9 Hz, 1H), 5.20 (s, 1H), 3.78 (s, 1H), 2.85 (t, J = 5.7 Hz, 1H), 2.70 (s, 1H), 2.29 (t, J = 5.8 Hz, 1H), 2.17 (s, 1H) |
| **EGFR_ 3365_17** | 567.3 | 10.02 (s, 1H), 8.70 (s, 1H), 8.65 (d, *J* = 4.9 Hz, 1H), 8.51 (s, 1H), 8.10 (d, *J* = 8.9 Hz, 2H), 7.53-7.43 (m, 2H), 7.31-7.22 (m, 1H), 7.16-7.10 (m, 3H), 7.05-6.96 (m, 3H), 6.41-6.28 (m, 1H), 6.23-6.12 (m, 1H), 5.71-5.65 (m, 1H), 3.77 (s, 3H), 2.91-2.80 (m, 2H), 2.68 (s, 3H), 2.38-2.26 (m, 2H), 2.23 (s, 6H) |
| **EGFR_ 3365_26** | 490.3 | 10.00 (s, 1H), 8.98 (d, *J* = 1.8 Hz, 1H), 8.72 (s, 1H), 8.69 (d, *J* = 4.9 Hz, 1H), 8.61 (d, *J* = 1.7 Hz, 1H), 8.48 (s, 1H), 8.20-8.16 (m, 1H), 7.22 (d, *J* = 4.9 Hz, 1H), 6.98 (s, 1H), 6.35 (dd, *J* = 16.9, 10.1 Hz, 1H), 6.15 (dd, *J* = 16.9, 2.0 Hz, 1H), 5.72 (dd, *J* = 10.1, 1.9 Hz, 1H), 3.79 (s, 3H), 2.86 (t, *J* = 5.8 Hz, 2H), 2.70 (s, 3H), 2.37-2.28 (m, 2H), 2.34 (s, 3H), 2.21 (s, 6H) |
| **EGFR_ 3365_28** | 505.4 | 10.02 (s, 1H), 8.65 (s, 1H), 8.62 (d, J = 4.9 Hz, 1H), 8.51 (s, 1H), 8.05 - 8.02 (d, J = 9.0 Hz, 2H), 8.05 - 8.01 (d, J = 9.0 Hz, 2H), 7.07 (d, J = 4.9 Hz, 1H), 7.01 (d, J = 9.0 Hz, 2H), 7.00 (s, 1H), 6.37 (dd, J = 16.9, 10.1 Hz, 1H), 6.20 (dd, J = 16.9, 2.0 Hz, 1H), 5.73 (dd, J = 10.1, 2.0 Hz, 1H), 3.84 (s, 3H), 3.78 (s, 3H), 2.85 (t, J = 5.7 Hz, 2H), 2.70 (s, 3H), 2.29 (t, J = 5.8 Hz, 2H), 2.18 (s, 6H) |
| **EGFR_ 3365_29** | 519.3 | 10.02 (s, 1H), 8.65 (s, 1H), 8.62 (d, J = 4.9 Hz, 1H), 8.50 (s, 1H), 8.01 (d, J = 8.9 Hz, 2H), 7.06 (d, J = 4.9 Hz, 1H), 7.01 - 6.96 (m, 3H), 6.37 (dd, J = 16.9, 10.1 Hz, 1H), 6.20 (dd, J = 16.9, 1.9 Hz, 1H), 5.73 (dd, J = 10.1,1.8 Hz, 1H), 4.11 (q, J = 7.0 Hz, 2H), 3.78 (s, 3H), 2.85 (t, J = 5.7 Hz, 2H), 2.70 (s, 3H), 2.29 (t, J = 5.8 Hz, 2H), 1.35 (t, J = 7.0 Hz, 3H) |

(continued)

| Code | ESI-MS [M+H]+ | 1H NMR (400 MHz, DMSO-$d_6$), δ |
|---|---|---|
| **EGFR_ 3365_30** | 533.3 | 10.02 (s, 1H), 8.65 (s, 1H), 8.62 (d, J = 4.9 Hz, 1H), 8.50 (s, 1H), 8.02 (d, J = 8.9 Hz, 2H), 7.07 (d, J = 4.9 Hz, 1H), 7.01- 6.98 (m, 3H), 6.37 (dd, J = 16.9, 10.1 Hz, 1H), 6.20 (dd, J = 16.9, 1.9 Hz, 1H), 5.76 - 5.69 (m, 1H), 4.01 (t, J = 6.5 Hz, 2H), 3.78 (s, 3H), 2.86 (t, J = 5.7 Hz, 2H), 2.69 (s, 3H), 2.30 (t, J = 5.7 Hz, 2H), 2.19 (s, 6H), 1.80 - 1.69 (m, 2H), 0.98 (t, J = 7.4 Hz, 3H) |
| **EGFR_ 3365_30a** | 535.3 | 1H NMR (400 MHz, DMSO) δ 10.10 (s, 1H), 9.03 (s, 1H), 8.39 (d, J = 5.1 Hz, 1H), 7.93 (s, 1H), 7.39 (d, J = 8.6 Hz, 2H), 7.04 - 6.93 (m, 2H), 6.83 (d, J = 8.7 Hz, 2H), 6.42 (dd, J = 16.9, 10.0 Hz, 1H), 6.27 (dd, J = 16.9, 2.0 Hz, 1H), 6.00 (d, J = 4.3 Hz, 1H), 5.76 (dd, J = 10.0, 1.9 Hz, 1H), 5.48 (d, J = 4.0 Hz, 1H), 3.86 (t, J = 6.5 Hz, 2H), 3.82 (s, 3H), 2.86 (t, J = 5.7 Hz, 2H), 2.69 (s, 3H), 2.28 (t, J = 5.7 Hz, 2H), 2.20 (s, 6H), 1.74 - 1.63 (m, 2H), 0.95 (t, J = 7.4 Hz, 3H) |
| **EGFR_ 3365_31** | 533.3 | 9.90 (s, 1H), 8.64 (s, 1H), 8.62 (d, J = 4.9 Hz, 1H), 8.42 (s, 1H), 8.15 (s, 1H), 8.00 (d, J = 8.9 Hz, 2H), 7.07 (d, J = 4.9 Hz, 1H), 7.01 - 6.97 (m, 3H), 6.42 (dd, J = 16.9, 10.2 Hz, 1H), 6.23 (dd, J = 16.9, 1.8 Hz, 1H), 5.79 - 5.71 (m, 1H), 4.74 (dt, J = 12.0, 6.0 Hz, 1H), 3.80 (s, 3H), 2.97 (s, 2H), 2.67 (s, 4H), 2.54 (s, 1H), 2.35 (s, 6H) |
| **EGFR_ 3365_31a** | 535.3 | 1H NMR (400 MHz, DMSO) δ 10.08 (s, 1H), 8.99 (s, 1H), 8.38 (d, J = 5.1 Hz, 1H), 7.93 (s, 1H), 7.37 (d, J = 8.6 Hz, 2H), 7.03 - 6.93 (m, 2H), 6.81 (d, J = 8.7 Hz, 2H), 6.42 (dd, J = 16.9, 10.1 Hz, 1H), 6.26 (dd, J = 16.9, 2.0 Hz, 1H), 5.97 (d, J = 4.3 Hz, 1H), 5.76 (dd, J = 10.1, 1.9 Hz, 1H), 5.46 (d, J = 4.5 Hz, 1H), 4.58 - 4.48 (m, 1H), 3.82 (s, 3H), 2.87 (t, J = 5.6 Hz, 2H), 2.69 (s, 3H), 2.29 (s, 2H), 2.21 (s, 6H), 1.23 (d, J = 6.0 Hz, 6H) |
| **EGFR_ 3365_32** | 505.4 | 10.00 (s, 1H), 8.66 (s, 1H), 8.64 (d, J = 4.9 Hz, 1H), 8.47 (s, 1H), 8.22 (s, 1H), 7.60 - 7.54 (m, 1H), 7.49 (dd, J = 2.5, 1.5 Hz, 1H), 7.41 (t, J = 7.9 Hz, 1H), 7.23 (ddd, J = 8.2, 2.7, 0.8 Hz, 1H), 7.10 (d, J = 4.9 Hz, 1H), 6.97 (s, 1H), 6.37 (dd, J = 16.9, 10.1 Hz, 1H), 6.19 (dd, J = 16.9, 2.0 Hz, 1H), 5.73 (dd, J = 10.1, 1.9 Hz, 1H), 3.77 (d, J = 5.1 Hz, 7H), 2.86 (t, J = 5.8 Hz, 2H), 2.69 (s, 3H), 2.31 (t, J = 5.8 Hz, 2H), 2.20 (s, 6H) |
| **EGFR_ 3365_33** | 505.2 | 9.98 (s, 1H), 8.61 (d, J = 4.9 Hz, 1H), 8.38 (s, 2H), 8.20 (s, 1H), 7.52 (td, J = 9.1, 1.5 Hz, 2H), 7.10 (d, J = 8.4 Hz, 1H), 7.07 - 7.00 (m, 2H), 6.91 (s, 1H), 6.40 (dd, J = 16.9, 10.1 Hz, 1H), 6.22 (dd, J = 16.9, 1.9 Hz, 1H), 5.78 - 5.69 (m, 1H), 3.73 (s, 3H), 3.62 (s, 4H), 2.86 (t, J = 5.7 Hz, 2H), 2.66 (s, 3H), 2.34 (t, J = 5.7 Hz, 2H), 2.23 (s, 6H) |
| **EGFR_ 3365_34** | 520.2 | 9.92 (s, 1H), 8.77-8.71 (m, 2H), 8.67 (s, 1H), 8.52-8.40 (m, 3H), 7.75 (t, *J* = 8.0 Hz, 1H), 7.29 (d, *J* = 4.8 Hz, 1H), 6.97 (s, 1H), 6.38-6.28 (m, 1H), 6.15-6.06 (m, 1H), 5.73-5.67 (m, 1H), 3.79 (s, 3H), 2.86 (t, *J* = 5.7 Hz, 2H), 2.68 (s, 3H), 2.34 (t, *J* = 5. 6 Hz, 2H), 2.22 (s, 6H) |
| **EGFR_ 3365_36** | 520.2 | 9.95 (s, 1H), 8.70 (d, *J* = 4.9 Hz, 1H), 8.49 (s, 1H), 8.11 (s, 1H), 8.05 (d, *J* = 8.1 Hz, 1H), 7.85 (m, 1H), 7.75 (m, 1H), 7.67 (dd, *J* = 7.5, 1.1 Hz, 1H), 7.37 (d, *J* = 4.9 Hz, 1H), 6.84 (s, 1H), 6.37 (m, 1H), 6.21 (dd, *J* = 16.9, 2.0 Hz, 1H), 5.76 (dd, *J* = 10.1, 2.0 Hz, 1H), 3.62 (s, 3H), 2.86 (t, *J* = 5.7 Hz, 2H), 2.69 (s, 3H), 2.34 (t, *J* = 5.7 Hz, 2H), 2.23 (s, 6H) |
| **EGFR_ 3365_50** | 531.3 | δ 10.06 (s, 1H), 8.79 - 8.77 (m, 1H), 8.38 (s, 1H), 8.24 (d, J = 4.7 Hz, 1H), 7.80 (d, J = 8.8 Hz, 2H), 7.09 (d, J = 8.8 Hz, 2H), 7.05 - 7.03 (m, 1H), 6.98 (s, 1H), 6.84 (dd, J = 5.1, 1.2 Hz, 1H), 6.40 (dd, J = 16.9, 10.1 Hz, 1H), 6.23 (dd, J = 16.9, 1.9 Hz, 1H), 5.74 (dd, J = 10.1, 1.8 Hz, 1H), 4.05 (t, J = 6.5 Hz, 2H), 3.81 (s, 3H), 2.87 (t, J = 5.7 Hz, 2H), 2.69 (s, 3H), 2.32 (t, J = 5.7 Hz, 2H), 2.22 (s, 6H), 1.83 - 1.70 (m, 2H), 1.00 (t, J = 7.4 Hz, 3H) |

(continued)

| Code | ESI-MS [M+H]+ | $^1$H NMR (400 MHz, DMSO-$d_6$), δ |
|---|---|---|
| **EGFR_ 3365_51** | 503.3 | δ 10.06 (s, 1H), 8.78 (s, 1H), 8.37 (s, 1H), 8.24 (d, J = 5.1 Hz, 1H), 7.82 (d, J = 8.8 Hz, 2H), 7.11 (d, J = 8.9 Hz, 2H), 7.04 (s, 1H), 6.98 (s, 1H), 6.84 (dd, J = 5.1, 1.2 Hz, 1H), 6.39 (dd, J = 17.0, 10.0 Hz, 1H), 6.23 (dd, J = 16.9, 2.0 Hz, 1H), 5.74 (dd, J = 10.1, 1.9 Hz, 1H), 3.87 (s, 3H), 3.81 (s, 3H), 2.86 (t, J = 5.7 Hz, 2H), 2.70 (s, 3H), 2.30 (t, J = 5.7 Hz, 2H), 2.20 (s, 6H) |
| **EGFR_ 3365_52** | 517.3 | δ 10.05 (s, 1H), 8.76 (s, 1H), 8.39 (s, 1H), 8.23 (d, J = 5.2 Hz, 1H), 7.80 (d, J = 8.8 Hz, 2H), 7.09 (d, J = 8.8 Hz, 2H), 7.04 (s, 1H), 6.97 (s, 1H), 6.84 (dd, J = 5.1, 1.2 Hz, 1H), 6.41 (dd, J = 16.9, 10.1 Hz, 1H), 6.23 (dd, J = 16.9, 2.0 Hz, 1H), 5.74 (dd, J = 10.1, 1.9 Hz, 1H), 4.15 (q, J = 7.0 Hz, 2H), 3.81 (s, 3H), 2.88 (t, J = 5.8 Hz, 2H), 2.68 (s, 3H), 2.36 (t, J = 5.7 Hz, 2H), 2.24 (s, 6H), 1.37 (t, J = 7.0 Hz, 3H) |
| **EGFR_ 3365_53** | 558.7 | δ 10.06 (s, 1H), 8.77 (s, 1H), 8.30 (s, 1H), 8.20 (d, J = 5.1 Hz, 1H), 7.68 (d, J = 8.7 Hz, 2H), 6.97 (d, J = 6.2 Hz, 2H), 6.78 (dd, J = 5.1, 1.1 Hz, 1H), 6.48 (d, J = 8.8 Hz, 2H), 6.38 (dd, J = 16.9, 10.0 Hz, 1H), 6.23 (dd, J = 16.9, 2.0 Hz, 1H), 5.74 (dd, J = 10.1, 1.8 Hz, 1H), 4.37 (dd, J = 8.0, 4.2 Hz, 1H), 4.24 - 4.13 (m, 2H), 3.81 (s, 3H), 3.80- 3.76 (m, 2H), 3.27 (s, 3H), 2.85 (t, J = 5.6 Hz, 2H), 2.70 (s, 3H), 2.29 (t, J = 5.7 Hz, 2H), 2.20 (s, 6H) |
| **EGFR_ 3365_54** | 544.3 | δ 10.07 (s, 1H), 8.78 (s, 1H), 8.29 (s, 1H), 8.19 (d, J = 5.1 Hz, 1H), 7.67 (d, J = 9.1 Hz, 2H), 6.98 - 6.97 (m, 2H), 6.78 (dd, J = 5.1, 1.2 Hz, 1H), 6.74 (d, J = 9.1 Hz, 2H), 6.38 (dd, J = 16.9, 10.0 Hz, 1H), 6.23 (dd, J = 16.9, 2.0 Hz, 1H), 5.74 (dd, J = 10.0, 1.9 Hz, 1H), 3.81 (s, 3H), 3.44 (q, J = 6.9 Hz, 4H), 2.85 (t, J = 5.7 Hz, 2H), 2.69 (s, 3H), 2.29 (t, J = 5.7 Hz, 2H), 2.19 (s, 6H), 1.13 (t, J = 7.0 Hz, 6H) |
| **EGFR_ 3365_55** | 571.3 | δ 10.05 (s, 1H), 8.82-8.70 (m, 1H), 8.34 (s, 1H), 8.21 (d, J = 5.1 Hz, 1H), 7.69 (d, J = 8.9 Hz, 2H), 7.08-6.94 (m, 4H), 6.80 (d, J = 5.1 Hz, 1H), 6.48-6.33 (m, 1H), 6.23 (dd, J = 17.0, 1.8 Hz, 1H), 5.74 (dd, J = 10.1, 1.7 Hz, 1H), 3.81 (s, 3H), 3.44-3.32 (m, 4H), 2.97-2.78 (m, 2H), 2.69 (s, 3H), 2.47-2.39 (m, 4H), 2.39-2.29 (m, 2H), 2.28-2.14 (m, 9H) |
| **EGFR_ 3365_56** | 542.3 | δ = 10.04 (s, 1H), 8.75 (s, 1H), 8.30 (s, 1H), 8.19 (d, J = 5.1 Hz, 1H), 7.71 - 7.64 (m, 2H), 6.99 - 6.94 (m, 2H), 6.76 (dd, J = 5.1, 1.4 Hz, 1H), 6.65 - 6.57 (m, 2H), 6.40 (dd, J = 16.9, 10.0 Hz, 1H), 6.22 (dd, J = 16.9, 2.1 Hz, 1H), 5.73 (dd, J = 10.1, 2.1 Hz, 1H), 3.81 (s, 3H), 3.34 (t, J = 6.5 Hz, 4H), 2.87 (t, J = 5.8 Hz, 2H), 2.68 (s, 3H), 2.33 (t, J = 5.8 Hz, 2H), 2.22 (s, 6H), 1.98 (t, J = 6.5 Hz, 4H) |
| **EGFR_ 3365_57** | 487.6 | δ 10.05 (s, 1H), 8.77 (s, 1H), 8.39 (s, 1H), 8.25 (d, J = 5.2 Hz, 1H), 7.69 - 7.75 (m, 2H), 7.36 - 7.43 (m, 2H), 7.07 (s, 1H), 6.97 (s, 1H), 6.86 (dd, J = |
| | | 5.1, 1.1 Hz, 1H), 6.34 - 6.43 (m, 1H), 6.20 - 6.27 (m, 1H), 5.74 (dd, J = 10.2, 1.8 Hz, 1H), 3.81 (s, 3H), 2.86 (t, J = 5.7 Hz, 2H), 2.69 (s, 3H), 2.42 (s, 3H), 2.30 (t, J = 5.7 Hz, 2H), 2.20 (s, 6H) |
| **EGFR_ 3365_58** | 529.6 | δ 10.01 (s, 1H), 8.58 (d, J = 4.9 Hz, 1H), 8.55 (d, J = 12.7 Hz, 2H), 7.87 (d, J = 8.8 Hz, 2H), 7.02 - 6.95 (m, 2H), 6.46 - 6.38 (m, 1H), 6.36 (d, J = 8.9 Hz, 2H), 6.23 (dd, J = 17.0, 1.7 Hz, 1H), 5.75 (d, J = 11.7 Hz, 1H), 3.97 (t, J = 7.4 Hz, 4H), 3.79 (s, 3H), 2.88 (t, J = 5.8 Hz, 2H), 2.70 (s, 3H), 2.43-2.36 (m, 2H), 2.34 (t, J = 5.6 Hz, 2H), 2.22 (s, 6H) |
| **EGFR_ 3365_61** | 559.7 | δ 10.02 (s, 1H), 8.58 (d, J = 4.8 Hz, 2H), 8.53 (s, 1H), 7.88 (d, J = 8.8 Hz, 2H), 6.99 (d, J = 4.8 Hz, 2H), 6.48 - 6.35 (m, 3H), 6.30 - 6.17 (m, 1H), 5.75 (d, J = 11.8 Hz, 1H), 4.40-4.32 (m, 1H), 4.22-4.11 (m, 2H), 3.79 (s, 3H), 3.78-3.75 (m, 2H), 3.27 (s, 3H), 2.87 (t, J = 5.7 Hz, 2H), 2.70 (s, 3H), 2.33 (d, J = 5.8 Hz, 2H), 2.20 (s, 6H) |
| **EGFR_ 3365_62** | 609.5 | δ 9.85 (s, 1H), 8.60 (d, J = 4.9 Hz, 1H), 8.57 (s, 1H), 8.41 (s, 1H), 7.90 (d, J = 9.0 Hz, 2H), 7.02 (d, J = 4.9 Hz, 1H), 6.98 (d, J = 4.7 Hz, 2H), 6.95 (s, 1H), 6.70-6.57 (m, 1H), 6.30 - 6.19 (m, 1H), 5.74 (d, J = 11.7 Hz, 1H), 3.82 (s, 3H), 3.43 - 3.35 (m, 6H), 3.08 (s, 2H), 2.78 (s, 2H), 2.65 (s, 3H), 2.48-2.46 (m, 8H), 2.26 (s, 3H) |

(continued)

| Code | ESI-MS $[M+H]^+$ | $^1H$ NMR (400 MHz, DMSO-$d_6$), δ |
|---|---|---|
| EGFR_ 3365_62a | 609.5 | - |
| EGFR_ 3365_63 | 543.3 | δ 10.01 (s, 1H), 8.58 - 8.55 (m, 3H), 7.88 (d, J = 9.0 Hz, 2H), 7.00 - 6.97 (m, 2H), 6.56 (d, J = 9.0 Hz, 2H), 6.39 (dd, J= 16.9, 10.1 Hz, 1H), 6.21 (dd, J = 16.9, 2.0 Hz, 1H), 5.73 (dd, J = 10.1, 1.8 Hz, 1H), 3.79 (s, 3H), 3.35 - 3.32 (m, 4H), 2.86 (t, J = 5.8 Hz, 2H), 2.70 (s, 3H), 2.31 (t, J = 5.8 Hz, 2H), 2.19 (s, 6H), 2.00 - 1.97 (m, 4H) |
| EGFR_ 3365_64 | 488.3 | δ 10.04 (s, 1H), 9.23 (s, 1H), 8.37 - 8.22 (m, 2H), 7.79 (d, J = 8.1 Hz, 2H), 7.34 (d, J = 8.1 Hz, 2H), 7.01 (s, 1H), 6.67 (d, J = 4.5 Hz, 1H), 6.37 (dd, J = 16.9, 10.1 Hz, 1H), 6.22 (dd, J = 16.9, 1.8 Hz, 1H), 5.81 - 5.68 (m, 1H), 3.79 (s, 3H), 2.85 (t, J = 5.6 Hz, 2H), 2.71 (s, 3H), 2.38 (s, 3H), 2.31 (t, J = 5.7 Hz, 2H), 2.19 (s, 6H) |
| EGFR_ 3365_66 | 546.3 | δ 10.06 (s, 1H), 8.68 (s, 1H), 8.63 (d, J = 4.9 Hz, 1H), 8.51 (s, 1H), 8.03 (d, J = 8.9 Hz, 2H), 7.08 (d, J = 4.9 Hz, 1H), 7.03 - 6.98 (m, 3H), 6.38 (dd, J = 16.9, 10.0 Hz, 1H), 6.21 (dd, J = 16.9, 2.0 Hz, 1H), 5.74 (dd, J = 10.1, 1.9 Hz, 1H), 4.06 (t, J = 6.5 Hz, 2H), 3.79 (s, 3H), 2.86 (t, J = 5.7 Hz, 2H), 2.71 (s, 3H), 2.29 (t, J = 5.7 Hz, 2H), 2.19 (s, 6H), 1.77 - 1.68 (m, 2H), 1.50 - 1.39 (m, 2H), 0.95 (t, J = 7.4 Hz, 3H) |
| EGFR_ 3365_67 | 572.7 | δ 10.06 (s, 1H), 8.63 (m, 2H), 8.52 (s, 1H), 8.51 (s, 1H), 8.01 (d, J = 8.9 Hz, 2H), 7.07 (d, J = 4.9 Hz, 1H), 7.04 - 6.94 (m, 3H), 6.38 (dd, J = 16.9, 10.1 Hz, 1H), 6.21 (dd, J = 16.9, 1.8 Hz, 1H), 5.74 (dd, J = 10.1, 1.7 Hz, 1H), 4.48 (m, 1H), 3.79 (s, 3H), 2.86 (t, J = 5.6 Hz, 2H), 2.70 (s, 3H), 2.30 (t, J = 5.6 Hz, 2H), 2.20 (s, 6H), 1.99 - 1.88 (m, 2H), 1.77 - 1.65 (m, 2H), 1.61 - 1.21 (m, 6H) |
| EGFR_ 3365_68 | 562.7 | δ 10.00 (s, 1H), 8.58 - 8.49 (m, 2H), 8.32 (s, 1H), 7.64 (d, J = 8.6 Hz, 1H), 6.95 (s, 1H), 6.87 (d, J = 4.9 Hz, 1H), 6.64 (dd, J = 8.7, 2.2 Hz, 1H), 6.56 (d, J = 2.2 Hz, 1H), 6.36 (dd, J = 16.9, 10.0 Hz, 1H), 6.21 (dd, J = 17.0, 2.2 Hz, 1H), 5.73 (dd, J = 9.9, 2.2 Hz, 1H), 4.13 (q, J = 7.0 Hz, 2H), 3.89 (q, J = 6.9 Hz, 2H), 3.76 (s, 3H), 2.85 (t, J = 5.9 Hz, 2H), 2.69 (s, 3H), 2.30 (t, J = 5.8 Hz, 2H), 2.20 (s, 6H), 1.36 (t, J = 6.9 Hz, 3H), 0.87 (t, J = 6.9 Hz, 3H) |
| EGFR_ 3365_69 | 534.6 | δ 10.00 (s, 1H), 8.56 (d, J = 4.9 Hz, 1H), 8.53 (s, 1H), 8.32 (s, 1H), 7.58 (d, J = 8.3 Hz, 1H), 6.98 - 6.86 (m, 2H), 6.66 - 6.60 (m, 2H), 6.37 (dd, J = 16.9, 10.0 Hz, 1H), 6.21 (dd, J = 16.9, 2.1 Hz, 1H), 5.73 (dd, J = 10.0, 2.2 Hz, 1H), 3.85 (s, 3H), 3.76 (s, 3H), 3.61 (s, 3H), 2.85 (t, J = 5.9 Hz, 2H), 2.67 (s, 3H), 2.31 (t, J = 5.8 Hz, 2H), 2.21 (s, 6H) |
| EGFR_ 3365_70 | 590.3 | δ 10.00 (s, 1H), 8.56 - 8.48 (m, 2H), 8.33 (s, 1H), 7.62 (d, J = 8.6 Hz, 1H), 6.94 (s, 1H), 6.89 (dd, J = 4.9, 2.2 Hz, 1H), 6.64 (dd, J = 8.7, 2.1 Hz, 1H), 6.56 (d, J = 2.1 Hz, 1H), 6.37 (dd, J = 16.9, 10.0 Hz, 1H), 6.21 (dd, J = 16.9, 1.9 Hz, 1H), 5.77 - 5.69 (m, 1H), 4.02 (t, J = 6.5 Hz, 2H), 3.81 (t, J = 6.3 Hz, 2H), 3.75 (s, 3H), 2.85 (t, J = 5.7 Hz, 2H), 2.68 (s, 3H), 2.31 (t, J = 5.7 Hz, 2H), 2.21 (s, 6H), 1.82 - 1.70 (m, 2H), 1.32 - 1.24 (m, 2H), 1.00 (t, J = 7.4 Hz, 3H), 0.66 (t, J = 7.4 Hz, 3H) |
| EGFR_ 3365_71 | 590.7 | δ 10.01 (s, 1H), 8.57 (s, 1H), 8.52 (d, J = 4.9 Hz, 1H), 8.28 (s, 1H), 7.64 (d, J = 8.7 Hz, 1H), 6.96 (s, 1H), 6.84 (d, J = 4.9 Hz, 1H), 6.62 (dd, J = 8.7, 2.0 Hz, 1H), 6.52 (d, J = 2.0 Hz, 1H), 6.37 (dd, J = 16.9, 10.0 Hz, 1H), 6.22 (dd, J = 16.9, 2.0 Hz, 1H), 5.73 (dd, J = 10.0, 2.0 Hz, 1H), 4.77 (m, 2H), 4.59 (m, 2H), 3.76 (s, 3H), 2.85 (t, J = 5.7 Hz, 2H), 2.69 (s, 3H), 2.30 (t, J = 5.7 Hz, 2H), 2.20 (s, 6H), 1.31 (d, J = 6.0 Hz, 3H), 0.95 (d, J = 6.0 Hz, 3H) |
| EGFR_ 3365_72 | 545.7 | δ 10.02 (s, 1H), 8.61 - 8.53 (m, 3H), 7.86 (d, J = 9.1 Hz, 2H), 7.01 - 6.94 (m, 2H), 6.68 (d, J = 9.2 Hz, 2H), 6.37 (d, J = 10.1 Hz, 1H), 6.22 (dd, J = 16.9, 1.9 Hz, 1H), 5.74 (d, J = 11.7 Hz, 1H), 3.79 (s, 3H), 3.43 (q, J = 7.0 Hz, 4H), 2.90 - 2.87 (m, 2H), 2.70 (s, 3H), 2.35-2.30 (m, 2H), 2.20 (s, 6H), 1.12 (t, J = 7.0 Hz, 6H) |

(continued)

| Code | ESI-MS $[M+H]^+$ | $^1H$ NMR (400 MHz, DMSO-$d_6$), δ |
|---|---|---|
| **EGFR_ 3365_73** | 517.6 | δ 10.02 (s, 1H), 8.56 (dd, J = 10.7, 5.0 Hz, 3H), 7.89 (d, J = 9.1 Hz, 2H), 7.02 - 6.95 (m, 2H), 6.71 (d, J = 9.1 Hz, 2H), 6.39 (dd, J = 16.9, 10.1 Hz, 1H), 6.22 (dd, J = 16.9, 1.9 Hz, 1H), 5.72 (s, 1H), 3.79 (s, 3H), 3.04 (s, 6H), 2.87 (t, J = 5.8 Hz, 2H), 2.70 (s, 3H), 2.31 (t, J = 5.8 Hz, 2H), 2.20 (s, 6H) |
| **EGFR_ 3365_77** | 545.3 | δ 10.04 (s, 1H), 8.76 (s, 1H), 8.37 (s, 1H), 8.24 (d, J = 5.1 Hz, 1H), 7.80 (d, J = 8.8 Hz, 2H), 7.10 (d, J = 8.9 Hz, 2H), 7.03 (s, 1H), 6.98 (s, 1H), 6.84 (dd, J = 5.1, 1.3 Hz, 1H), 6.40 (dd, J = 16.8, 10.3 Hz, 1H), 6.23 (dd, J = 16.9, 1.9 Hz, 1H), 5.74 (dd, J = 10.0, 1.9 Hz, 1H), 3.87 (d, J = 6.5 Hz, 2H), 3.81 (s, 3H), 2.88 (s, 2H), 2.69 (s, 3H), 2.42 - 2.12 (m, 8H), 2.06 (dt, J = 13.3, 6.6 Hz, 1H), 1.00 (d, J = 6.7 Hz, 6H) |
| **EGFR_ 3365_78** | 546.3 | δ 10.04 (s, 1H), 9.21 (s, 1H), 8.31 - 8.30 (m, 2H), 7.85 (d, J = 8.9 Hz, 2H), 7.11 - 6.95 (m, 3H), 6.67 (s, 1H), 6.39 (dd, J = 16.9, 10.1 Hz, 1H), 6.27 - 6.14 (m, 1H), 5.74 (d, J = 11.7 Hz, 1H), 3.84 (d, J = 6.5 Hz, 2H), 3.79 (s, 3H), 2.87 (t, J = 5.6 Hz, 2H), 2.71 (s, 3H), 2.35 (t, J = 5.7 Hz, 2H), 2.21 (s, 6H), 2.08 - 2.01 (m, 1H), 0.99 (d, J = 6.7 Hz, 6H) |
| **EGFR_ 3365_85** | 553.3 | δ 10.05 (s, 1H), 8.80 (s, 1H), 8.41 (s, 1H), 8.32 (s, 1H), 8.26 (d, J = 5.1 Hz, 1H), 8.01 (s, 1H), 7.82 - 7.76 (m, 4H), 7.10 (s, 1H), 6.98 (s, 1H), 6.89 (dd, J = 5.1, 1.3 Hz, 1H), 6.39 (dd, J = 16.9, 10.1 Hz, 1H), 6.23 (dd, J = 16.9, 2.0 Hz, 1H), 5.74 (dd, J = 10.0, 2.0 Hz, 1H), 3.90 (s, 3H), 3.82 (s, 3H), 2.86 (t, J = 5.7 Hz, 2H), 2.69 (s, 3H), 2.30 (t, J = 5.8 Hz, 2H), 2.20 (s, 6H) |
| **EGFR_ 3365_86** | 540.6 | δ 10.00 (s, 1H), 8.74 - 8.67 (m, 2H), 8.49 (s, 1H), 8.42 (s, 1H), 8.17 (d, J = 8.7 Hz, 2H), 7.87 (s, 1H), 7.79 (d, J = 8.7 Hz, 2H), 7.23 - 7.16 (m, 2H), 7.03 (s, 1H), 6.31 (dd, J= 16.9, 10.1 Hz, 1H), 6.17 (dd, J = 17.0, 1.7 Hz, 1H), 5.67 (d, J = 11.6 Hz, 1H), 3.81 (s, 3H), 2.84 (t, J = 5.7 Hz, 2H), 2.68 (s, 3H), 2.25 (t, J = 5.7 Hz, 2H), 2.13 (s, 6H) |
| **EGFR_ 3365_87** | 533.6 | δ 10.05 (s, 1H), 8.80 (s, 2H), 8.34 (s, 1H), 8.18 (d, J = 5.2 Hz, 1H), 7.40 (d, J = 8.5 Hz, 1H), 7.08 (s, 1H), 6.96 (s, 1H), 6.82 - 6.77 (m, 1H), 6.72 - 6.63 (m, 2H), 6.38 (dd, J = 16.9, 10.0 Hz, 1H), 6.22 (dd, J = 16.9, 1.9 Hz, 1H), 5.78 - 5.70 (m, 2H), 3.87 (s, 3H), 3.79 (s, 3H), 3.69 (s, 4H), 2.86 (t, J = 5.7 Hz, 2H), 2.69 (s, 3H), 2.30 (t, J = 5.7 Hz, 2H), 2.21 (s, 6H) |
| **EGFR_ 3365_88** | 503.6 | δ 10.05 (s, 1H), 8.76 (s, 2H), 8.40 (s, 1H), 8.20 (d, J = 5.2 Hz, 1H), 7.63 - 7.51 (m, 1H), 7.37 (dd, J = 7.5, 1.5 Hz, 1H), 7.20 (d, J = 8.4 Hz, 1H), 7.14 - 7.04 (m, 2H), 6.95 (s, 1H), 6.84 (dd, J = 5.2, 1.1 Hz, 1H), 6.38 (dd, J = 16.9, 10.0 Hz, 1H), 6.22 (dd, J = 16.9, 1.9 Hz, 1H), 5.74 (dd, J = 10.1, 1.8 Hz, 1H), 3.78 (s, 3H), 3.70 (s, 4H), 2.85 (t, J = 5.7 Hz, 2H), 2.69 (s, 3H), 2.29 (t, J = 5.7 Hz, 2H), 2.20 (s, 6H) |
| **EGFR_ 3365_90** | 557.7 | δ 10.04 (s, 1H), 8.57 (dd, J = 11.2, 9.1 Hz, 3H), 7.88 (d, J = 9.1 Hz, 2H), 7.05 - 6.97 (m, 2H), 6.91 (d, J = 9.2 Hz, 2H), 6.38 (dd, J = 16.9, 10.1 Hz, 1H), 6.22 (dd, J = 16.9, 1.9 Hz, 1H), 5.79 - 5.67 (m, 1H), 3.79 (s, 3H), 3.46 - 3.37 (m, 4H), 2.86 (t, J = 5.7 Hz, 2H), 2.70 (s, 3H), 2.30 (, J = 5.6 Hz, 2H), 2.19 (s, 6H), 1.65-1.55 (m, J = 3.9 Hz, 6H) |
| **EGFR_ 3365_91** | 557.7 | δ 9.96 (s, 1H), 8.67 - 8.63 (m, 2H), 8.48 (s, 1H), 8.08 (d, J = 8.9 Hz, 2H), 7.80 (d, J = 8.9 Hz, 2H), 7.11 (d, J = 4.9 Hz, 1H), 6.98 (s, 1H), 6.48 (dd, J = 16.9, 10.2 Hz, 1H), 6.20 (dd, J = 17.0, 1.8 Hz, 1H), 5.75 - 5.69 (m, 1H), 3.88 (t, J = 7.0 Hz, 2H), 3.79 (s, 3H), 2.94 (t, J = 5.7 Hz, 2H), 2.68 (s, 3H), 2.56 (t, J = 8.1 Hz, 2H), 2.46 (t, J = 5.7 Hz, 2H), 2.28 (s, 6H), 2.15 - 2.04 (m, 2H) |
| **EGFR_ 3365_92** | 548.6 | δ 10.04 (s, 1H), 8.66 (s, 1H), 8.63 (d, J = 4.9 Hz, 1H), 8.51 (s, 1H), 8.03 (d, J = 8.9 Hz, 2H), 7.07 (d, J = 4.9 Hz, 1H), 7.03 (d, J = 8.9 Hz, 2H), 7.00 (s, 1H), 6.41-6.34 (m, 1H), 6.23-6.16 (m, 1H), 5.75-5.71 (m, 1H), 4.21 - 4.16 (m, 2H), 3.79 (s, 3H), 3.71 - 3.66 (m, 2H), 3.32 (s, 3H), 2.86 (t, J = 5.7 Hz, 2H), 2.70 (s, 3H), 2.30 (t, J = 5.7 Hz, 2H), 2.19 (s, 6H) |

(continued)

| Code | ESI-MS $[M+H]^+$ | $^1H$ NMR (400 MHz, DMSO-$d_6$), δ |
|---|---|---|
| EGFR_ 3365_93 | 547.7 | δ 10.05 (s, 1H), 8.77 (s, 1H), 8.37 (s, 1H), 8.23 (d, J = 5.1 Hz, 1H), 7.84 - 7.74 (m, 2H), 7.14 - 7.07 (m, 2H), 7.03 (s, 1H), 6.97 (s, 1H), 6.83 (dd, J = 5.1, 1.4 Hz, 1H), 6.37 (dd, J = 16.9, 10.1 Hz, 1H), 6.22 (dd, J = 16.9, 2.1 Hz, 1H), 5.73 (dd, J = 10.0, 2.1 Hz, 1H), 4.24 - 4.17 (m, 2H), 3.80 (s, 3H), 3.72 - 3.66 (m, 2H), 3.32 (s, 3H), 2.85 (t, J = 5.8 Hz, 2H), 2.69 (s, 3H), 2.28 (t, J = 5.8 Hz, 2H), 2.19 (s, 6H) |
| EGFR 3365_94 | 527.6 | 9.97 (s, 1H), 8.74 (s, 1H), 8.65 (d, J = 4.9 Hz, 1H), 8.49 (s, 1H), 7.70 (d, J = 8.1 Hz, 1H), 7.65 - 7.57 (m, 2H), 7.45 - 7.37 (m, 1H), 7.17 (d, J = 4.9 Hz, 1H), 7.13 (t, J = 7.5 Hz, 1H), 7.03 (s, 1H), 6.38 (dd, J = 16.9, 10.2 Hz, 1H), 6.19 (d, J = 16.9 Hz, 1H), 5.72 (d, J = 10.4 Hz, 1H), 4.08 (s, 3H), 3.82 (s, 3H), 2.89 (s, 2H), 2.69 (s, 3H), 2.38 (s, 2H), 2.23 (s, 6H) |
| EGFR_ 3365_97 | 582.7 | 10.04 (s, 1H), 8.60 - 8.59 (m, 2H), 8.55 (d, J = 7.7 Hz, 1H), 7.91 (d, J = 9.0 Hz, 2H), 7.05 - 6.99 (m, 2H), 6.96 (d, J = 9.0 Hz, 2H), 6.39 (dd, J = 16.8, 10.1 Hz, 1H), 6.22 (d, J = 15.6 Hz, 1H), 5.75 (d, J = 11.5 Hz, 1H), 3.79 (s, 3H), 3.66 - 3.61 (m, 2H), 3.33 -3.27 (m, 2H), 3.19 - 3.11 (m, 1H), 2.86 (t, J = 5.7 Hz, 2H), 2.70 (s, 3H), 2.30 (t, J = 5.7 Hz, 2H), 2.19 (s, 6H), 2.00 - 1.94 (m, 2H), 1.80 - 1.72 (m, 2H) |
| EGFR_ 3365_98 | 587.7 | 10.04 (s, 1H), 8.62 - 8.51 (m, 3H), 7.89 (d, J = 9.1 Hz, 2H), 7.03 - 6.97 (m, 2H), 6.94 (d, J = 9.1 Hz, 2H), 6.39 (dd, J = 16.9, 10.1 Hz, 1H), 6.27 - 6.18 (m, 1H), 5.75 (d, J = 11.6 Hz, 1H), 3.79 (s, 3H), 3.75 - 3.65 (m, 2H), 3.48 - 3.40 (m, 1H), 3.28 (s, 3H), 3.22 - 3.12 (m, 2H), 2.86 (t, J = 5.7 Hz, 2H), 2.70 (s, 3H), 2.31 (t, J = 5.7 Hz, 2H), 2.19 (s, 6H), 1.92 - 1.88 (m, 2H), 1.52 - 1.41 (m, 2H) |
| EGFR_ 3365_101 | 562.7 | 10.03 (s, 1H), 8.60 - 8.52 (m, 2H), 8.33 (s, 1H), 7.57 (d, J = 8.4 Hz, 1H), 6.95 (s, 1H), 6.92 (d, J = 4.9 Hz, 1H), 6.65-6.62 (m, 2H), 6.36 (dd, J = 16.9, 10.0 Hz, 1H), 6.26-6.18 (m, 1H), 5.74 (d, J = 10.0 Hz, 1H), 4.03 (t, J = 6.5 Hz, 2H), 3.76 (s, 3H), 3.61 (s, 3H), 2.84 (t, J = 5.5 Hz, 2H), 2.69 (s, 3H), 2.28 (t, J = 5.7 Hz, 2H), 2.19 (s, 6H), 1.82 - 1.71 (m, 2H), 1.01 (t, J = 7.4 Hz, 3H) |
| EGFR_ 3365_102 | 562.7 | 9.99 (s, 1H), 8.56 - 8.48 (m, 2H), 8.32 (s, 1H), 7.63 (d, J = 8.6 Hz, 1H), 6.95 (s, 1H), 6.90 (d, J = 4.9 Hz, 1H), 6.65 (dd, J = 8.7, 2.2 Hz, 1H), 6.58 (d, J = 2.1 Hz, 1H), 6.36 (dd, J = 16.9, 10.0 Hz, 1H), 6.21 (dd, J = 17.0, 2.0 Hz, 1H), 5.77 - 5.69 (m, 1H), 3.85 (s, 3H), 3.82 (t, J = 6.3 Hz, 2H), 3.75 (s, 3H), 2.85 (t, J = 5.7 Hz, 2H), 2.69 (s, 3H), 2.29 (t, J= 5.7 Hz, 2H), 2.20 (s, 6H), 1.31 - 1.26 (m, 2H), 0.66 (t, J = 7.4 Hz, 3H) |
| EGFR_ 3365_103 | 562.7 | 10.03 (s, 1H), 8.57 - 8.55 (m, 2H), 8.35 (s, 1H), 7.58 (d, J = 8.6 Hz, 1H), 6.95 (s, 1H), 6.91 (d, J = 4.9 Hz, 1H), 6.64 (dd, J = 8.7, 2.2 Hz, 1H), 6.59 (d, J = 2.1 Hz, 1H), 6.37 (dd, J = 16.9, 10.0 Hz, 1H), 6.22 (dd, J = 16.9, 2.1 Hz, 1H), 5.74 (dd, J = 10.0, 2.0 Hz, 1H), 4.80 - 4.74 (m, 1H), 3.76 (s, 3H), 3.61 (s, 3H), 2.85 (t, J = 5.7 Hz, 2H), 2.69 (s, 3H), 2.30 (d, J = 5.5 Hz, 2H), 2.20 (s, 6H), 1.31 (d, J = 6.0 Hz, 6H) |
| EGFR_ 3365_104 | 562.7 | 10.01 (s, 1H), 8.56 (s, 1H), 8.53 (d, J = 4.9 Hz, 1H), 8.28 (s, 1H), 7.66 (d, J = 8.6 Hz, 1H), 6.95 (s, 1H), 6.85 (d, J = 4.9 Hz, 1H), 6.64 (dd, J = 8.7, 2.2 Hz, 1H), 6.57 (d, J = 2.1 Hz, 1H), 6.36 (dd, J = 16.9, 10.0 Hz, 1H), 6.21 (dd, J = 16.9, 2.0 Hz, 1H), 5.73 (dd, J = 10.0, 1.9 Hz, 1H), 4.63 - 4.57 (m, 1H), 3.85 (s, 3H), 3.76 (s, 3H), 2.85 (t, J = 5.7 Hz, 2H), 2.69 (s, 3H), 2.29 (t, J = 5.7 Hz, 2H), 2.19 (s, 6H), 0.96 (d, J = 6.0 Hz, 6H) |
| EGFR_ 3365_105 | 547.7 | 10.03 (s, 1H), 8.59 - 8.50 (m, 3H), 7.79 (d, J = 8.9 Hz, 2H), 6.99 (s, 1H), 6.95 (d, J = 4.9 Hz, 1H), 6.88 (t, J = 5.6 Hz, 1H), 6.62 (d, J = 8.9 Hz, 2H), 6.38 (dd, J = 16.9, 10.0 Hz, 1H), 6.22 (dd, J = 16.9, 1.9 Hz, 1H), 5.77 - 5.70 (m, 1H), 3.79 (s, 3H), 3.50 (t, J = 5.5 Hz, 2H), 3.32 (s, 3H), 3.29 (s, 3H), 2.86 (t, J = 5.7 Hz, 2H), 2.71 (s, 3H), 2.31 (t, J = 5.6 Hz, 2H), 2.20 (s, 6H) |

(continued)

| Code | ESI-MS $[M+H]^+$ | $^1H$ NMR (400 MHz, DMSO-$d_6$), δ |
|---|---|---|
| **EGFR_ 3365_106** | 579.7 | 10.03 (s, 1H), 8.56 - 8.54 (m, 3H), 7.78 (d, J = 8.9 Hz, 2H), 6.99 (s, 1H), 6.95 (d, J = 4.9 Hz, 1H), 6.83 (t, J = 5.5 Hz, 1H), 6.61 (d, J = 8.9 Hz, 2H), 6.38 (dd, J = 16.9, 10.1 Hz, 1H), 6.22 (dd, J = 17.0, 2.0 Hz, 1H), 5.76 - 5.72 (m, 1H), 4.77 (t, J = 5.3 Hz, 1H), 3.79 (s, 3H), 3.60 - 3.53 (m, 2H), 3.22 - 3.17 (m, 2H), 2.86 (t, J = 5.7 Hz, 2H), 2.71 (s, 3H), 2.30 (t, J = 5.7 Hz, 2H), 2.19 (s, 6H) |
| **EGFR_ 3365_106** a | 579.7 | - |
| **EGFR_ 3365_108** | 573.7 | 10.03 (s, 1H), 8.59 - 8.58 (m, 2H), 8.54 (s, 1H), 7.87 (d, J = 9.1 Hz, 2H), 7.02 - 6.97 (m, 2H), 6.90 (d, J = 9.2 Hz, 2H), 6.39 (dd, J = 16.9, 10.1 Hz, 1H), 6.22 (dd, J = 16.9, 1.9 Hz, 1H), 5.78 - 5.69 (m, 1H), 4.86 (s, 1H), 3.79 (s, 4H), 3.72 - 3.68 (m, 1H), 3.56-3.51 (m, 1H), 3.01 - 2.96 (m, 1H), 2.88 - 2.83 (m, 3H), 2.70 (s, 3H), 2.32 (t, J = 5.7 Hz, 2H), 2.20 (s, 6H), 1.92 -1.89 (m, 1H), 1.80 - 1.71 (m, 1H), 1.51 - 1.35 (m, 2H) |
| **EGFR_ 3365_109** | 573.7 | 10.03 (s, 1H), 8.59 - 8.53 (m, 3H), 7.87 (d, J = 9.1 Hz, 2H), 7.04 - 6.95 (m, 2H), 6.90 (d, J = 9.1 Hz, 2H), 6.39 (dd, J = 16.9, 10.0 Hz, 1H), 6.22 (dd, J = 16.9, 1.9 Hz, 1H), 5.76 - 5.73 (m, 1H), 4.93 (s, 1H), 3.79 (s, 4H), 3.70 (d, J = 13.0 Hz, 1H), 3.57 - 3.51 (m, 1H), 3.03 - 2.94 (m, 1H), 2.88 - 2.83 (m, 3H), 2.70 (s, 3H), 2.33 (t, J = 5.7 Hz, 2H), 2.21 (s, 6H), 1.92 - 1.88 (m, 1H), 1.78 - 1.73 (m, 1H), 1.50 - 1.37 (m, 2H) |
| **EGFR_ 3365_110** | 587.7 | 10.04 (s, 1H), 8.64 - 8.48 (m, 3H), 7.87 (d, J = 9.1 Hz, 2H), 7.00 - 6.99 (m, 2H), 6.93 (d, J = 9.0 Hz, 2H), 6.39 (dd, J = 16.8, 10.0 Hz, 1H), 6.22 (d, J = 15.3 Hz, 1H), 5.74 (d, J = 11.5 Hz, 1H), 3.79 (s, 3H), 3.76 - 3.75 (m, 1H), 3.61 - 3.55 (m, 1H), 3.29 (s, 3H+1H), 3.22 - 3.08 (m, 2H), 2.86 (t, J = 5.6 Hz, 2H), 2.70 (s, 3H), 2.31 (s, 2H), 2.20 (s, 6H), 1.99 - 1.96 (m, 1H), 1.78 - 1.71 (m, 1H), 1.55 - 1.43 (m, 2H) |
| **EGFR_ 3365_111** | 587.7 | 10.03 (s, 1H), 8.59 - 8.58 (m, 2H), 8.54 (s, 1H), 7.87 (d, J = 9.0 Hz, 2H), 7.00 - 6.99 (m, 2H), 6.93 (d, J = 9.1 Hz, 2H), 6.39 (dd, J = 16.9, 10.1 Hz, 1H), 6.22 (dd, J = 16.9, 1.9 Hz, 1H), 5.74 (d, J = 11.7 Hz, 1H), 3.79 (s, 3H), 3.76 - 3.75 (m, 1H), 3.61 - 3.56 (m, 1H), 3.29 (s, 3H), 3.21 - 3.09 (m, 3H), 2.87 (t, J = 5.7 Hz, 2H), 2.70 (s, 3H), 2.33 (t, J = 5.7 Hz, 2H), 2.21 (s, 6H), 2.00 - 1.94 (m, 1H), 1.78 - 1.71 (m, 1H), 1.56 - 1.42 (m, 2H) |
| **EGFR_ 3365_112** | 605.7 | 9.99 (s, 1H), 8.58 - 8.53 (m, 3H), 8.20 (s, 1H), 7.89 (d, J = 9.0 Hz, 2H), 7.02 - 6.95 (m, 2H), 6.55 (d, J = 9.0 Hz, 2H), 6.43 (dd, J = 16.9, 10.1 Hz, 1H), 6.22 (dd, J = 17.0, 2.0 Hz, 1H), 5.74 (dd, J = 10.1, 1.8 Hz, 1H), 4.47- 4.40 (m, 1H), 3.80 (s, 3H), 3.50 - 3.39 (m, 4H), 3.20 (d, J = 10.9 Hz, 1H), 2.91 (t, J = 5.8 Hz, 2H), 2.69 (s, 3H), 2.40 (t, J = 5.7 Hz, 2H), 2.25 (s, 6H), 2.10-2.01 (m, 1H), 1.99 - 1.88 (m, 1H) |
| **EGFR_ 3365_112 a** | 605.7 | - |
| **EGFR_ 3365_113** | 559.7 | 9.97 (s, 1H), 8.58 - 8.51 (m, 3H), 7.88 (d, J = 9.0 Hz, 2H), 7.07 - 6.91 (m, 2H), 6.55 (d, J = 9.0 Hz, 2H), 6.50 - 6.38 (m, 1H), 6.23 (dd, J = 16.9, 1.9 Hz, 1H), 5.74 (dd, J = 10.2, 1.8 Hz, 1H), 5.05 (s, 1H), 4.43 (s, 1H), 3.80 (s, 3H), 3.52-3.38 (m, 3H), 3.21 (d, J = 11.0 Hz, 1H), 2.93 (s, 2H), 2.69 (s, 3H), 2.43 (s, 2H), 2.28 (s, 6H), 2.10-2.01 (m, 1H), 1.98 - 1.89 (m, 1H) |
| **EGFR_ 3365_114** | 573.7 | 9.92 (s, 1H), 8.58 (d, J = 4.9 Hz, 1H), 8.55 (s, 1H), 8.48 (s, 1H), 7.89 (d, J = 8.9 Hz, 2H), 7.04 - 6.93 (m, 2H), 6.57 (d, J = 9.0 Hz, 2H), 6.51 (s, 1H), 6.23 (dd, J = 16.9, 1.7 Hz, 1H), 5.74 (d, J = 11.8 Hz, 1H), 4.12 (d, J = 2.5 Hz, 1H), 3.81 (s, 3H), 3.52 -3.32 (m, 6H), 3.28 (s, 3H), 2.97 (s, 2H), 2.67 (s, 3H), 2.33 (s, 6H), 2.14 - 2.05 (m, 2H) |
| **EGFR_ 3365_115** | 573.7 | 9.96 (s, 1H), 8.58 (d, J = 4.9 Hz, 1H), 8.55 (s, 1H), 8.51 (s, 1H), 7.89 (d, J = 8.9 Hz, 2H), 6.99 - 6.98 (m, 2H), 6.57 (d, J = 9.0 Hz, 2H), 6.44 (s, 1H), 6.23 (dd, J = 16.9, 1.7 Hz, 1H), 5.74 (d, J = 11.7 Hz, 1H), 4.12 (d, J = 2.4 Hz, 1H), 3.80 (s, 3H), 3.52 - 3.32 (m, 6H), 3.28 (s, 3H), 2.93 (s, 2H), 2.69 (s, 3H), 2.27 (s, 6H), 2.15 - 2.04 (m, 2H) |

(continued)

| Code | ESI-MS [M+H]+ | 1H NMR (400 MHz, DMSO-$d_6$), δ |
|---|---|---|
| **EGFR_ 3365_116** | 530.6 | 10.03 (s, 1H), 8.67 (s, 1H), 8.63 (d, J = 4.9 Hz, 1H), 8.52 (s, 1H), 8.04 (d, J = 8.9 Hz, 2H), 7.14 (d, J = 8.9 Hz, 2H), 7.08 (d, J = 4.9 Hz, 1H), 7.00 (s, 1H), 6.38 (dd, J = 16.9, 10.1 Hz, 1H), 6.21 (dd, J= 16.9, 1.9 Hz, 1H), 5.73 (dd, J = 10.1, 1.8 Hz, 1H), 3.97 - 3.93 (m, 1H), 3.79 (s, 3H), 2.86 (t, J = 5.7 Hz, 2H), 2.70 (s, 3H), 2.30 (t, J = 5.7 Hz, 2H), 2.19 (s, 6H), 0.87 - 0.82 (m, 2H), 0.71 - 0.67 (m, 2H) |
| **EGFR_ 3365_120** | 602.7 | 10.03 (s, 1H), 8.62 (s, 1H), 8.51 (d, J = 4.9 Hz, 1H), 8.26 (s, 1H), 7.53 (d, J = 8.9 Hz, 1H), 6.96 (s, 1H), 6.81 (d, J = 4.9 Hz, 1H), 6.61 (dd, J = 8.9, 2.1 Hz, 1H), 6.46 (d, J = 2.0 Hz, 1H), 6.37 (dd, J = 16.9, 10.0 Hz, 1H), 6.22 (dd, J = 17.0, 2.0 Hz, 1H), 5.74 (dd, J = 10.0, 1.9 Hz, 1H), 3.78 (s, 3H), 3.59 (s, 3H), 3.41 - 3.34 (m, 4H), 2.85 (t, J = 5.7 Hz, 2H), 2.69 (s, 3H), 2.47 - 2.40 (m, 4H), 2.29 (t, J = 5.8 Hz, 2H), 2.23 (s, 3H), 2.19 (s, 6H) |
| **EGFR_ 3365_121** | 572.7 | 10.05 (s, 1H), 8.59 - 8.54 (m, 3H), 7.88 (d, J = 9.0 Hz, 2H), 7.03 - 6.97 (m, 2H), 6.92 (d, J = 9.1 Hz, 2H), 6.38 (dd, J = 16.9, 10.1 Hz, 1H), 6.22 (dd, J = 16.9, 1.9 Hz, 1H), 5.74 (d, J = 11.8 Hz, 1H), 3.88 (d, J = 13.0 Hz, 2H), 3.79 (s, 3H), 2.99 - 2.94 (m, J = 11.1 Hz, 2H), 2.87 - 2.79 (m, 3H), 2.71 (s, 3H), 2.30 (t, J = 5.7 Hz, 2H), 2.19 (s, 6H), 1.78 - 1.75 (m, 2H), 1.28- 1.18 (m, 2H) |
| **EGFR_ 3365_121 a** | 600.8 | 10.03 (s, 1H), 8.62 - 8.56 (m, 2H), 8.53 (s, 1H), 7.89 (d, J = 9.1 Hz, 2H), 7.00 - 6.99 (m, 2H), 6.93 (d, J = 9.1 Hz, 2H), 6.39 (dd, J = 16.9, 10.1 Hz, 1H), 6.22 (dd, J = 16.9, 1.9 Hz, 1H), 5.75 (d, J = 11.8 Hz, 1H), 3.97 (d, J = 12.9 Hz, 2H), 3.79 (s, 3H), 2.93 - 2.85 (m, 4H), 2.70 (s, 3H), 2.37 - 2.28 (m, 3H), 2.19 (s, 6H), 2.20 (s, 6H), 1.82 (d, J = 11.4 Hz, 2H), 1.44-1.34 (m, 2H) |
| **EGFR_ 3365_122** | 587.7 | 10.04 (s, 1H), 8.63 (s, 1H), 8.51 (d, J = 4.9 Hz, 1H), 8.25 (s, 1H), 7.53 (d, J = 8.9 Hz, 1H), 6.96 (s, 1H), 6.80 (d, J = 4.9 Hz, 1H), 6.59 (dd, J = 9.0, 2.1 Hz, 1H), 6.45 - 6.31 (m, 2H), 6.22 (dd, J = 16.9, 2.0 Hz, 1H), 5.78-5.69 (m, 1H), 3.78 (s, 3H), 3.58 (s, 3H), 3.43 - 3.41 (m, 4H), 2.85 (t, J = 5.7 Hz, 2H), 2.69 (s, 3H), 2.29 (t, J = 5.8 Hz, 2H), 2.19 (s, 6H), 1.61 (s, 6H) |
| **EGFR_ 3365_123** | 573.7 | 10.03 (s, 1H), 8.65 (s, 1H), 8.49 (d, J = 4.9 Hz, 1H), 8.20 (s, 1H), 7.55 (d, J = 8.8 Hz, 1H), 6.96 (s, 1H), 6.76 (d, J = 4.9 Hz, 1H), 6.37 (dd, J = 16.9, 10.0Hz, 1H), 6.26 - 6.19 (m, 2H), 6.04 (d, J = 1.7 Hz, 1H), 5.73 (d, J = 11.7 Hz, 1H), 3.79 (s, 3H), 3.58 (s, 3H), 3.38 - 3.34 (m, 4H), 2.85 (t, J = 5.8 Hz, 2H), 2.69 (s, 3H), 2.28 (t, J = 5.8 Hz, 2H), 2.00 - 1.97 (m, 4H) |
| **EGFR_ 3365_124** | 564.6 | 10.02 (s, 1H), 8.59 (d, J = 4.9 Hz, 1H), 8.46 (s, 1H), 8.34 (s, 1H), 7.02 (d, J = 4.9 Hz, 1H), 6.93 (s, 1H), 6.38 (dd, J = 16.9, 10.1 Hz, 1H), 6.27 (s, 2H), 6.22 (dd, J = 17.0, 2.1 Hz, 1H), 5.78 - 5.70 (m, 1H), 3.84 (s, 3H), 3.73 (s, 3H), 3.63 (s, 6H), 2.85 (t, J = 5.7 Hz, 2H), 2.69 (s, 3H), 2.30 (t, J = 5.8 Hz, 2H), 2.20 (s, 6H) |
| **EGFR_ 3365_126** | 602.74 | 1H NMR (400 MHz, DMSO) δ 10.02 (s, 1H), 8.61 (s, 1H), 8.52 (d, *J* = 4.9 Hz, 1H), 8.31 (s, 2H), 8.25 (s, 1H), 7.54 (d, *J* = 8.9 Hz, 1H), 6.96 (s, 1H), 6.81 (d, *J* = 4.9 Hz, 1H), 6.63 (dd, *J* = 9.0, 2.1 Hz, 1H), 6.50 - 6.39 (m, 2H), 6.22 (dd, *J* = 17.0, 2.0 Hz, 1H), 5.74 (dd, *J* = 10.1, 1.9 Hz, 1H), 4.03 (d, *J* = 13.4 Hz, 2H), 3.78 (s, 3H), 3.60 (s, 3H), 3.30-3.23 (m, 1H), 2.98 (t, *J* = 12.2 Hz, 2H), 2.91 (t, *J* = 5.7 Hz, 2H), 2.67 (s, 3H), 2.41 (t, *J* = 5.8 Hz, 2H), 2.26 (s, 6H), 1.96 (d, *J* = 10.7 Hz, 2H), 1.57 - 1.49 (m, 2H) |
| **EGFR_ 3365_127** | 630.79 | 1H NMR (400 MHz, DMSO) δ 10.03 (s, 1H), 8.62 (s, 1H), 8.51 (d, *J* = 4.8 Hz, 1H), 8.25 (s, 1H), 7.53 (d, *J* = 8.8 Hz, 1H), 6.96 (s, 1H), 6.80 (d, *J* = 4.9 Hz, 1H), 6.61 (d, *J* = 8.7 Hz, 1H), 6.47 - 6.32 (m, 2H), 6.22 (d, *J* = 17.1 Hz, 1H), 5.74 (d, *J* = 10.4 Hz, 1H), 3.99 (d, *J* = 12.6 Hz, 2H), 3.78 (s, 3H), 3.59 (s, 3H), 2.96 - 2.82 (m, 4H), 2.69 (s, 3H), 2.42 -2.28 (m, 3H), 2.21 (s, 12H), 1.84 (d, *J* = 11.9 Hz, 2H), 1.50 - 1.35 (m, 2H) |

**Example 11.** Determination of chemical stability in simulated biological fluids.

1) Determination of chemical stability in simulated intestinal fluid, simulated gastric fluid and human blood plasma.

SGF concentrate without enzymes, pH=1,4 (Sigma Ireland, cat#01651) was used as simulated gastric fluid. Initial candidate solution (10 mM in DMSO) was diluted with a SGF working solution to a concentration of 10 μm (test solution). The test solution was incubated in a dry block thermostat for 2 hours at 37°C. SIF concentrate without enzymes, pH=6.5 (Sigma Ireland, cat#55331) was used as simulated intestinal fluid. Initial candidate solution (10 mM in DMSO) was diluted with a SIF working solution to a concentration of 10 μm (test solution). The test solution was incubated in a dry block thermostat for 2 hours at 37°C. HPLC with Agilent 1200 liquid chromatography system (Agilent, USA) was employed to determine peak areas of the compounds in test samples, said peak areas corresponding to the initial test time (prior to incubation) and the final test time (after incubation in a dry block thermostat for 2 hours at 37°C). We used gradient elution (1 mL/min flow rate). Substance amount in % in a sample after thermostatting was determined.

The stability of the compounds was estimated. The compounds described herein showed chemical stability values of more than 80% in simulated biological fluids (see Table 9), i.e. they are chemically stable in the acidic environment of simulated gastric fluid and weak acidic environment of simulated intestinal fluid.

**Table 9**. Results of determination of chemical stability in simulated biological fluids.

| Compound | Stability in SGF, % | Stability in SIF, % |
|---|---|---|
| **EGFR_3365** | 100,0 | 100,0 |
| **EGFR_3365_3** | 100,0 | 100,0 |
| **EGFR_3365_11** | 100,0 | 100,0 |
| **EGFR_3365_12** | 100,0 | 100,0 |
| **EGFR_3365_13** | 98,9 | 100,0 |
| **EGFR_3365_16** | 100,0 | 100,0 |
| **EGFR_3365_17** | 100,0 | 98,8 |
| **EGFR_ 3365_28** | 100,0 | 100,0 |
| **EGFR_3365_29** | 98,1 | 100,0 |
| **EGFR_3365_31** | 100,0 | 97,2 |
| **EGFR_3365_32** | 98,0 | 99,7 |
| **EGFR_ 3365_33** | 99,5 | 96,8 |
| **EGFR_ 3365_34** | 99,0 | 100,0 |
| **EGFR_3635_4** | 100,0 | 100,0 |
| **EGFR_3635_5** | 100,0 | 89,8 |
| **EGFR_3635_10** | 100,0 | 100,0 |
| **EGFR_ 3635_30** | 100,0 | 92,9 |
| **EGFR 363536** | 100,0 | 100,0 |
| **EGFR_ 3635_50** | 100,0 | 98,5 |
| **EGFR_3635_51** | 100,0 | 100,0 |
| **EGFR_ 3635_52** | 100,0 | 100,0 |
| **EGFR_ 3635_53** | 100,0 | 98,8 |
| **EGFR_ 3635_54** | 99,9 | 98,8 |
| **EGFR_ 3635_55** | 98,8 | 97,2 |
| **EGFR_ 3635_56** | 99,8 | 96,4 |
| **EGFR_3635_57** | 100,0 | 96,6 |

(continued)

| Compound | Stability in SGF, % | Stability in SIF, % |
|---|---|---|
| EGFR_ 3635_58 | 89,9 | 100,0 |
| EGFR_3635_61 | 100,0 | 98,8 |
| EGFR_ 3635_62 | 100,0 | 100,0 |
| EGFR_ 3635_63 | 100,0 | 100,0 |
| EGFR_ 3635_64 | 100,0 | 100,0 |
| EGFR_ 3635_66 | 100,0 | 96,5 |
| EGFR_ 3635_68 | 100,0 | 98,2 |
| EGFR_ 3635_69 | 100,0 | 100,0 |
| EGFR_3635_70 | 100,0 | 98,6 |
| EGFR_3635_71 | 100,0 | 100,0 |
| EGFR_3635_72 | 100,0 | 91,5 |
| EGFR_3635_73 | 100,0 | 100,0 |
| EGFR_3635_78 | 91,1 | 91,1 |
| EGFR_ 3635_85 | 100,0 | 98,4 |
| EGFR_ 3635_86 | 99,7 | 99,4 |

2) Determination of chemical stability in human blood plasma.

Determination of stability in human blood plasma was performed using pooled human blood plasma taken from ten healthy donors. The initial candidate solution (10 mM in DMSO) was diluted with pooled blood plasma to a concentration of 10 μm (test solution). The test solution was incubated in a dry block thermostat for 4 hours at 37°C. HPLC with Agilent 1200 liquid chromatography system (Agilent, USA) was employed to determine peak areas of the compounds in test samples, said peak areas corresponding to the initial test time (prior to incubation) and the final test time (after incubation in a dry block thermostat for 4 hours at 37°C), proteins were preliminarily precipitated with acetonitrile. We used gradient elution (1 mL/min flow rate). Substance amount in % in a sample after thermostatting was determined.

The stability of the compounds was estimated. The compounds described herein showed chemical stability values of more than 60% in human blood plasma (see Table 10).

**Table 10.** Results of determination of chemical stability of compounds in human blood plasma.

| Compound | Stability in blood plasma, % |
|---|---|
| EGFR_3365 | 90,3 |
| EGFR_3365_3 | 82,1 |
| EGFR_3365_11 | 85,4 |
| EGFR_3365_12 | 76,3 |
| EGFR_3365_13 | 82,4 |
| EGFR_3365_16 | 83,5 |
| EGFR_3365_17 | 97,0 |
| EGFR_ 3365_28 | 86,2 |
| EGFR_3365_29 | 79,7 |
| EGFR_3365_31 | 93,1 |
| EGFR_3365_32 | 67,5 |
| EGFR_ 3365_33 | 60,5 |

(continued)

| Compound | Stability in blood plasma, % |
|---|---|
| **EGFR_ 3365_34** | 82,7 |
| **EGFR_3635_10** | 94,2 |
| **EGFR_ 3635_36** | 98,5 |
| **EGFR_ 3635_50** | 89,0 |
| **EGFR_ 3635_53** | 91,8 |
| **EGFR_ 3635_54** | 69,9 |
| **EGFR_ 3635_55** | 84,8 |
| **EGFR_ 3635_56** | 83,2 |
| **EGFR_ 3635_58** | 99,7 |
| **EGFR_3635_61** | 91,8 |
| **EGFR_ 3635_62** | 98,4 |
| **EGFR_ 3635_63** | 100,0 |
| **EGFR_ 3635_64** | 87,2 |
| **EGFR_ 3635_66** | 97,0 |
| **EGFR_3635_67** | 92,8 |
| **EGFR_ 3635_68** | 78,4 |
| **EGFR_ 3635_69** | 100,0 |
| **EGFR_3635_70** | 90,0 |
| **EGFR_3635_71** | 100,0 |
| **EGFR_3635_72** | 94,6 |
| **EGFR_3635_73** | 90,5 |
| **EGFR_3635_78** | 81,7 |
| **EGFR_ 3635_85** | 100,0 |
| **EGFR_ 3635_86** | 100,0 |
| **EGFR_3635_87** | 73,3 |
| **EGFR_ 3635_88** | 68,2 |
| **EGFR_ 3635_90** | 98,1 |
| **EGFR_3635_91** | 95,4 |
| **EGFR_ 3635_92** | 99,1 |
| **EGFR_ 3635_93** | 96,3 |
| **EGFR_ 3635_94** | 83,3 |
| **EGFR_3635_97** | 99,3 |
| **EGFR_ 3635_98** | 96,1 |
| **EGFR_3635_101** | 99,2 |
| **EGFR_3635_102** | 82,7 |
| **EGFR_3635_103** | 96,2 |
| **EGFR_3635_104** | 77,2 |
| **EGFR_3635_105** | 89,4 |

(continued)

| Compound | Stability in blood plasma, % |
|---|---|
| **EGFR_3635_106** | 95,2 |
| **EGFR_3635_108** | 100,0 |
| **EGFR_3635_109** | 98,2 |
| **EGFR_3635_110** | 95,7 |
| **EGFR_3635_111** | 100,0 |
| **EGFR_ 3635_112** | 96,9 |
| **EGFR_ 3635_113** | 94,0 |
| **EGFR_3635_114** | 93,9 |
| **EGFR_3635_115** | 94,6 |
| **EGFR_3635_116** | 86,0 |
| **EGFR_3635_120** | 100,0 |
| **EGFR_ 3635_121** | 98,3 |
| **EGFR_ 3635_121a** | 99,0 |
| **EGFR_3635_122** | 92,2 |
| **EGFR_3635_123** | 87,3 |
| **EGFR_3635_124** | 90,7 |

Thus, as follows from example 11, the disclosed compounds of formula I show sufficient stability in the acidic environment of simulated gastric fluid, in weak acidic environment of simulated intestinal fluid and in human blood plasma.

**Example 12.** Determination of enzyme stability.

Measuring of candidate enzyme stability enabled estimation of the stability of the compounds towards the action of liver enzymes.

Enzyme degradation rate was measured by incubating the reaction mixture in a dry block thermostat at 37°C, said reaction mixture comprising 0.5 mg/mL of pooled human liver S9 fractions (XenoTech, USA, cat# H0610), 10 μM compound, 2 mM β-nicotinamide adenine dinucleotide (Carbosynth, UK, cat#NN10871) and 4 mM magnesium chloride in 0,1 M sodium-phosphate buffer, pH=7,4. The reaction was quenched with acetonitrile (100 μL of acetonitrile/100 μL of the reaction mixture). After quenching, the samples were centrifuged at 10000 rpm for 10 minutes. Supernatant fluid was tested by chromatographic technique using Agilent1200 (Agilent, USA). We used gradient elution (1 mL/min flow rate). A graph of the logarithm of substance's peak area as a function of time was made. The dependent factor of the line corresponded to the elimination rate constant K based on which the drug's half-life T1/2 and intrinsic clearance CLint were calculated:

$$\text{Elimination rate constant (k)} = (-\text{gradient})$$

$$\text{Half life } (t_{1/2})\ (\text{min}) = \frac{0.693}{k}$$

$$V\ (\mu L/mg) = \frac{\text{volume of incubation } (\mu L)}{\text{protein in the incubation (mg)}}$$

$$\text{Intrinsic Clearance } (CL_{int}) (\mu L/min/mg \text{ protein}) = \frac{V \times 0.693}{t_{1/2}}$$

Based on the data obtained, candidate enzyme stability in human liver S9 fractions was determined (see Table 11).

Table 11. Results of measurement of enzyme stability of compounds

| Compound | Stability in liver S9 fractions, Clint, μL/min/mg |
|---|---|
| **EGFR_3365** | 13,6 |
| **EGFR_3365_16** | 17,9 |
| **EGFR_3365_17** | 10,9 |
| **EGFR_3635_4** | 0,9 |
| **EGFR_3635_10** | 6,8 |
| **EGFR_3635_30** | 1,2 |
| **EGFR_3635_50** | 1,1 |
| **EGFR_3635_51** | 16,2 |
| **EGFR_3635_52** | 16,6 |
| **EGFR_3635_53** | 10,1 |
| **EGFR_3635_54** | 12,3 |
| **EGFR_3635_55** | 5,0 |
| **EGFR_3635_56** | 9,1 |
| **EGFR_3635_58** | 17,7 |
| **EGFR_3635_61** | 12,8 |
| **EGFR_3635_62** | 3,7 |
| **EGFR_3635_63** | 15,6 |
| **EGFR_3635_64** | 16,6 |
| **EGFR_3635_66** | 1,3 |
| **EGFR_3635_67** | 18,7 |
| **EGFR_3635_68** | 14,5 |
| **EGFR_3635_69** | 14,4 |
| **EGFR_3635_70** | 0,7 |
| **EGFR_3635_72** | 20,2 |
| **EGFR_3635_73** | 11,1 |
| **EGFR_3635_85** | 17,8 |
| **EGFR_ 3635_87** | 11,6 |
| **EGFR_3635_88** | 17,0 |
| **EGFR_3635_90** | 23,1 |
| **EGFR_3635_93** | 19,5 |
| **EGFR_3635_101** | 21,3 |
| **EGFR_ 3635_102** | 21,9 |

(continued)

| Compound | Stability in liver S9 fractions, Clint, μL/min/mg |
|---|---|
| EGFR_ 3635_103 | 20,4 |
| EGFR_ 3635_104 | 21,8 |
| EGFR_ 3635_105 | 11,3 |
| EGFR_ 3635_106 | 6,7 |
| EGFR_ 3635_108 | 12,8 |
| EGFR_ 3635_109 | 14,8 |
| EGFR_3635_112 | 6,8 |
| EGFR_3635_113 | 10,5 |
| EGFR_3635_114 | 16,0 |
| EGFR_3635_115 | 19,8 |
| EGFR_3635_120 | 3,8 |
| EGFR_ 3635_121 | 1,4 |
| EGFR_ 3635_121a | 2,1 |
| EGFR_ 3635_123 | 9,2 |
| EGFR_3635_124 | 22,2 |

Thus, as follows from example 11, the disclosed compounds of the formula I showed sufficient stability towards liver enzymes, with enzymatic intrinsic clearance Clint of less than 24 μl/min/mg.

**Example 13.** Measurement of permeability of compounds

1) Measurement of passive permeability through a synthetic membrane.

Passive permeability was measured using a synthetic membrane, where L-α-phosphatidylcholine membrane plays the role of a lipid bilayer. 5 μl of L-α-Phosphatidylcholine solution, from soybean, (Sigma, Ireland, cat #8002-43-5) at a concentration of 20 mg/ml in DMSO was applied to the membrane of an acceptor filter plate, 150 μl of 0.01 M sodium phosphate buffer pH = 7.4 was added to the wells of the acceptor filter plate. 300 μl of the test compound solution (10 mM in DMSO) was added to the wells of a donor plate. The assembled system was incubated for 20 hours at room temperature. Samples from the donor and acceptor plates were tested by chromatographic technique using an Agilent1200 chromatograph (Agilent, USA). We used gradient elution (1 mL/min flow rate). The peak areas of analytes in the donor and acceptor were measured on the chromatograms, and the concentration of the compound was calculated. Passive permeability through an artificial membrane *Pe* was calculated by the following formula:

$$P_e = \left\{-2,303\times\left(\frac{V_D\times V_A}{(V_D+V_A)Area\times(t_1-t_0)}\right)\times \lg\left(1-\frac{V_D+V_A}{V_D\times S}\times\frac{C_{a(t)}}{C_{d(0)}}\right)\right\},$$

**wherein** $S = \frac{V_A}{V_D}\times\frac{C_{a(t)}}{C_{d(0)}}+\frac{C_{d(t)}}{C_{d(0)}}$

$P_e$ - the effective constant of permeability, m/s

$V_D$ - the volume of donor solution (0.3 ml), ml

$V_A$ - the volume of acceptor solution (0.15 ml), ml

Area - the surface area of the membrane (0.24 $cm^2$), $cm^2$

$t_1$ - the time of incubation (72000 sec), sec

$t_0$ - the time required to fill the membrane (1140 sec), s

$C_{d(0)}$ - the concentration of the acceptor solution at the initial time, μM

$C_{d(0)}$ - the concentration of the donor solution at the initial time, μM

$C_{d(t)}$- the concentration of the acceptor solution after 20 hours, μM

$C_{d(t)}$ - the concentration of the donor solution after 20 hours, μM

The compounds showed a high rate of passive transport (see Table 12), i.e. the compounds are able to penetrate into the cell through the membrane.

**Table 12**. Results of measurement of permeability of compounds.

| Compound | $P_e$, cm/sec |
|---|---|
| **EGFR_3365** | 6,0 |
| **EGFR 3365 3** | 16,1 |
| **EGFR_3365_11** | 16,6 |
| **EGFR_3365_12** | 12,3 |
| **EGFR_3365_13** | 19,0 |
| **EGFR_3365_16** | 12,5 |
| **EGFR_3365_17** | 22,0 |
| **EGFR_ 3365_28** | 7,1 |
| **EGFR 3365 29** | 14,1 |
| **EGFR 3365 31** | 14,9 |
| **EGFR 3365 32** | 11,1 |
| **EGFR_ 3365_34** | 17,2 |
| **EGFR_3635_4** | 12,8 |
| **EGFR_3635_10** | 21,1 |
| **EGFR_ 3635_30** | 14,9 |
| **EGFR_ 3635_50** | 7,2 |
| **EGFR_3635_51** | 10,3 |
| **EGFR_ 3635_52** | 17,0 |
| **EGFR_ 3635_53** | 4,8 |
| **EGFR_ 3635_54** | 10,2 |
| **EGFR_ 3635_56** | 7,8 |
| **EGFR_3635_57** | 21,1 |
| **EGFR_ 3635_58** | 3,9 |
| **EGFR_ 3635_63** | 5,6 |
| **EGFR_ 3635_66** | 6,1 |
| **EGFR_3635_67** | 11,8 |

(continued)

| Compound | $P_e$, *cm/sec* |
|---|---|
| **EGFR_ 3635_68** | 9,5 |
| **EGFR_3635_70** | 11,7 |
| **EGFR_3635_71** | 13,0 |
| **EGFR_3635_78** | 2,9 |

2) Assessment of permeability through monolayer of Caco-2 cells.

Assessment of permeability through the monolayer of Caco-2 cells allows to evaluate the ability of the candidate compounds to penetrate through biological membranes by active and passive transport.

Caco-2, the cells of the intestinal epithelium, had been cultured in transwell plate inserts with the filters (with pores of 0.4 μm, BD Falcon with High Density) for 21 days, and then the integrity of the monolayer were estimated with Lucifer Yellow (Sigma-Aldrich, USA) by standard protocol. When setting the A→B transfer ("intestinal lumen" - "blood stream" transfer), solutions of test substances were added in a buffer with pH 6.5 (HBSS, 10 mM HEPES, 15 mM glucose solution) with the concentration of 10 μM into the upper chamber; the lower chamber was filled with a buffer with pH 7.4 (HBSS, 10 mM HEPES, 15 mM Glucose, 1% BSA). When setting B→A transfer ("blood stream" - "intestinal lumen" transfer), the upper chamber was filled with the buffer with pH 6.5, and solutions of the test substances were added in the buffer with pH 7.4 at the concentration of 10 μM in the lower chamber. Propranolol was used as a control substance (as it has high permeability).

After incubating for 2 h at 37°C under 5% $CO_2$, the amounts of test substances were determined in the upper and lower chambers by HPLC using Agilent1200 chromatograph (Agilent, USA) with preliminary protein precipitation with acetonitrile. We used gradient elution (1 mL/min flow rate). We determined the areas of peaks corresponding to the compounds. On the basis of peak areas in the calibration standards we determined the concentration of compound in the initial solution and in the samples from the wells of the upper and lower chambers.
*Papp,* permeability through the cell layer, was calculated using the following formula:

$$\boldsymbol{P_{app}} = (\boldsymbol{C_{(t)}} * \mathrm{V}) / (\boldsymbol{C_{(0)}} * \mathrm{t} * \mathrm{Area}),$$

wherein

$\boldsymbol{P_{app}}$ - the effective constant of permeability, m/s
V - the volume of solution (0.8 ml in A→B test, 0.2 ml in B→A test), ml
Area - the surface area of the membrane (0.33 $cm^2$), $cm^2$
**t** - the time of incubation (7200 sec), sec
$\boldsymbol{C_{(0)}}$ - the concentration of the initial solution, μM
$\boldsymbol{C_{(t)}}$- the concentration of the solution after 2 hours (the concentration in the sample from the well of the lower chamber in A→B test; the concentration in the sample from the well of the top chamber in in B→A test), μM

The efflux coefficient shows the ability of cells to eliminate the substance from the bloodstream. The value was calculated with the following formula:

$$\boldsymbol{efflux} = \boldsymbol{P_{app}}_{\ \mathrm{B-A}} / \boldsymbol{P_{app}}_{\ \mathrm{A-B}},$$

wherein

$\boldsymbol{P_{app}}_{\mathrm{A-B}}$ - the volume of permeability in A - > B test;
$\boldsymbol{P_{app}}_{\mathrm{B-A}}$ - he volume of permeability in B - > A *test.*

The compounds show a high rate of direct "intestinal lumen" - "blood stream" transport, while the efflux coefficient does not exceed 2 (see Table 13), which indicates that the Pgp transporter does not impose any restrictions on bioavailability of the test compounds.

**Table 13.** Results of assessment of permeability through the monolayer of Caco-2 cells.

| Compound | A - B, Papp $_{M/C}$ | efflux |
|---|---|---|
| **EGFR_3365** | 5,28 | 1,29 |
| **EGFR_3365_3** | 5,90 | 0,80 |
| **EGFR_3365_12** | 1,94 | 0,98 |
| **EGFR_3365_28** | 3,84 | 1,87 |
| **EGFR_3365_29** | 4,26 | 1,50 |
| **EGFR_3365_31** | 3,70 | 1,10 |
| **EGFR_3635_4** | 2,86 | 2,00 |
| **EGFR_3635_10** | 2,88 | 0,58 |
| **EGFR_3635_30** | 4,36 | 0,50 |
| **EGFR_3635_50** | 2,00 | 0,30 |
| **EGFR_3635_51** | 3,20 | 1,70 |
| **EGFR_3635_52** | 3,00 | 1,20 |
| **EGFR_3635_54** | 1,80 | 1,00 |
| **EGFR_3635_56** | 1,80 | 1,70 |
| **EGFR_3635_58** | 5,80 | 1,10 |
| **EGFR_3635_61** | 5,50 | 1,80 |
| **EGFR_3635_63** | 4,90 | 1,00 |
| **EGFR_3635_66** | 1,30 | 0,40 |
| **EGFR_3635_70** | 2,50 | 0,40 |
| **EGFR_3635_72** | 3,50 | 1,50 |
| **EGFR_3635_73** | 5,10 | 1,60 |
| **EGFR_3635_90** | 2,20 | 1,49 |
| **EGFR_3635_94** | 6,54 | 0,86 |
| **EGFR_ 3635_102** | 27,52 | 1,62 |
| **EGFR_ 3635_121** | 1,87 | 0,87 |

Thus, as follows from Example 13, the disclosed compounds of formula I show quite a high rate of passive and active transport, which means that the compounds disclosed herein have potentially good bioavailability.

**Example 14.** In vitro inhibitory activity against EGFR.

To assess $IC_{50}$ values of the compounds disclosed herein, a biochemical assay of inhibition of kinase activity in a non-cellular system and a functional cellular antiproliferation assay were used.

To assess inhibition of WT EGFR (Wild-Type EGFR), DM EGFR (DoubleMutantEGFR, L828R/T790M) kinase activity, SignalChem kinase system and ADP-Glo™ Kinase Assay (V9102, Promega) detection kit were used.

The measurements were carried out in a 25 μL reaction volume using a 96-well plate. The kinase enzyme and inhibitor were pre-incubated for 10 minutes in the reaction buffer containing 25 mM MOPS (pH 7.2), 12.5 mM β-glycerophosphate, 27 mM MgCl2, 2 mM MnCl2, 5 mM EGTA, 2 mM EDTA, 0.3 mM DTT, and 1.2 mg/mL of bovine serum albumin. Staurosporine (S4400, Sigma) was used as a reference inhibitor and 0.1% DMSO in the reaction buffer - as a negative control. The solution of 0.5 mg/mL peptide substrate and 50 μM ATP in the same buffer were added; the solution was incubated for 180 minutes at 37 °C. To detect the amount of ATP taken up during the kinase reaction, the ADP Glo Detection Kit (V9102, Promega) was used. The luminescence was measured with the Infinite M200Pro plate

spectrophotometer (Tecan, Switzerland). $IC_{50}$ values were calculated using Magellan 7.2 software (Tecan, Switzerland) approximating experimental points by four-parameter logistic model with the optimization by Levenberg-Marquardt (Table 14).

Antiproliferative activity of EGFR inhibitors (Tables 15 and 16) was measured in cell-based bioassay on continuous epithelial cell cultures: A549 (lung adenocarcinoma, ATCC® CRM-CCL-185™ - WT), HCC827 (lung adenocarcinoma, ATCC® CRL-2868™ - SM (SingleMutantEGFR, exon19delE746-A750)) and H1975 (lung adenocarcinoma, ATCC® CRL-5908™ - DM (DoubleMutantEGFR, L828R/T790M)) using cell viability reagent Alamar Blue (ThermoFisher, #DAL1100). Cells were cultured in 10% FBS-supplemented (Gibco, #16140071) RPMI-1640 (PanEco, S330p) for at least 1 passage after thawing, washed with PBS and passaged in 96-well culture plates (3599, Corning) with RPMI-1640 growth medium supplemented with 2% FBS in an amount of $5*10^3$ cells/well for A549, $10*10^3$ cells/well for HCC827 and $15*10^3$ cells/well for H1975 in 100 μL, and incubated at 37°C in an incubator (Thermo Forma, USA) under 5% of $CO_2$ for 16-18 h for cell attachment.

The test compounds were dissolved in DMSO in the selected range of concentrations and transferred to RPMI-1640 medium (PanEco, S330p) supplemented with 2% FBS. After adding 50 μl of the prepared dilutions to the cells, the incubation mixture contained the final concentrations of the test substances and no more than 1% DMSO. The plates were Incubated at 37°C for 72 h. After incubation, 15 μl of Alamar Blue vital dye (ThermoFisher, #DAL1100) was added to each well. The plates were shaken on an orbital shaker (Biosan, Latvia) and further incubated for 3-5 h in a $CO_2$ incubator (Thermo Forma, USA). Number of living cells was estimated using a plate spectrophotometer Infinite M200Pro, (Tecan, Switzerland) measuring fluorescent signal at the excitation wavelength (λEx) of 540 nm and emission wavelength (λEm) of 590 nm.

$IC_{50}$ was calculated using Magellan 7.2 software (Tecan, Switzerland) approximating experimental points by four-parameter logistic model with the optimization by Levenberg-Marquardt.

The $CC_{50}$ values were determined in the test for General cytotoxicity on HepG2 cells (rhepatocellular carcinoma, ATCC® HB-8065™) (Table 16). The cells were subcultured in DMEM medium (PanEco, S420p) supplemented with 10% FBS (Gibco, #16140071) for at least 1 passage after thawing, washed with PBS and passaged in 96-well culture plates (3599, Corning) at a concentration of $2*10^4$ cells/100 μL per well, and incubated for 16-18 h. The test substances were titrated in DMSO and transferred to DMEM (PanEco, S420p) medium supplemented with 2% FBS, added to the cells and incubated at 37°C for 72 hours; viability of the cells was then assessed using Alamar Blue dye (ThermoFisher, #DAL1100). $CC_{50}$ was determined similarly to $IC_{50}$.

The ratio between $CC_{50}$ for HepG2 cell line and $IC_{50}$ for H1975 cell line is the therapeutic index (TI):

$$\text{Therapeutic index} = \frac{\mathrm{CC}_{50}(HepG2)}{\mathrm{IC}_{50}(H1975)}$$

The ratio between $IC_{50}$ for cell lines containing wild-type EGFR (A549) and $IC_{50}$ for cell lines containing target mutation L828R/T790M (H1975) is the selectivity index (SI):

$$\text{Selectivity index} = \frac{\mathrm{IC}_{50}(A549)}{\mathrm{IC}_{50}(H1975)}$$

The compounds of the present invention demonstrated effective inhibition of kinase activity with a target EGFR mutation (L828R/T790M), and also demonstrated low activity against wild-type EGFR. The compounds of the present invention showed high selectivity for mutant EGFR.

The compounds of the present invention showed high antiproliferation activity against target cell lines (EGFR with a mutation L828R/T790M and with exon 19 deletion) in cell-based assays.

The compounds of the present invention showed low toxicity in the test for general cytotoxicity.

**Table 14.** Results of inhibition of kinase activity.

| **Compound** | **$IC_{50}$, HM** | | **SI,** |
|---|---|---|---|
| | **EGFR DM** | **EGFR WT** | **$IC_{50}$(WT)/$IC_{50}$(DM)** |
| **EGFR_3365** | 22,01 | 587,37 | 26,69 |
| **EGFR_ 3365-3** | 14,47 | 76,59 | 5,29 |

(continued)

| Compound | $IC_{50}$, HM | | SI, |
|---|---|---|---|
| | EGFR DM | EGFR WT | $IC_{50}(WT)/IC_{50}(DM)$ |
| EGFR_ 3365-11 | 6,44 | 170,25 | 26,43 |
| EGFR_ 3365-12 | 25,53 | 389,33 | 15,25 |
| EGFR_ 3365-13 | 18,15 | 112,87 | 6,22 |
| EGFR_ 3365-16 | 32,78 | 153,93 | 4,70 |
| EGFR_ 3365-17 | 21,54 | 171,31 | 7,95 |
| EGFR_ 3365-28 | 6,27 | 127,52 | 20,33 |
| EGFR_ 3365-29 | 10,73 | 201,58 | 18,79 |
| EGFR_ 3365-31 | 8,65 | 130,77 | 15,12 |
| EGFR_ 3365-32 | 5,26 | 202,09 | 38,41 |
| EGFR_ 3365-33 | 6,79 | 75,78 | 11,16 |
| EGFR_ 3365-34 | 6,95 | 514,46 | 74,07 |
| EGFR_ 3365-4 | 21,35 | 230,43 | 10,79 |
| EGFR_ 3365-5 | 22,04 | 463,61 | 21,03 |
| EGFR_ 3365-10 | 7,28 | 211,48 | 29,06 |
| EGFR_ 3365-30 | 7,69 | 613,49 | 79,77 |
| EGFR_ 3365-36 | 18,90 | 360,00 | 19,05 |
| EGFR_ 3365-50 | 3,29 | 32,00 | 9,74 |
| EGFR_ 3365-66 | 1,86 | 46,82 | 25,13 |
| EGFR_ 3365-70 | 14,74 | 250,00 | 16,96 |
| EGFR_ 3365-77 | 4,97 | 49,88 | 10,04 |
| EGFR_ 3365-51 | 4,72 | 50,78 | 10,75 |
| EGFR_ 3365-52 | 6,41 | 67,87 | 10,58 |
| EGFR_ 3365-54 | 5,53 | 144,98 | 26,20 |
| EGFR_ 3365-55 | 25,39 | 76,63 | 3,02 |
| EGFR_ 3365-56 | 6,30 | 120,57 | 19,14 |
| EGFR_ 3365-63 | 0,94 | 22,86 | 24,32 |
| EGFR_ 3365-85 | 8,36 | 59,96 | 7,17 |
| EGFR_ 3365-57 | 6,24 | 49,69 | 7,96 |
| EGFR_ 3365-58 | 2,36 | 39,69 | 16,81 |
| EGFR_ 3365-62 | 6,58 | 127,37 | 19,37 |
| EGFR_ 3365-67 | 10,52 | 97,51 | 9,27 |
| EGFR_ 3365-68 | 9,03 | 93,90 | 10,40 |
| EGFR_ 3365-69 | 2,59 | 23,63 | 9,12 |
| EGFR_ 3365-73 | 2,35 | 32,68 | 13,89 |
| EGFR_ 3365-53 | 7,31 | 183,11 | 25,06 |
| EGFR_ 3365-61 | 2,76 | 53,09 | 19,27 |
| EGFR_ 3365-72 | 3,71 | 55,92 | 15,09 |

(continued)

| Compound | IC$_{50}$, HM | | SI, |
|---|---|---|---|
| | EGFR DM | EGFR WT | IC$_{50}$(WT)/IC$_{50}$(DM) |
| EGFR_ 3365-87 | 2,75 | 67,36 | 24,50 |
| EGFR_ 3365-88 | 5,09 | 78,19 | 15,37 |
| EGFR_ 3365-90 | 0,75 | 32,33 | 43,40 |
| EGFR_ 3365-91 | 4,55 | 107,46 | 23,60 |
| EGFR_ 3365-92 | 7,57 | 132,95 | 17,56 |
| EGFR_ 3365-93 | < 1 | 41,48 | >41,48 |
| EGFR_ 3365-115 | 1,99 | 126,32 | 63,44 |
| EGFR_ 3365-94 | 1,41 | 25,43 | 18,02 |
| EGFR_ 3365-110 | 2,32 | 97,74 | 42,20 |
| EGFR_ 3365-105 | 2,93 | 108,92 | 37,17 |
| EGFR_ 3365-114 | 2,02 | 51,35 | 25,37 |
| EGFR_ 3365-106 | 5,50 | 108,22 | 19,68 |
| EGFR_ 3365-97 | 2,30 | 84,18 | 36,55 |
| EGFR_ 3365-98 | 1,86 | 83,21 | 44,62 |
| EGFR_ 3365-103 | 2,14 | 22,64 | 10,60 |
| EGFR_ 3365-108 | 1,71 | 25,41 | 14,85 |
| EGFR_ 3365-109 | 3,14 | 25,23 | 8,03 |
| EGFR_ 3365-111 | 2,33 | 185,82 | 79,88 |
| EGFR_ 3365-112 | 2,05 | 23,55 | 11,48 |
| EGFR_ 3365-116 | 1,75 | 34,99 | 19,99 |
| EGFR_ 3365-124 | 3,57 | 76,35 | 21,41 |
| EGFR_ 3365-121 | 0,89 | 21,62 | 24,30 |
| EGFR_ 3365-121a | 0,96 | 29,59 | 30,92 |
| EGFR_ 3365-101 | 2,54 | 29,32 | 11,55 |
| EGFR_ 3365-102 | 11,17 | 205,58 | 18,40 |
| EGFR_ 3365-120 | 2,37 | 30,88 | 13,01 |
| EGFR_ 3365-122 | 2,67 | 19,66 | 7,35 |
| EGFR_ 3365-123 | 2,65 | 51,98 | 19,58 |
| EGFR_ 3365-113 | 2,54 | 43,39 | 17,09 |
| EGFR_ 3365-104 | 6,42 | 113,09 | 17,61 |
| EGFR_ 3365-126 | 1,86 | 19,75 | 10,62 |
| EGFR_ 3365-127 | 2,96 | 24,54 | 8,29 |

* IC$_{50}$ values after ">" and "<" are out of the range of concentrations considered.
**After "<" and ">" given are approximate values calculated based on an IC$_{50}$ value that is out of the range of concentrations considered.

**Table 15.** Results of assessment of specific activity of compounds in cell-based antiproliferative test using cell lines: H1975 (DM), HCC827 (SM).

| Compound | $IC_{50}$, HM | | $IC_{50}$, HM | SI |
|---|---|---|---|---|
| | H1975 (DM) | HCC827 (SM) | A549 (WT) | $IC_{50}$(A549)/ $IC_{50}$ (DM) |
| EGFR_3365 | 60,1 | 14,3 | >10000* | >166** |
| EGFR_3365_3 | 44,5 | 2,3 | 6069 | 136,32 |
| EGFR_3365_11 | 351,2 | 6,2 | 12420 | 35,37 |
| EGFR_3365_12 | 21,5 | 20,3 | 15419 | 716,20 |
| EGFR_3365_13 | 30,8 | 10,3 | 4481 | 145,63 |
| EGFR_3365_16 | 53,7 | 10,0 | 2206 | 41,10 |
| EGFR_3365_17 | 393,5 | 41,3 | 2391 | 6,08 |
| EGFR_3365_28 | 17,1 | 15,3 | 3639 | 213,04 |
| EGFR_3365_29 | 26,9 | 6,2 | 16022 | 595,06 |
| EGFR_3365_31 | 7,3 | 8,3 | 11078 | 1509,19 |
| EGFR_3365_32 | 93,4 | 11,1 | 35133 | 376,12 |
| EGFR_3365_33 | 5,6 | 5,9 | >10000* | >1786** |
| EGFR_3365_34 | 164,5 | 111,2 | 16162 | 98,26 |
| EGFR_3365_4 | 35,3 | 15,3 | 5378 | 152,17 |
| EGFR_3365_5 | 57,3 | 28,6 | 7590 | 132,56 |
| EGFR_3365_10 | 211,3 | 5,9 | 13247 | 62,70 |
| EGFR_3365_30 | 16,4 | 2,0 | 5545 | 337,72 |
| EGFR 3365 36 | 183,7 | - | 73924 | 402,37 |
| EGFR_3365_50 | 47,51 | 26,85 | 4922 | 104 |
| EGFR_3365_66 | 34,73 | 12,80 | 4640 | 134 |
| EGFR_3365_70 | 203,08 | 36,79 | 4390 | 22 |
| EGFR_ 3365_77 | 249,28 | 23,51 | 2167 | 9 |
| EGFR_3365_51 | 33,78 | 8,60 | 11488 | 340 |
| EGFR_3365_52 | 66,08 | 22,54 | 10824 | 164 |
| EGFR_3365_54 | 34,22 | 13,87 | 4424 | 129 |
| EGFR_3365_55 | 211,30 | 20,07 | 11636 | 55 |
| EGFR_3365_56 | 29,22 | 29,89 | 4544 | 156 |
| EGFR_3365_63 | 5,79 | 4,27 | 10816 | 5383 |
| EGFR_3365_85 | 138,17 | 18,02 | 1410 | 10 |
| EGFR_3365_57 | 69,39 | 5,64 | 8638 | 124 |
| EGFR_3365_58 | 10,47 | 6,23 | >10000 | >1235 |
| EGFR 3365 62 | 60,79 | 12,10 | 12268 | 539 |
| EGFR_3365_67 | 220,67 | 26,27 | 2471 | 11 |
| EGFR_3365_68 | 34,61 | 19,05 | 8141 | 235 |
| EGFR_3365_69 | 7,93 | 2,46 | >10000 | >1261 |

(continued)

| Compound | IC$_{50}$, HM | | IC$_{50}$, HM | SI |
|---|---|---|---|---|
| | H1975 (DM) | HCC827 (SM) | A549 (WT) | IC$_{50}$(A549)/ IC$_{50}$ (DM) |
| **EGFR_3365_73** | 10,28 | 4,47 | 16014 | 1678 |
| **EGFR_3365_53** | 44,94 | 26,55 | 8235 | 183 |
| **EGFR_3365_61** | 8,12 | 5,77 | 23418 | 2884 |
| **EGFR_3365_72** | 11,52 | 13,52 | 10732 | 932 |
| **EGFR_ 3365_87** | 57,59 | - | 8020 | 139 |
| **EGFR_3365_88** | 62,64 | - | 14877 | 238 |
| **EGFR_3365_90** | 7,58 | 4,39 | 5305 | 1060 |
| **EGFR_3365_91** | >200 | - | 31571 | <158 |
| **EGFR_3365_92** | 35,06 | - | 22853 | 652 |
| **EGFR_3365_93** | 103,68 | - | 9152 | 88 |
| **EGFR_3365_115** | 19,11 | - | 12810 | 670 |
| **EGFR_3365_94** | 6,65 | - | 6895 | 1037 |
| **EGFR_3365_110** | 21,66 | - | 9514 | 439 |
| **EGFR_ 3365_105** | 21,57 | - | 84086 | 3898 |
| **EGFR_3365_114** | 12,09 | - | 14208 | 1175 |
| **EGFR_ 3365_106** | 33,31 | - | >20000 | >601 |
| **EGFR_ 3365_97** | 22,95 | - | 13798 | 601 |
| **EGFR_3365_98** | 13,65 | - | 14542 | 1065 |
| **EGFR_ 3365_103** | 13,17 | - | 12569 | 954 |
| **EGFR_ 3365_108** | 4,35 | - | 21410 | 4925 |
| **EGFR_ 3365_109** | 8,66 | - | 17705 | 2043 |
| **EGFR_3365_111** | 36,59 | - | 11241 | 307 |
| **EGFR_ 3365_112** | 15,26 | - | 37286 | 2443 |
| **EGFR_3365_116** | 29,47 | - | 11795 | 400 |
| **EGFR_ 3365_124** | 66,31 | - | 19726 | 298 |
| **EGFR_ 3365_121** | 4,21 | 15,19 | 8354 | 1983 |
| **EGFR_3365_121a** | 2,55 | 13,52 | 3268 | 1284 |
| **EGFR_3365_101** | 12,90 | - | 8324 | 645 |
| **EGFR_ 3365_102** | 80,48 | - | 5569 | 69 |
| **EGFR_ 3365_120** | 4,50 | 5,19 | 16395 | 3644 |
| **EGFR_ 3365_122** | 2,21 | - | 5307 | 2398 |
| **EGFR_ 3365_123** | 1,18 | - | 7194 | 6105 |
| **EGFR_ 3365_113** | 4,42 | - | 13589 | 3073 |
| **EGFR_ 3365_104** | 257,58 | - | 3780 | 15 |

(continued)

| Compound | $IC_{50}$, HM | | $IC_{50}$, HM | SI |
|---|---|---|---|---|
| | H1975 (DM) | HCC827 (SM) | A549 (WT) | $IC_{50}$(A549)/ $IC_{50}$ (DM) |
| **EGFR_3365_127** | 8,45 | - | >10000* | >166** |

* $IC_{50}$ values after ">" and "<" are out of the range of concentrations considered.
** After "<" and ">" given are approximate values calculated based on an $IC_{50}$ value that is out of the range of concentrations considered.

**Table 16.** Results of assessment of specific activity and general toxicity of compounds using H1975 and HepG2 cell lines. The results are presented as average values of activity obtained in several tests.

| Compound | H1975 (DM) $IC_{50}$, HM | HepG2 $CC_{50}$, HM | TI, $CC_{50}/IC_{50}$ |
|---|---|---|---|
| **EGFR_3365** | 60,12 | 25408 | 423 |
| **EGFR 3365 3** | 44,52 | 15164 | 341 |
| **EGFR_3365_11** | 351,18 | 15046 | 43 |
| **EGFR_3365_12** | 21,53 | 22618 | 1051 |
| **EGFR_3365_13** | 30,77 | 14148 | 460 |
| **EGFR_3365_16** | 53,68 | 5789 | 108 |
| **EGFR_3365_17** | 393,47 | 6319 | 16 |
| **EGFR_ 3365_28** | 17,08 | 31361 | 1836 |
| **EGFR_3365_29** | 26,92 | 30564 | 1135 |
| **EGFR_3365_31** | 7,34 | 9494 | 1293 |
| **EGFR_3365_32** | 93,41 | 24783 | 265 |
| **EGFR_ 3365_33** | 5,60 | 57591 | 10285 |
| **EGFR_ 3365_34** | 164,49 | 10273 | 62 |
| **EGFR 3365 4** | 35,34 | 14809 | 419 |
| **EGFR 3365 5** | 57,26 | 17704 | 309 |
| **EGFR_3365_10** | 211,26 | 30388 | 144 |
| **EGFR_ 3365_30** | 16,42 | 10942 | 666 |
| **EGFR_ 3365_36** | 183,72 | 36549 | 199 |
| **EGFR_ 3365_50** | 47,5 | 12432 | 262 |
| **EGFR_ 3365_66** | 34,7 | 9640 | 278 |
| **EGFR_3365_70** | 203,1 | 11470 | 56 |
| **EGFR_3365_77** | 249,3 | 8342 | 33 |
| **EGFR_3365_51** | 33,8 | 20941 | 620 |
| **EGFR_ 3365_52** | 66,1 | 15635 | 237 |
| **EGFR_ 3365_54** | 34,2 | 7823 | 229 |
| **EGFR_ 3365_55** | 211,3 | 13298 | 63 |
| **EGFR_ 3365_56** | 29,2 | 9512 | 325 |
| **EGFR_ 3365_63** | 2,0 | 12968 | 2239 |
| **EGFR_ 3365_85** | 138,2 | 21168 | 153 |

(continued)

| Compound | H1975 (DM) $IC_{50}$, HM | HepG2 $CC_{50}$, HM | TI, $CC_{50}/IC_{50}$ |
|---|---|---|---|
| EGFR_3365_57 | 69,4 | 22787 | 328 |
| EGFR_ 3365_58_ | 8,1 | 44405 | 4241 |
| EGFR_ 3365_62 | 22,8 | 31149 | 512 |
| EGFR_3365_67 | 220,7 | 9456 | 43 |
| EGFR_ 3365_68 | 34,6 | 28560 | 825 |
| EGFR_ 3365_69 | 7,9 | 51929 | 6549 |
| EGFR_3365_73 | 9,5 | 35637 | 3465 |
| EGFR_ 3365_53 | 44,9 | 20559 | 457 |
| EGFR_3365_61 | 8,1 | 43010 | 5296 |
| EGFR_3365_72 | 11,5 | 16225 | 1408 |
| EGFR_3365_87 | 57,6 | 19469 | 338 |
| EGFR_ 3365_88 | 62,6 | 26857 | 429 |
| EGFR_ 3365_90 | 5,0 | 12804 | 1689 |
| EGFR_ 3365_93 | 103,7 | 23557 | 227 |
| EGFR_3365_115 | 19,11 | 22048 | 1154 |
| EGFR_ 3365_94 | 6,65 | 11974 | 1801 |
| EGFR_3365_110 | 21,66 | 18531 | 855 |
| EGFR_3365_105 | 21,57 | 45604 | 2114 |
| EGFR_3365_114 | 12,09 | 23812 | 1970 |
| EGFR_3365_106 | 33,31 | >50000* | >1501** |
| EGFR_3365_97 | 22,95 | 21632 | 943 |
| EGFR_ 3365_98 | 13,65 | 24158 | 1770 |
| EGFR_3365_103 | 13,17 | 29392 | 2232 |
| EGFR_3365_108 | 4,35 | 47934 | 11026 |
| EGFR_3365_109 | 8,66 | 45448 | 5245 |
| EGFR_3365_111 | 36,59 | 27143 | 742 |
| EGFR 3365_112 | 15,26 | >50000 | >3267 |
| EGFR_3365_116 | 29,47 | 22079 | 749 |
| EGFR 3365 124 | 66,31 | >50000 | >754 |
| EGFR 3365_121 | 4,21 | 75129 | 17832 |
| EGFR_3365_121a | 2,55 | 11832 | 4649 |
| EGFR_3365_101 | 12,90 | 15975 | 1238 |
| EGFR_3365_102 | 80,48 | 27519 | 342 |
| EGFR_3365_120 | 4,50 | 29309 | 6514 |
| EGFR_3365_122 | 2,21 | 12638 | 5710 |
| EGFR_3365_123 | 1,18 | 40021 | 33959 |
| EGFR_ 3365_113 | 4,42 | 46343 | 10481 |

(continued)

| Compound | H1975 (DM) $IC_{50}$, HM | HepG2 $CC_{50}$, HM | TI, $CC_{50}/IC_{50}$ |
|---|---|---|---|
| **EGFR_3365_104** | 257,58 | 18722 | 73 |
| * $IC_{50}$ values after ">" and "<" are out of the range of concentrations considered.<br>** After "<" and ">" given are approximate values calculated based on an $IC_{50}$ value that is out of the range of concentrations considered. | | | |

**Claims**

**1.** A compound of formula I:

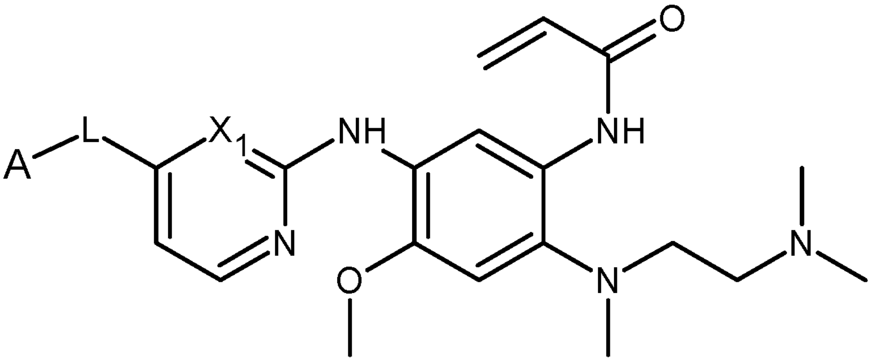

I,

or pharmaceutically acceptable salt, solvate or stereoisomer thereof,

wherein L is -C(O)- or -CHOH-;
$X_1$ is CH or N;
A is

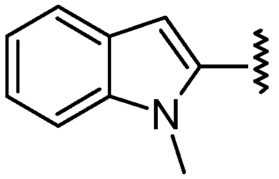

or

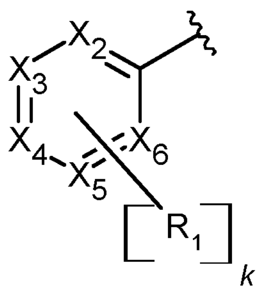

,

wherein
each $X_2$, $X_3$, $X_4$, $X_5$, $X_6$ is independently C, CH or N,
each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;

each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
k is 0, 1, 2 or 3;
Hal is fluoro, bromo, chloro or iodo.

**2.** The compound according to claim 1, wherein the fragment

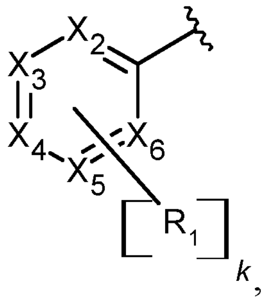

is selected from group, comprising:

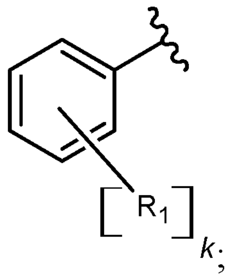
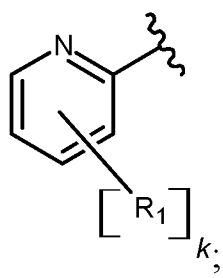
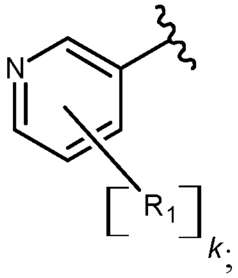
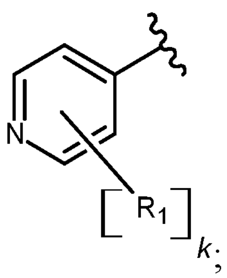
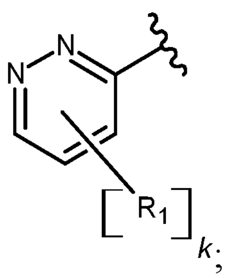
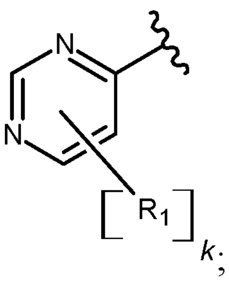
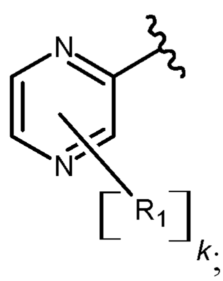
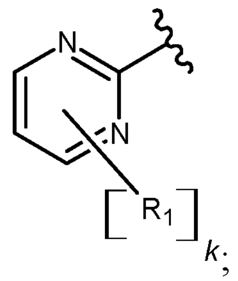
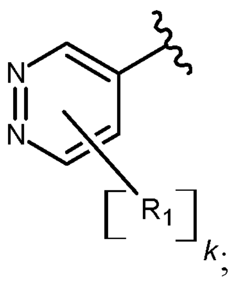
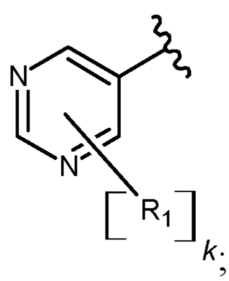
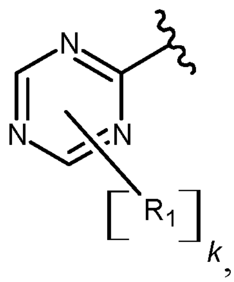

each $R_1$ is independently hydrogen; Hal; cyano; nitro; hydroxy group; $C_1$-$C_6$ alkyl, unsubstituted or substituted by one or several radicals selected from Hal, hydroxy group, $-NR_2R_3$; $C_1$-$C_6$ alkoxy group, unsubstituted or substituted by one or several radicals selected from Hal, $-NR_2R_3$, hydroxy group, $C_1$-$C_6$ alkyloxy, aryl, unsubstituted or substituted by one or several radicals selected from Hal, hydroxy group, $-NR_2R_3$; aryloxy, unsubstituted or substituted by one or several radicals selected from Hal, $C_1$-$C_6$ alkyl, hydroxy group, $-NR_2R_3$; $C_3$-$C_6$ cycloalkyloxy, unsubstituted or substituted by one or several radicals selected from Hal, hydroxy group, $-NR_2R_3$; $C_1$-$C_6$ alkyloxy $C_1$-$C_6$ alkyl; $-NR_2R_3$; aryl, unsubstituted or substituted by one or several radicals selected from Hal, hydroxy group, $-NR_2R_3$; 5-6 membered heteroaryl with 1-2 heteroatoms, selected from N, O and/or S,

unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
k is 0, 1, 2 or 3;
Hal is fluoro, bromo, chloro or iodo.

**3.** The compound according to claim 1, wherein the fragment

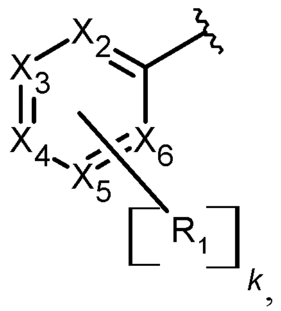

is selected from group, comprising:

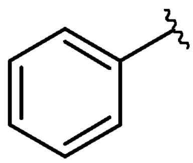 ; 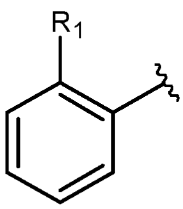 ; 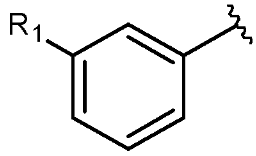 ; 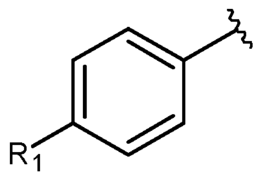 ; 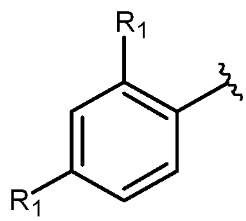 ;

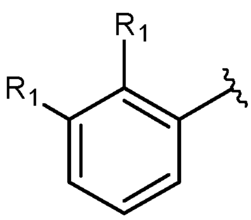 ; 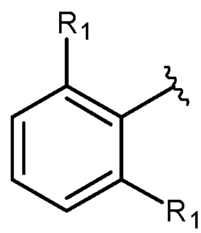 ; 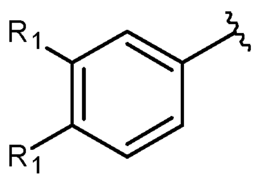 ; 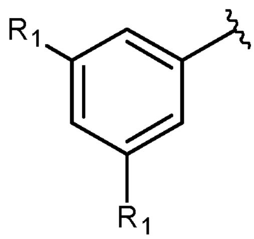 ;

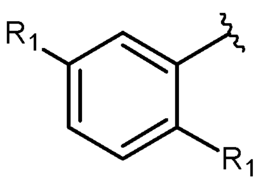 ; 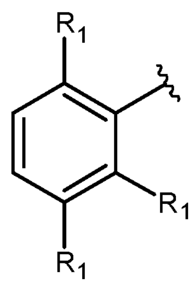 ; 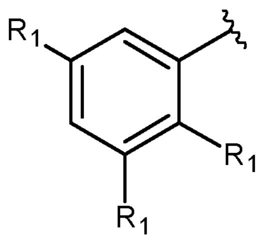 ; 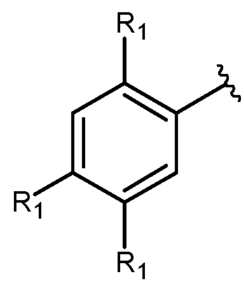 ;

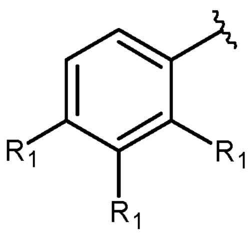 ; 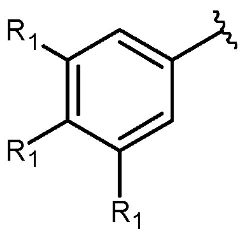 ; 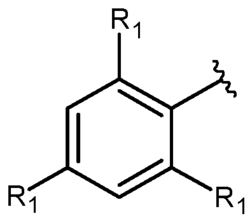 ; 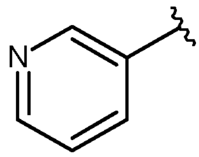 ; 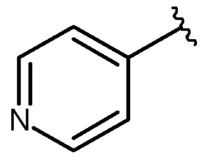 ;

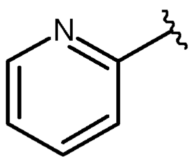 ; 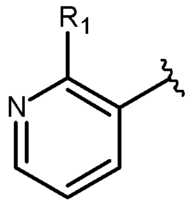 ; 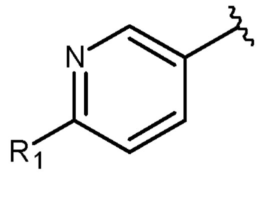 ; 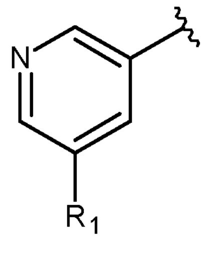 ; 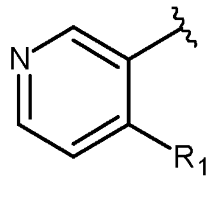 ;

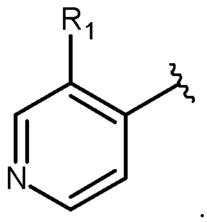 ; 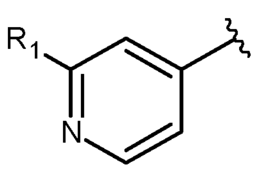 ; 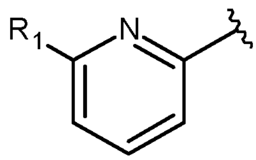 ; 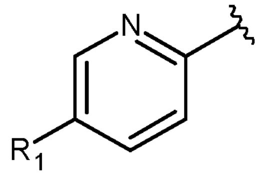 ; 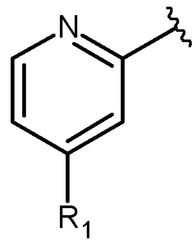 ;

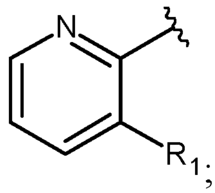 ;

each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;

each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;

k is 0, 1, 2 or 3;

Hal is fluoro, bromo, chloro or iodo.

**4.** The compound according to claim 1, wherein the fragment

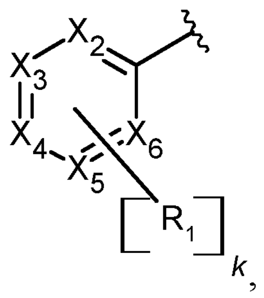

is selected from group, comprising:

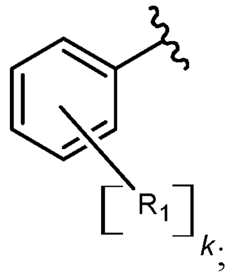 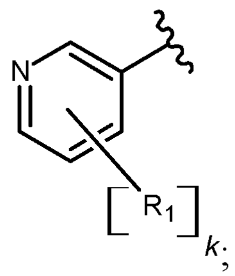

each $R_1$ is independently hydrogen; Hal; cyano; nitro; hydroxy group; $C_1$-$C_6$ alkyl, unsubstituted or substituted by one or several radicals selected from Hal, hydroxy group, $-NR_2R_3$; $C_1$-$C_6$ alkoxy group, unsubstituted or substituted by one or several radicals selected from Hal, $-NR_2R_3$, hydroxy group, $C_1$-$C_6$ alkyloxy, aryl, unsubstituted or substituted by one or several radicals selected from Hal, hydroxy group, $-NR_2R_3$; aryloxy, unsubstituted or substituted by one or several radicals selected from Hal, $C_1$-$C_6$ alkyl, hydroxy group, $-NR_2R_3$; $C_3$-$C_6$ cycloalkyloxy, unsubstituted or substituted by one or several radicals selected from Hal, hydroxy group, $-NR_2R_3$; $C_1$-$C_6$ alkyloxy $C_1$-$C_6$ alkyl; $-NR_2R_3$; aryl, unsubstituted or substituted by one or several radicals selected from Hal, hydroxy group, $-NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, $-NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, $-NR_2R_3$;

each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;

k is 0, 1, 2 or 3;

Hal is fluoro, bromo or chloro.

**5.** The compound according to claim 1, wherein the fragment

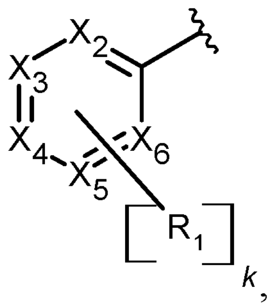

is selected from group, comprising:

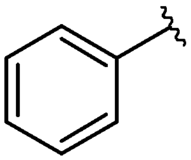;
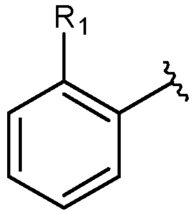;
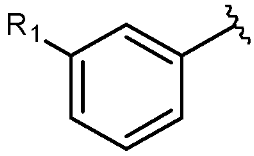;
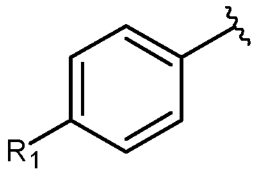;
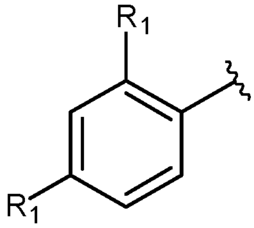;
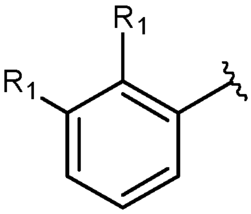;
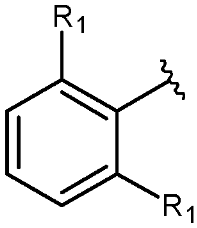;
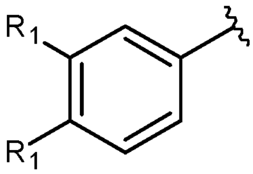;
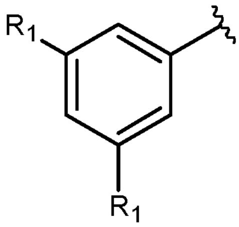;
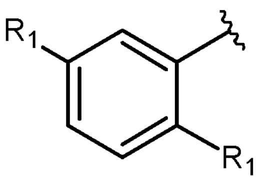;
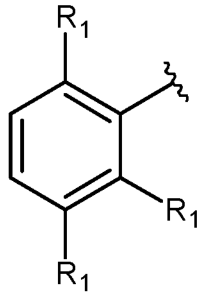;
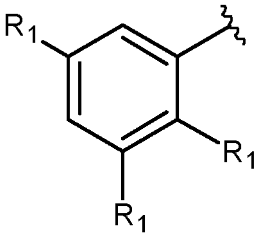;
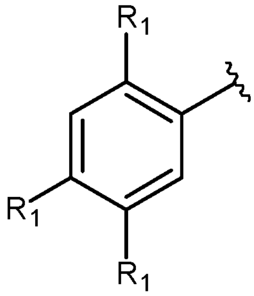;
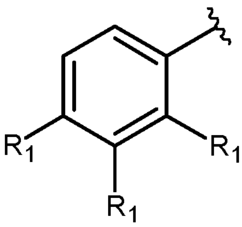;
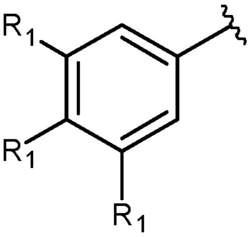;
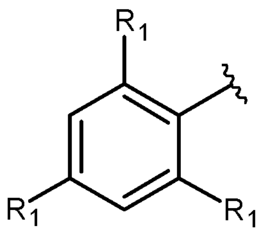;
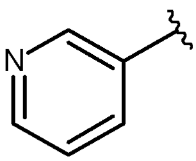;
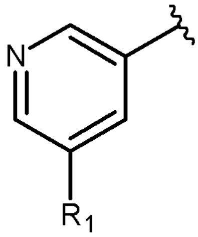;

each $R_1$ is independently hydrogen; Hal; cyano; nitro; hydroxy group; $C_1$-$C_6$ alkyl, unsubstituted or substituted by one or several radicals selected from Hal, hydroxy group, -$NR_2R_3$; $C_1$-$C_6$ alkoxy group, unsubstituted or substituted by one or several radicals selected from Hal, -$NR_2R_3$, hydroxy group, $C_1$-$C_6$ alkyloxy, aryl, unsubstituted or substituted by one or several radicals selected from Hal, hydroxy group, -$NR_2R_3$; aryloxy, unsubstituted or substituted by one or several radicals selected from Hal, $C_1$-$C_6$ alkyl, hydroxy group, -$NR_2R_3$; $C_3$-$C_6$ cycloalkyloxy, unsubstituted or substituted by one or several radicals selected from Hal, hydroxy group, -$NR_2R_3$; $C_1$-$C_6$ alkyloxy $C_1$-$C_6$ alkyl; -$NR_2R_3$; aryl, unsubstituted or substituted by one or several radicals selected from Hal, hydroxy group, -$NR_2R_3$; 5-6 membered heteroaryl with 1-2 heteroatoms, selected from N, O and/or S, unsubstituted or substituted by one or several substituents, selected from Hal, cyano, $C_1$-$C_6$ alkyl, hydroxy group, $C_1$-$C_6$ alkyloxy, -$NR_2R_3$; 4-7 membered heterocyclyl with 1-2 heteroatoms, selected from N and/or O, unsubstituted or substituted by one or several substituents, selected from Hal, cyano, hydroxy group, oxo, $C_1$-$C_6$ alkyl, $C_1$-$C_6$ alkyloxy, -$NR_2R_3$;
each $R_2$ or $R_3$ is independently hydrogen, $C_1$-$C_6$ alkyl, unsubstituted or substituted by one or several radicals selected from Hal, hydroxy group, $C_1$-$C_6$ alkyloxy;
Hal is fluoro, bromo or chloro.

**6.** The compound according to any one of claims 1-5, wherein the compound is:

N-(2-((2-(dimethylamino)ethyl)(methyl)amino)-4-methoxy-5-((4-(2-(trifluoromethyl)benzoyl)pyrimidin-2-yl)amino)phenyl)acrylamide (EGFR_3365) N-(5-((4-benzoylpyridin-2-yl)amino)-2-((2-(dimethylamino)ethyl)(methyl)amino)-4-methoxyphenyl)acrylamide (EGFR_3365_3)

N-(5-((4-(4-(dimethylamino)benzoyl)pyridin-2-yl)amino)-2-((2-(dimethylamino) ethyl)(methyl)amino)-4-methoxyphenyl)acrylamide 2,2,2-trifluoroacetate (EGFR_3365_4)

N-(5-((4-(4-(dimethylamino)benzoyl)pyridin-2-yl)amino)-2-((2-(dimethylamino) ethyl)(methyl)amino)-4-methoxyphenyl)acrylamide (EGFR_3365_4a)

N-(2-((2-(dimethylamino)ethyl)(methyl)amino)-4-methoxy-5-((4-(4-morpholino benzoyl)pyridin-2-yl)amino)phenyl)acrylamide 2,2,2-trifluoroacetate (EGFR_3365_5)

N-(2-((2-(dimethylamino)ethyl)(methyl)amino)-4-methoxy-5-((4-(4-morpholinobenzoyl)pyridin-2-yl)amino)phenyl)acrylamide (EGFR_3365_5a) N-(2-((2-(dimethylamino)ethyl)(methyl)amino)-5-((4-(4-fluorobenzoyl)pyrimidin-2-yl)amino)-4-methoxyphenyl)acrylamide (EGFR_3365_10)

N-(2-((2-(dimethylamino)ethyl)(methyl)amino)-5-((4-(3-fluorobenzoyl)pyrimidin-2-yl)amino)-4-methoxyphenyl)acrylamide (EGFR_3365_11)

N-(5-((4-(2-bromobenzoyl)pyrimidin-2-yl)amino)-2-((2-(dimethylamino)ethyl) (methyl)amino)-4-methoxyphenyl)acrylamide (EGFR_3365_12)

N-(5-((4-(4-bromobenzoyl)pyrimidin-2-yl)amino)-2-((2-(dimethylamino)ethyl) (methyl)amino)-4-methoxyphenyl)acrylamide (EGFR_3365_13)

N-(5-((4-(4-cyanobenzoyl)pyrimidin-2-yl)amino)-2-((2-(dimethylamino)ethyl) (methyl)amino)-4-methoxyphenyl)acrylamide 2,2,2-trifluoroacetate (EGFR_3365_14)

N-(5-((4-(4-cyanobenzoyl)pyrimidin-2-yl)amino)-2-((2-(dimethylamino)ethyl) (methyl)amino)-4-methoxyphenyl)acrylamide (EGFR_3365_14a)

N-(2-((2-(dimethylamino)ethyl)(methyl)amino)-4-methoxy-5-((4-nicotinoylpyrimidin-2-yl)amino)phenyl)acrylamide (EGFR_3365_15)

N-(5-((4-(4-(benzyloxy)benzoyl)pyrimidin-2-yl)amino)-2-((2-(dimethylamino) ethyl)(methyl)amino)-4-methoxyphenyl)acrylamide (EGFR_3365_16)

N-(2-((2-(dimethylamino)ethyl)(methyl)amino)-4-methoxy-5-((4-(4-phenoxybenzoyl)pyrimidin-2-yl)amino)phenyl)acrylamide (EGFR_3365_17)

N-(2-((2-(dimethylamino)ethyl)(methyl)amino)-4-methoxy-5-((4-(5-methylnicotinoyl)pyrimidin-2-yl)amino)phenyl)acrylamide (EGFR_3365_26)

N-(2-((2-(dimethylamino)ethyl)(methyl)amino)-4-methoxy-5-((4-(4-methoxybenzoyl)pyrimidin-2-yl)amino)phenyl)acrylamide (EGFR_3365_28)

N-(2-((2-(dimethylamino)ethyl)(methyl)amino)-5-((4-(4-ethoxybenzoyl)pyrimidin-2-yl)amino)-4-methoxyphenyl)acrylamide (EGFR_3365_29)

N-(2-((2-(dimethylamino)ethyl)(methyl)amino)-4-methoxy-5-((4-(4-propoxybenzoyl)pyrimidin-2-yl)amino)phenyl)acrylamide (EGFR_3365_30)

N-(2-((2-(dimethylamino)ethyl)(methyl)amino)-5-((4-(hydroxy(4-propoxyphenyl) methyl)pyrimidin-2-yl)amino)-4-methoxyphenyl)acrylamide (EGFR_3365_30a)

N-(2-((2-(dimethylamino)ethyl)(methyl)amino)-5-((4-(4-isopropoxybenzoyl) pyrimidin-2-yl)amino)-4-methoxyphenyl)acrylamide (EGFR_3365_31)

N-(2-((2-(dimethylamino)ethyl)(methyl)amino)-5-((4-(hydroxy(4-isopropoxyphenyl)methyl)pyrimidin-2-yl)amino)-4-methoxyphenyl)acrylamide (EGFR_3365_31a)

N-(2-((2-(dimethylamino)ethyl)(methyl)amino)-4-methoxy-5-((4-(3-methoxybenzoyl)pyrimidin-2-yl)amino)phenyl)acrylamide (EGFR_3365_32)

N-(2-((2-(dimethylamino)ethyl)(methyl)amino)-4-methoxy-5-((4-(2-methoxybenzoyl)pyrimidin-2-yl)amino)phenyl)acrylamide (EGFR_3365_33)

N-(2-((2-(dimethylamino)ethyl)(methyl)amino)-4-methoxy-5-((4-(3-nitrobenzoyl)pyrimidin-2-yl)amino)phenyl)acrylamide (EGFR_3365_34)

N-(2-((2-(dimethylamino)ethyl)(methyl)amino)-4-methoxy-5-((4-(2-nitrobenzoyl)pyrimidin-2-yl)amino)phenyl)acrylamide (EGFR_3365_36)

N-(2-((2-(dimethylamino)ethyl)(methyl)amino)-4-methoxy-5-((4-(4-propoxybenzoyl)pyridin-2-yl)amino)phenyl)acrylamide (EGFR_3365_50)

N-(2-((2-(dimethylamino)ethyl)(methyl)amino)-4-methoxy-5-((4-(4-methoxybenzoyl)pyridin-2-yl)amino)phenyl)acrylamide (EGFR_3365_51)

N-(2-((2-(dimethylamino)ethyl)(methyl)amino)-5-((4-(4-ethoxybenzoyl)pyridin-2-yl)amino)-4-methoxyphenyl)acrylamide (EGFR_3365_52)

N-(2-((2-(dimethylamino)ethyl)(methyl)amino)-4-methoxy-5-((4-(4-(3-methoxy azetidin-1-yl)benzoyl)pyridin-2-yl)amino)phenyl)acrylamide (EGFR_3365_53)

N-(5-((4-(4-(diethylamino)benzoyl)pyridin-2-yl)amino)-2-((2-(dimethylamino) ethyl)(methyl)amino)-4-methoxyphenyl)acrylamide (EGFR_3365_54)
N-(2-((2-(dimethylamino)ethyl)(methyl)amino)-4-methoxy-5-((4-(4-(4-methyl piperazin-1-yl)benzoyl)pyridin-2-yl)amino)phenyl)acrylamide (EGFR_3365_55)
N-(2-((2-(dimethylamino)ethyl)(methyl)amino)-4-methoxy-5-((4-(4-(pyrrolidin-1-yl)benzoyl)pyridin-2-yl)amino)phenyl)acrylamide (EGFR_3365_56)
N-(2-((2-(dimethylamino)ethyl)(methyl)amino)-4-methoxy-5-((4-(4-methylbenzoyl)pyridin-2-yl)amino)phenyl)acrylamide (EGFR_3365_57)
N-(5-((4-(4-(azetidin-1-yl)benzoyl)pyrimidin-2-yl)amino)-2-((2-(dimethylamino) ethyl)(methyl)amino)-4-methoxyphenyl)acrylamide (EGFR 3365 58)
N-(2-((2-(dimethylamino)ethyl)(methyl)amino)-4-methoxy-5-((4-(4-(3-methoxy azetidin-1-yl)benzoyl)pyrimidin-2-yl)amino)phenyl)acrylamide (EGFR _3365_61)
N-(2-((2-(dimethylamino)ethyl)(methyl)amino)-4-methoxy-5-((4-(4-(4-methylpiperazin-1-yl)benzoyl)pyrimidin-2-yl)amino)phenyl)acrylamide formate (EGFR_3365_62)
N-(2-((2-(dimethylamino)ethyl)(methyl)amino)-4-methoxy-5-((4-(4-(4-methylpiperazin-1-yl)benzoyl)pyrimidin-2-yl)amino)phenyl)acrylamide (EGFR_3365_62a)
N-(2-((2-(dimethylamino)ethyl)(methyl)amino)-4-methoxy-5-((4-(4-(pyrrolidin-1-yl)benzoyl)pyrimidin-2-yl)amino)phenyl)acrylamide (EGFR_3365_63)
N-(2-((2-(dimethylamino)ethyl)(methyl)amino)-4-methoxy-5-((4-(4-methylbenzoyl)pyrimidin-2-yl)amino)phenyl)acrylamide (EGFR_3365_64)
N-(5-((4-(4-butoxybenzoyl)pyrimidin-2-yl)amino)-2-((2-(dimethylamino) ethyl)(methyl)amino)-4-methoxyphenyl)acrylamide (EGFR_3365_66)
N-(5-((4-(4-(cyclohexyloxy)benzoyl)pyrimidin-2-yl)amino)-2-((2-(dimethylamino) ethyl)(methyl)amino)-4-methoxyphenyl)acrylamide (EGFR_3365_67)
N-(5-((4-(2,4-diethoxybenzoyl)pyrimidin-2-yl)amino)-2-((2-(dimethylamino)ethyl) (methyl)amino)-4-methoxyphenyl)acrylamide (EGFR_3365_68)
N-(5-((4-(2,4-dimethoxybenzoyl)pyrimidin-2-yl)amino)-2-((2-(dimethylamino) ethyl)(methyl)amino)-4-methoxyphenyl)acrylamide (EGFR_3365_69)
N-(2-((2-(dimethylamino)ethyl)(methyl)amino)-5-((4-(2,4-dipropoxybenzoyl) pyrimidin-2-yl)amino)-4-methoxyphenyl)acrylamide (EGFR_3365_70)
N-(5-((4-(2,4-diisopropoxybenzoyl)pyrimidin-2-yl)amino)-2-((2-(dimethylamino) ethyl)(methyl)amino)-4-methoxyphenyl)acrylamide (EGFR_3365_71)
N-(5-((4-(4-(diethylamino)benzoyl)pyrimidin-2-yl)amino)-2-((2-(dimethylamino) ethyl)(methyl)amino)-4-methoxyphenyl)acrylamide (EGFR_3365_72)
N-(5-((4-(4-(dimethylamino)benzoyl)pyrimidin-2-yl)amino)-2-((2-(dimethyl amino)ethyl)(methyl)amino)-4-methoxyphenyl)acrylamide (EGFR_3365_73)
N-(2-((2-(dimethylamino)ethyl)(methyl)amino)-5-((4-(4-isobutoxybenzoyl) pyridin-2-yl)amino)-4-methoxyphenyl)acrylamide (EGFR_3365_77)
N-(2-((2-(dimethylamino)ethyl)(methyl)amino)-5-((4-(4-isobutoxybenzoyl) pyrimidin-2-yl)amino)-4-methoxyphenyl)acrylamide (EGFR_3365_78)
N-(2-((2-(dimethylamino)ethyl)(methyl)amino)-4-methoxy-5-((4-(4-(1-methyl-1H-pyrazol-4-yl)benzoyl)pyridin-2-yl)amino)phenyl)acrylamide (EGFR_3365 _85)
N-(5-((4-(4-(1H-imidazol-1-yl)benzoyl)pyrimidin-2-yl)amino)-2-((2-(dimethyl amino)ethyl)(methyl)amino)-4-methoxyphenyl)acrylamide (EGFR_3365_86)
N-(5-((4-(2,4-dimethoxybenzoyl)pyridin-2-yl)amino)-2-((2-(dimethylamino) ethyl)(methyl)amino)-4-methoxyphenyl)acrylamide (EGFR_3365 87)
N-(2-((2-(dimethylamino)ethyl)(methyl)amino)-4-methoxy-5-((4-(2-methoxybenzoyl)pyridin-2-yl)amino)phenyl)acrylamide (EGFR_3365_88)
N-(2-((2-(dimethylamino)ethyl)(methyl)amino)-4-methoxy-5-((4-(4-(piperidin-1-yl)benzoyl)pyrimidin-2-yl)amino)phenyl)acrylamide (EGFR _3365 _90)
N-(2-((2-(dimethylamino)ethyl)(methyl)amino)-4-methoxy-5-((4-(4-(2-oxopyrrolidin-1-yl)benzoyl)pyrimidin-2-yl)amino)phenyl)acrylamide (EGFR_3365_91)
N-(2-((2-(dimethylamino)ethyl)(methyl)amino)-4-methoxy-5-((4-(4-(2-methoxy ethoxy)benzoyl)pyrimidin-2-yl)amino)phenyl)acrylamide (EGFR_3365_92)
N-(2-((2-(dimethylamino)ethyl)(methyl)amino)-4-methoxy-5-((4-(4-(2-methoxyethoxy)benzoyl)pyridin-2-yl)amino)phenyl)acrylamide (EGFR_3365_93)
N-(2-((2-(dimethylamino)ethyl)(methyl)amino)-4-methoxy-5-((4-(1-methyl-1H-indole-2-carbonyl)pyrimidin-2-yl)amino)phenyl)acrylamide (EGFR _3365 _94)
N-(5-((4-(4-(4-cyanopiperidin-1-yl)benzoyl)pyrimidin-2-yl)amino)-2-((2-(dimethylamino)ethyl)(methyl)amino)-4-methoxyphenyl)acrylamide (EGFR_3365_97)
N-(2-((2-(dimethylamino)ethyl)(methyl)amino)-4-methoxy-5-((4-(4-(4-methoxypiperidin-1-yl)benzoyl)pyrimidin-2-yl)amino)phenyl)acrylamide (EGFR_3365_98)
N-(2-((2-(dimethylamino)ethyl)(methyl)amino)-4-methoxy-5-((4-(2-methoxy-4-propoxybenzoyl)pyrimidin-2-yl)amino)phenyl)acrylamide (EGFR_3365_101)
N-(2-((2-(dimethylamino)ethyl)(methyl)amino)-4-methoxy-5-((4-(4-methoxy-2-propoxybenzoyl)pyrimidin-2-yl)amino)phenyl)acrylamide (EGFR_3365_102)
N-(2-((2-(dimethylamino)ethyl)(methyl)amino)-5-((4-(4-isopropoxy-2-methoxy benzoyl)pyrimidin-2-yl)amino)-4-methoxyphenyl)acrylamide (EGFR_3365_103)
N-(2-((2-(dimethylamino)ethyl)(methyl)amino)-5-((4-(2-isopropoxy-4-methoxy benzoyl)pyrimidin-2-yl)amino)-4-methoxyphenyl)acrylamide (EGFR_3365_104)
N-(2-((2-(dimethylamino)ethyl)(methyl)amino)-4-methoxy-5-((4-(4-((2-methoxy ethyl)amino)benzoyl)pyrimidin-2-yl)amino)phenyl)acrylamide (EGFR_3365_105)
N-(2-((2-(dimethylamino)ethyl)(methyl)amino)-5-((4-(4-((2-hydroxyethyl)amino) benzoyl)pyrimidin-2-yl)amino)-4-methoxyphenyl)acrylamide formate (EGFR _3365_106)
N-(2-((2-(dimethylamino)ethyl)(methyl)amino)-5-((4-(4-((2-hydroxyethyl)amino) benzoyl)pyrimidin-2-yl)amino)-4-methoxyphenyl)acrylamide (EGFR_3365_106a)
(S)-N-(2-((2-(dimethylamino)ethyl)(methyl)amino)-5-((4-(4-(3-hydroxypiperidin-1-yl)benzoyl)pyrimidin-2-yl)amino)-4-methoxyphenyl)acrylamide (EGFR_3365_108)
(R)-N-(2-((2-(dimethylamino)ethyl)(methyl)amino)-5-((4-(4-(3-hydroxypiperidin-1-yl)benzoyl)pyrimidin-2-yl)amino)-4-methoxyphenyl)acrylamide (EGFR _3365_109)
(S)-N-(2-((2-(dimethylamino)ethyl)(methyl)amino)-4-methoxy-5-((4-(4-(3-methoxypiperidin-1-yl)benzoyl)pyrimidin-2-yl)amino)phenyl)acrylamide (EGFR_3365_110)
(R)-N-(2-((2-(dimethylamino)ethyl)(methyl)amino)-4-methoxy-5-((4-(4-(3-methoxypiperidin-1-yl)benzoyl)pyrimidin-2-yl)amino)phenyl)acrylamide (EGFR_3365_111)
(S)-N-(2-((2-(dimethylamino)ethyl)(methyl)amino)-5-((4-(4-(3-hydroxypyrrolidin-1-yl)benzoyl)pyrimidin-2-yl)amino)-4-methoxyphenyl)acrylamide formate (EGFR_3365_112)
(S)-N-(2-((2-(dimethylamino)ethyl)(methyl)amino)-5-((4-(4-(3-hydroxypyrrolidin-1-yl)benzoyl)pyrimidin-2-yl)amino)-4-methoxyphenyl)acrylamide (EGFR _3365 _112a)
(R)-N-(2-((2-(dimethylamino)ethyl)(methyl)amino)-5-((4-(4-(3-hydroxypyrrolidin-1-yl)benzoyl)pyrimidin-2-yl)amino)-4-methoxyphenyl)acrylamide (EGFR_3365_113)
(S)-N-(2-((2-(dimethylamino)ethyl)(methyl)amino)-4-methoxy-5-((4-(4-(3-methoxypyrrolidin-1-yl)benzoyl)pyrimidin-2-yl)amino)phenyl)acrylamide (EGFR _3365 _114)
(R)-N-(2-((2-(dimethylamino)ethyl)(methyl)amino)-4-methoxy-5-((4-(4-(3-methoxypyrrolidin-1-yl)benzoyl)pyrimidin-2-yl)amino)phenyl)acrylamide (EGFR_3365_115)
N-(5-((4-(4-cyclopropoxybenzoyl)pyrimidin-2-yl)amino)-2-((2-(dimethylamino) ethyl)(methyl)amino)-4-methoxyphenyl)acrylamide (EGFR_3365_116)
N-(2-((2-(dimethylamino)ethyl)(methyl)amino)-4-methoxy-5-((4-(2-methoxy-4-(4-methylpiperazin-1-yl)benzoyl)pyrimidin-2-yl)amino)phenyl)acrylamide (EGFR _3365_120)
N-(5-((4-(4-(4-aminopiperidin-1-yl)benzoyl)pyrimidin-2-yl)amino)-2-((2-(dimethylamino)ethyl)(methyl)amino)-4-methoxyphenyl)acrylamide (EGFR _3365_121)
N-(2-((2-(dimethylamino)ethyl)(methyl)amino)-5-((4-(4-(4-(dimethylamino) piperidin-1-yl)benzoyl)pyrimidin-2-yl)amino)-4-methoxyphenyl)acrylamide (EGFR_3365_121a)
N-(2-((2-(dimethylamino)ethyl)(methyl)amino)-4-methoxy-5-((4-(2-methoxy-4-(piperidin-1-yl)benzoyl)pyrimidin-2-yl)amino)phenyl)acrylamide (EGFR_3365_122)
N-(2-((2-(dimethylamino)ethyl)(methyl)amino)-4-methoxy-5-((4-(2-methoxy-4-(pyrrolidin-1-yl)benzoyl)pyrimidin-2-yl)amino)phenyl)acrylamide (EGFR _3365_123)
N-(2-((2-(dimethylamino)ethyl)(methyl)amino)-4-methoxy-5-((4-(2,4,6-trimethoxybenzoyl)pyrimidin-2-yl)amino)phenyl)acrylamide (EGFR_3365_124)
N-(5-((4-(4-(4-aminopiperidin-1-yl)-2-methoxybenzoyl)pyrimidin-2-yl)amino)-2-((2-(dimethylamino)ethyl)(methyl)amino)-4-methoxyphenyl)acrylamide (EGFR _3365_126)
N-(2-((2-(dimethylamino)ethyl)(methyl)amino)-5-((4-(4-(4-(dimethylamino) piperidin-1-yl)-2-methoxybenzoyl)pyrimidin-2-yl)amino)-4-methoxyphenyl) acrylamide (EGFR_3365_127)

**7.** A method for inhibiting biological activity of epidermal growth factor receptor (EGFR) in a subject, comprising contacting EGFR with the compound according to any of claims 1-6.

**8.** A pharmaceutical composition, comprising a therapeutically effective amount of the compound according to any of

claims 1-6 and one or more pharmaceutically acceptable excipients, wherein said pharmaceutical composition being intended for preventing or treating a disease or disorder mediated by the activation of EGFR.

**9.** The pharmaceutical composition according to claim 8, wherein the disease or disorder is the disease or disorder mediated by the activation of EGFR with a L858R mutation and/or a T790M mutation and/or an exon 19 deletion and/or a C797S mutation.

**10.** A method for treating a disease or disorder mediated by the activation of EGFR comprising administering a therapeutically effective amount of the compound according to any of claims 1-6 or the pharmaceutical composition according to claim 8 in a subject in need thereof.

**11.** The method according to claim 10, wherein the disease or disorder is the disease or disorder mediated by the activation of EGFR with a L858R mutation and/or a T790M mutation and/or an exon 19 deletion and/or a C797S mutation.

**12.** The method according to claim 11, wherein the disease or disorder mediated by the activation of EGFR is oncological disease.

**13.** The method according to claim 12, wherein the disease or disorder is bladder cancer, ovarian cancer, cervical cancer, colorectal cancer, breast cancer, pancreatic cancer, head and neck cancer, glioma, glioblastoma , melanoma, prostate cancer, leucosis, lymphoma, non-Hodgkin lymphoma, Hodgkin's lymphoma, lung cancer, hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

**14.** The method according to claim 13, wherein the oncological disease is non-small cell lung cancer.

**15.** Use of the compound according to any of claims 1-6 or a pharmaceutical composition according to claim 8 for the treatment of a disease or disorder mediated by the activation of EGFR in a subject in need thereof.

**16.** The use according to claim 15, wherein the disease or disorder is the disease or disorder mediated by the activation of EGFR with a L858R mutation and/or a T790M mutation and/or an exon 19 deletion and/or a C797S mutation.

**17.** The use according to claim 16, wherein the disease or disorder mediated by the activation of EGFR is oncological disease.

**18.** The use according to claim 17, wherein the disease or disorder is bladder cancer, ovarian cancer, cervical cancer, colorectal cancer, breast cancer, pancreatic cancer, head and neck cancer, glioma, glioblastoma, melanoma, prostate cancer, leucosis, lymphoma, non-Hodgkin lymphoma, Hodgkin's lymphoma, lung cancer, hepatocellular cancer, esophageal cancer, stomach cancer, gastrointestinal stromal tumor, thyroid cancer, bile duct cancer, endometrial cancer, renal cell cancer, liver cancer, anaplastic large-cell lymphoma, acute myeloid leukemia, multiple myeloma, melanoma, mesothelioma, hematological malignant tumors.

**19.** The use according to claim 18, wherein the oncological disease is non-small cell lung cancer.

## INTERNATIONAL SEARCH REPORT

International application No.
PCT/RU 2018/050122

A. CLASSIFICATION OF SUBJECT MATTER
see extra sheet

According to International Patent Classification (IPC) or to both national classification and IPC

B. FIELDS SEARCHED

Minimum documentation searched (classification system followed by classification symbols)
C07D 401/06, 403/06, 239/42, 213/74, 401/10, 403/10, A61K 31/44, 31/444, 31/505, 31/506, A61P 35/00

Documentation searched other than minimum documentation to the extent that such documents are included in the fields searched

Electronic data base consulted during the international search (name of data base and, where practicable, search terms used)
PatSearch (RUPTO internal), Esp@cenet, PAJ, USPTO, Information Retrieval System of FIPS

C. DOCUMENTS CONSIDERED TO BE RELEVANT

| Category* | Citation of document, with indication, where appropriate, of the relevant passages | Relevant to claim No. |
|---|---|---|
| Y | EP 3181559 A1 (CHANGZHOU RUNNOR BIOLOGICAL TECHNOLOGY CO., LTD.) 21.06.2017, the abstract, paragraphs [0003] - [0023], [0050] - [0075], the claims | 1-19 |
| Y | WO 2011/140338 A1 (GATEKEEPER PHARMACEUTICALS, INC et al.) 10.11.2011, the abstract, the claims | 1-19 |
| Y | US 2011/0207736 A1 (GATEKEEPER PHARMACEUTICALS, INC.) 25.08.2011, the abstract, paragraphs [0018] - [0021], the claims | 1-19 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 November 2018 (23.11.2018) | 20 December 2018 (20.12.2018) |
| Name and mailing address of the ISA/ RU | Authorized officer |
| Facsimile No. | Telephone No. |

Form PCT/ISA/210 (second sheet) (July 1998)

INTERNATIONAL SEARCH REPORT

CLASSIFICATION OF SUBJECT MATTER

International application No.
PCT/RU 2018/050122

***C07D 401/06*** *(2006.01)*
***C07D 403/06*** *(2006.01)*
***C07D 239/42*** *(2006.01)*
***C07D 213/74*** *(2006.01)*
***C07D 401/10*** *(2006.01)*
***C07D 403/10*** *(2006.01)*
***A61 K 31/506*** *(2006.01)*
***A61K 31/505*** *(2006.01)*
***A61K 31/44*** *(2006.01)*
***A61K 31/444*** *(2006.01)*
***A61P 35/00*** *(2006.01)*

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A


### Non-patent literature cited in the description


- **FERGUSON et al.** *Mol Cell,* 2003, vol. 11, 507-517
- **GARRETT et al.** *Cell,* 2002, vol. 110, 763-773
- **OGISO et al.** *Cell,* 2002, vol. 110, 775-787
- *Ciardiello F. N. Engl J Med,* 2008, vol. 358, 1160-1174
- **ROBERT ROSKOSKI JR.** *Biochemical and Biophysical Research Communications,* 2004, vol. 319, 1-11
- **DOWNWARD J.** *Nature,* 1984, vol. 307, 521-527
- *Olofsson B. Eur. J. Biochem.,* 1986, vol. 160, 261-266
- *Gusterson B. Cell Biol. Int. Rep.,* 1984, vol. 8, 649-658
- *Gusterson B. Int. J. Cancer.,* 1985, vol. 36, 689-693
- *Veale D. Br. J. Cancer.,* 1987, vol. 55, 513-516
- **WONG A.J.** *Proc. Natl. Acad. Sci. USA.,* 1987, vol. 84, 6899-6903
- **ATALAY et al.** *Ann. Oncology,* 2003, vol. 14, 1346-1363
- **HERBST R.S.** *Cancer.,* 2002, vol. 94, 1593-1611
- **MODJTAHEDI et al.** *Br. J. Cancer.,* 1996, vol. 73, 228-235
- **HERBST R.S.** *Cancer,* 2002, vol. 94, 1593-1611
- **HERBST R.S.** *Cancer,* 2002, vol. 94, 1593-1611
- **PING WEE.** *Cancers (Basel),* May 2017, vol. 9 (5), 52
- **BERGE S.M. et al.** Pharmaceutical Salts. *J. Pharm. Sci.,* 1977, vol. 66, 1-19
- **STERN et al.** *Critical reviews in oncology/haematology,* 2005, vol. 54, 11-29